(19) Europäisches Patentamt
European Patent Office
Office européen des brevets

(11) EP 4 335 370 A1

(12) EUROPEAN PATENT APPLICATION

(43) Date of publication:
13.03.2024  Bulletin 2024/11

(21) Application number: 22382837.7

(22) Date of filing: 09.09.2022

(51) International Patent Classification (IPC):
$A61B\ 5/16^{(2006.01)}$    $A61B\ 5/246^{(2021.01)}$
$A61B\ 5/374^{(2021.01)}$   $A61B\ 5/378^{(2021.01)}$
$A61B\ 5/00^{(2006.01)}$    $A61B\ 5/055^{(2006.01)}$

(52) Cooperative Patent Classification (CPC):
A61B 5/168; A61B 5/0042; A61B 5/0075;
A61B 5/055; A61B 5/246; A61B 5/374;
A61B 5/378; A61B 5/7246; G16H 50/20;
G16H 50/30; G16H 40/63

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Bitsphi Diagnosis SL.
28023 Madrid (ES)

(72) Inventors:
• Medrano López, Álvaro
  28023 Madrid (ES)
• Blanco Carmona, Miguel
  28023 Madrid (ES)
• Mestu Unturbe, Fernando
  28023 Madrid (ES)

(74) Representative: Grünecker Patent- und
Rechtsanwälte
PartG mbB
Leopoldstraße 4
80802 München (DE)

(54) **APPARATUS FOR GENERATING A SCORE THAT IS INDICATIVE FOR A COGNITIVE CONDITION LIKE ATTENTION DEFICIT HYPERACTIVITY DISORDER**

(57)    The present disclosure relates to the generation of a score that is indicative for a subject having a cognitive condition, such as a cognitive disorder like attention deficit hyperactivity disorder, ADHD. In particular, the score may be generated based on electro- or magnetoencephalography (EEG/MEG) data by calculating one or more correlation values between pairs of time series that are determined based on the EEG/MEG data. An apparatus, system, computer-readable medium and computer-implemented method described herein receive EEG/MEG data of a subject. Then a first plurality of time series is determined based on the EEG/MEG data, wherein each of the first plurality of times series corresponds to a respective source position located inside a cranial cavity of the subject. Then, a first correlation value may be calculated for a first pair of time series, the first pair of time series being included in the determined first plurality of time series. A score is generated based on the first correlation value, the score being indicative for the subject having a cognitive condition, such as a cognitive disorder like attention deficit hyperactivity disorder, ADHD. The score is outputted, e.g., to a physician who may diagnose the subject.

FIG. 1

**Description**

BACKGROUND

The Bayesian brain theory establishes that the entire cortex represents probability distributions which only collapse onto estimates when decisions are needed. Nevertheless, the way in which this collapse takes place, passing from a probabilistic brain to certitude or one-shot decisions, is not well known. Therefore, it is pending to derive a metric that reconciles stimulus-bounded surprise (Shannon surprise) and that allows the computation of the information gain between prior and posterior beliefs (Bayesian surprise). Expressing the probability of an event in terms of its information content (e.g., the -log function of the mentioned probability), also known as Shannon surprise, implies passing from a probabilistic environment to a deterministic one; indeed, being surprised by the occurrence of an event, may imply that it has already occurred, so the bits of information generated may give accuracy, i.e., they may cancel the uncertainty about the occurrence of the event. Thus, it may be necessary to differentiate the bits of accuracy from the Shannon information content from the bits of the Shannon entropy, which express uncertainty.

In attention research it is postulated that a salience map generated by the sensory salience of objects is modulated by top-down behavioral aware signals to generate a priority map. Although a lot of research has been done on the neural encoding (e.g., through computation modeling of attention) a proper treatment of top-down attention signals has not been achieved. Instead, most of the prior art modeling research has been focused on the bottom-up component of visual attention.

In addition, due to the complexity and heterogeneity of cognitive conditions, such as a cognitive disorders like attention deficit hyperactivity disorder (ADHD), the traditional gap between neurobiology and symptoms in such disorders is often large, and therefore, its underlying neurocognitive mechanisms are often unknown.

As such, it may be beneficial to provide an improved system for generating a score that is indicative for a subject having a cognitive condition, such as a cognitive disorder like attention deficit hyperactivity disorder, ADHD, a corresponding computer-readable medium and a corresponding computer-implemented method.

SUMMARY

The present disclosure relates to the generation of a score that is indicative for a subject having a cognitive condition, such as a cognitive disorder like attention deficit hyperactivity disorder, ADHD. In particular, the score may be generated based on electro- or magnetoencephalography (EEG/MEG) data by calculating one or more correlation values between pairs of time series that are determined based on the EEG/MEG data. An apparatus, system, computer-readable medium and computer-implemented method described herein generate of a score that is indicative for a subject having a cognitive condition, such as a cognitive disorder like attention deficit hyperactivity disorder, ADHD.

According to the present disclosure an apparatus is configured to receive first EEG/MEG data of a subject. The apparatus is further configured to determine a first plurality of time series based on the EEG/MEG data, wherein each of the first plurality of times series corresponds to a respective source position located inside a cranial cavity of the subject. In addition, the apparatus is configured to calculate a first correlation value for a first pair of time series, wherein the first pair of time series is included in the determined first plurality of time series. The apparatus is further configured to generate a score based on the first correlation value, the score being indicative for the subject having a cognitive condition, such as a cognitive disorder like attention deficit hyperactivity disorder, ADHD. In addition, the apparatus is configured to output the score.

According to one embodiment the respective source position of a first time series of the first pair of time series may be located in a frontal lobe, such as in a middle frontal gyrus (MFG) and/or in an inferior frontal gyrus (IFG) in the cranial cavity of the subject, and the respective source position of a second time series of the first pair of time series may be located in a superior parietal lobe (SPL) in the cranial cavity of the subject.

According to a further embodiment, the apparatus may be further configured to calculate a second correlation value for a second pair of time series, the second pair of time series being included in the determined first plurality of time series. The score may be generated based on the first correlation value and the second correlation value. According to this embodiment, the respective source position of a first time series of the second pair of time series may be located in a ventral attention network area, such as in a temporoparietal junction (TPJ) in the cranial cavity of the subject, and the respective source position of a second time series of the second pair of time series may be located in a dorsal attention network area, such as in a superior parietal lobe (SPL) in the cranial cavity of the subject.

According to an embodiment, the apparatus may be further configured to calculate a third correlation value for a third pair of time series, the third pair of time series being included in the determined first plurality of time series. The score may be generated based on the first correlation value, the second correlation value and the third correlation value. In addition, the respective source position of a first time series of the third pair of time series may be located in the ventral attention network area, such as the temporoparietal junction (TPJ) in the cranial cavity of the subject, and the respective source position of a second time series of the second pair of time series is located in a ventral visual pathway area, such as an inferior temporal gyrus (ITG) in the cranial cavity of the subject.

In a further embodiment the apparatus may be further configured to receive second EEG/MEG data of the subject and to determine a second plurality of time series based on the second EEG/MEG data, wherein each of the second plurality of times series corresponds to a respective source position located inside the cranial cavity of the subject. In addition, the apparatus may be further configured to calculate a fourth correlation value for a fourth pair of time series, the fourth pair of time series being included in the determined second plurality of time series, and the fourth pair of time series corresponding to the same respective source positions as the first pair of time series. Further, the apparatus may be configured to calculate a comparison value between the fourth correlation value and the first correlation value. In addition, the score may be generated based on the comparison value.

In an embodiment, the step of determining the first plurality of time series may comprise filtering the first EEG/MEG data such that the first plurality of time series only includes a subset of all available time series in the first EEG/MEG data, the subset including the first pair of time series, the second pair of time series and the third pair of time series.

In an embodiment, a system may comprise the apparatus according to any of the above-described embodiments, and may additionally comprise an EEG or MEG device for measuring at least the first EEG or MEG data of the subject, and a task presentation device for presenting one or more tasks to the subject while the EEG/MEG device is measuring at least the first EEG/MEG data of the subject.

According to the disclosure, a computer-implemented method comprises receiving first electro- or magnetoencephalography (EEG/MEG) data of a subject. The method further comprises determining a first plurality of time series based on the first EEG/MEG data, wherein each of the first plurality of times series corresponds to a respective source position located inside a cranial cavity of the subject. In addition, the method comprises calculating a first correlation value for a first pair of time series, the first pair of time series being included in the determined first plurality of time series. The method further comprises generating a score based on the first correlation value, the score being indicative for the subject having a cognitive condition, such as a cognitive disorder like ADHD, and outputting the generated score.

According to an embodiment the respective source position of a first time series of the first pair of time series may be located in a frontal lobe, such as in a middle frontal gyrus (MFG) and/or in an inferior frontal gyrus (IFG) in the cranial cavity of the subject, and the respective source position of a second time series of the first pair of time series may be located in a superior parietal lobe (340), SPL in the cranial cavity of the subject.

According to an embodiment the method may further comprise calculating a second correlation value for a second pair of time series, the second pair of time series being included in the determined first plurality of time series. The score may be generated based on the first correlation value and the second correlation value. In this embodiment, the respective source position of a first time series of the second pair of time series may be located in ventral attention network area, such as in a temporoparietal junction (TPJ) in the cranial cavity of the subject, and the respective source position of a second time series of the second pair of time series may be located in a dorsal attention network area, such as in a superior parietal lobe (SPL) in the cranial cavity of the subject.

According to a further embodiment the method may further comprise calculating a third correlation value for a third pair of time series, the third pair of time series being included in the determined first plurality of time series. The score may be generated based on the first correlation value, the second correlation value and the third correlation value. In addition, the respective source position of a first time series of the third pair of time series may be located in the ventral attention network area, such as in the temporoparietal junction (TPJ), in the cranial cavity of the subject, and the respective source position of a second time series of the second pair of time series may be located in a ventral visual pathway area, such as an inferior temporal gyrus (ITG) in the cranial cavity of the subject.

According to embodiments, the method may further comprise receiving second EEG/MEG data of the subject (and determining a second plurality of time series based on the second EEG/MEG data, wherein each of the second plurality of times series corresponds to a respective source position located inside the cranial cavity of the subject. In addition, the method may further comprise calculating a fourth correlation value for a fourth pair of time series, the fourth pair of time series being included in the determined second plurality of time series, and the fourth pair of time series corresponding to the same respective source positions as the first pair of time series. In addition, the method may comprise calculating a comparison value between the fourth correlation value and the first correlation value. The score may be generated based on the comparison value.

According to an embodiment the step of the determining the first plurality of time series may comprise filtering the first EEG/MEG data such that the first plurality of time series only includes a subset of all available time series in the first EEG/MEG data, the subset including the first pair of time series, the second pair of time series, and the third pair of time series.

The above described embodiments can be combined with each other. The above described embodiments may also be implemented on a computer-readable medium comprising computer-readable instructions, that, when executed by a processor, cause the processor to perform the above described steps.

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

BRIEF SUMMARY OF THE DRAWINGS

These and further aspects and features of the present disclosure will be described by the following detailed description of the embodiments of the disclosure under reference to the accompanying drawings, wherein

Fig. 1 is a diagram illustrating a Shannonian brain model according an embodiment of the present disclosure.

Fig. 2 is a diagram illustrating a dorsal-ventral attention network according to an embodiment of the present disclosure.

Fig. 3 is a diagram illustrating conditional probabilities and information according to an exemplary task ("Task 2") to be performed by a subject while EEG or MEG data is being measured from the subject.

Fig. 4 is a diagram illustrating experimental results that show links with significantly different PLV-values in the comparison between two conditions of a same task ("Task1") measured from healthy subjects.

Fig. 5 is a diagram illustrating experimental results that show links with significantly different PLV-values in the comparison between two conditions of two respective tasks ("Task1" and "Task2") measured from healthy subjects.

Fig. 6 is a diagram illustrating a diagram illustrating experimental results that show links with significantly different PLV-values in the comparison between two conditions of two respective tasks ("Task1" and "Task2") measured from subjects having ADHD.

Fig. 7 is a diagram illustrating experimental results that show links of the central executive network with significantly different PLV-values in the comparison between healthy subjects and subjects having ADHD for a condition of a task ("Task1").

Fig. 8 is a diagram illustrating experimental results that show links of the salience network with significantly different PLV-values in the comparison between healthy subjects and subjects having ADHD for a condition of a task ("Task1").

Fig. 9 is a diagram illustrating experimental results that show links of the central executive network with significantly different PLV-values in the comparison between healthy subjects and subjects having ADHD for a condition of a task ("Task2").

Fig. 10 is a diagram illustrating experimental results that show links of the central executive network with significantly different PLV-values in the comparison between healthy subjects and subjects having ADHD for a condition of a task ("Task2").

Fig. 11 is a diagram illustrating experimental results that show links of the salience network with significantly different PLV-values in the comparison between healthy subjects and subjects having ADHD for a condition of a task ("Task1").

Fig. 12 is a diagram illustrating experimental results that show links of the central executive network with significantly different PLV-values in the comparison between healthy subjects and subjects having ADHD for a condition of a task ("Task1").

Fig. 13 is a diagram illustrating an example system according to one or more embodiments of the present disclosure.

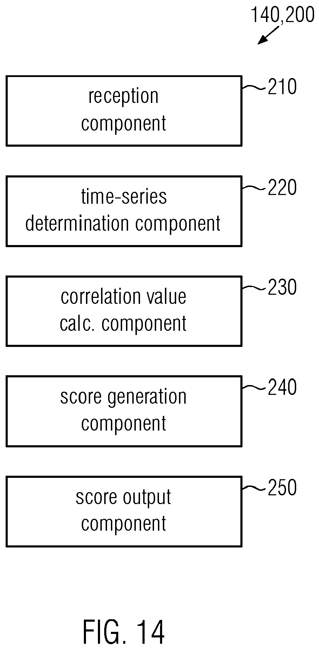

Fig. 14 is a diagram illustrating an example data analysis apparatus according to an embodiment of the present disclosure.

Fig. 15 is a diagram illustrating a brain within a cranial cavity of a subject.

Fig. 16 is a diagram illustrating a method according to an embodiment of the present disclosure.

Fig. 17 is a block diagram showing various exemplary components of an attention detection system for the accurate detection of attention deficiency, according to some embodiments of the present disclosure.

Figs. 18A-B are tabular representations of exemplary tasks for attention deficiency detection, according to some embodiments of the present disclosure.

Fig. 19 illustrates connection networks between regions of the brain, according to some embodiments of the present disclosure.

Fig. 20 is a flowchart depicting operations of an exemplary method for attention deficiency detection, according to some embodiments of present disclosure.

Fig. 21 is a flowchart depicting operations of an exemplary method for attention deficiency detection, according to some embodiments of present disclosure.

## DETAILED DESCRIPTION

Reference will now be made in detail to the exemplary embodiments, examples of which are illustrated in the accompanying drawings, therein like reference numerals reference to like elements throughout.

According to one embodiment probabilities in Bayes' Theorem may be converted into Shannon information contents by applying the -log function on both sides of the equation. As such, a Shannon metric, the Transfer Information Content (TIC), may be defined that may reconcile Shannonian surprise and Bayesian surprise. In addition, the TIC, inside the fields of action selection or decision making, can explain how information flows (e.g., via which pathway or network) to increase or decrease the uncertainty about the action depending on the behavioral relevance of a stimulus. Therefore, the TIC may be a link between belief and knowledge in cognitive modeling.

A breakdown of the TIC may show that two information contents may be generated by a single observation of a stimulus. One of them, called the bottom-up component, may be the information content associated to the evidence, (e.g., the stimulus-bounded surprise that may provoke the occurrence of the mentioned event), which may contribute with positive bits to the information flow; the other one, the top-down component, may be the information content associated to the Bayes' Likelihood, which may contribute with negative informative bits to the information flow. So that, if the latter is smaller in absolute value than the former, this may result in a net positive informative measurement (TIC>0); otherwise, if the negative bits introduced by the top-down component are bigger than the positive bits of the bottom-up, this may result in a net negative informative measurement (TIC<0).

In addition, a conditional TIC may be defined, that can be derived from the conditional Bayes' Theorem, so that it is possible to derive the information flow conditioned on internally maintained context representations (referred to as contextual TIC in the following) or on temporal context (referred to as episodic TIC in the following). The TIC may have the additivity property, and therefore, the total amount of uncertainty reduction may be computed by adding up the successive sensorimotor, contextual and episodic TICs generated during evidence integration, so that the uncertainty about the action selection may be increased or decreased depending on the sign of these TICs, and only when this uncertainty is converted into certitude, a given action may be selected.

According to an embodiment, the insights gained from the TIC may be used for the discovery of an attention network for a cognitive condition, such as a cognitive disorder like Attention-Deficit Hyperactivity Disorder (ADHD). For example, a subject having a cognitive condition such as a cognitive disorder like ADHD may need to increase activity in the prefrontal cortex (PFC) when dealing with an exception from a particular condition (such as an episodic task switching) or when filtering unimportant stimuli, while a healthy subject may show a better performance when dealing with an exception from a condition of when filtering unimportant stimuli by increasing activity of a dorsal-ventral attention network found according to one or more embodiments of the present disclosure, which does not involve an increase in PFC activity. This may explain why a subject having a cognitive condition (like ADHD) may have more difficulties in staying focused, filtering out unimportant information or dealing with an exception (as compared to a healthy subject), because the subject having the cognitive condition may need to use the PFC to filter out unimportant information or when dealing with exceptions, which may be more exhausting as compared to using a direct dorsal-ventral attention network without involvement of the PFC (as a healthy subject may do). Therefore, this network may play a central role in filling the gap between neurobiology and symptoms in ADHD.

As discussed in the following, at least one of activity in the PFC pathway or activity in the the dorsal-ventral attention network may be used as a marker for detecting whether a subject is having a cognitive condition such as a cognitive disorder like ADHD or not.

*Shannonian Brain Model*

It was proposed in the prior art, that neurons code for the log probability that a feature (e.g., of an image), may take on a particular value. That neurons code for log probability was discussed in Rao, R.P.N., 2004, Bayesian Computation in Recurrent Neural Circuits (Neural Comput.16, pages 1 to 38) and in Pouget, A., et al., 2013, Probabilistic brains: knowns and unknowns (Nat. Neurosci. 16 1170-1178). Nevertheless, the way in which the Bayesian brain collapse may take place, passing from a probabilistic brain to certitude or one-shot decisions, is not well understood in the prior art.

According to an embodiment, Shannonian information content or also simply called information content in the following (which may be measured in bits) of an event $x_i$, may be given by:

$$I(x_i) = -\log_2[p(x_i)](bits) \quad \text{(Eq. 1)}$$

The Shannon entropy, also measured in bits, can be expressed as the sum of the information content of each event weighted by its probability of occurrence:

$$H(X) = \sum_{i=1}^{n} -p(x_i) \cdot \log_2[p(x_i)](bits) \quad \text{(Eq. 2)}$$

So, the probability of the event $x_i$ may be equivalent to its information content. The probability may be expressed in terms of the information content, from equation (1):

$$p(x_i) = 2^{-I(x_i)} \quad \text{(Eq. 3)}$$

Shannon explained the amount of information carried by a message as a measure of how surprised one is to learn the message. Thus, it may be natural to apply the same concept to the unaveraged information content given above in equation (1), i.e., it can be stated that this metric may represent, in bits, the surprise that may be provoked by the occurrence of a given event. From equations (1) and (3), it can be seen that the higher/lower the probability of a given event, the lower/higher its information content, i.e., the surprise it provokes.

According to one embodiment it is shown that by expressing classic probability theorems (such as Bayes' Theorem) in terms of Shannon's information contents, it may be possible to understand the way information is transferred, and how classical negative information is generated. Averaging, like in the expression of entropy in equation (2), may give interesting statistical meaning, like the uncertainty about the content of a message, which is in what Shannon was interested for communications purposes, but the accuracy that is obtain by a given measurement may be lost. When an event occurs, probability inference (taking place in the brain) may collapse into a deterministic environment given by Shannon's information contents. As shown further below, this deterministic character of Shannon's information contents may be the bridge between belief and knowledge in cognitive modeling.

According to one embodiment, a Shannon metric may be derived that may reconcile stimulus bounded surprise (such as Shannon surprise) and that may allow the computation of the information gain between prior and posterior beliefs (such as Bayesian surprise). According to this embodiment, Bayes' Theorem is expressed in terms of Shannon's information contents by applying the -log function at both sides of Bayes' Theorem. The effect of data x on an observer may be to change a prior distribution $P(\theta)$ of a model $\theta$ into a posterior distribution $P(\theta|x)$ via Bayes' Theorem:

$$P(\theta|x) = P(x|\theta)P(\theta)/P(x) \quad \text{(Eq. 4)}$$

where P(x) is known as the evidence, and P(x|θ) as the likelihood. Then, taking the $-\log_2$ function on both sides in equation (4) may yield:

$$-\log_2 P(\theta|x) = -\log_2[P(x|\theta)P(\theta)/P(x)] \quad \text{(Eq. 5)}$$

By applying the properties of the log-function the right-hand side of equation (5) can be expanded to obtain:

$$-\log_2 P(\theta|x) = -\log_2 P(x|\theta) - \log_2 P(\theta) + \log_2 P(x) \quad \text{(Eq. 6)}$$

By taking into account equation (1), equation (6) can be expressed in terms of the information contents corresponding to each of the probabilities that appear in Bayes' Theorem:

$$I(\theta|x)(bits) = I(\theta)(bits) - I(x)(bits) + I(x|\theta)(bits) \qquad (Eq.\ 7)$$

In equation (7), each of the terms may be the information content of a certain event, and it may represent, in bits, the surprise that provokes the occurrence of the mentioned event. The interpretation of this last equation may allow understanding how the bits of information, introduced in a space of models, may be transferred to each of them, and concepts like negative information may arise naturally. From equation (7) it can be deduced that the effect of data on the observer may be to change the information content of a given model $\theta$ in the space of models, i.e., it may change (e.g., increases or decreases) the surprise that provokes the occurrence of the mentioned event. Therefore, the term on the left hand side in equation (7), I($\theta$|x), may be called the Posterior Information Content of a single model $\theta$, and the first term of the right hand side in that equation, I($\theta$), may be called the Prior Information Content of a single model $\theta$.

An aspect about equation (7) is that the mentioned change of information content, of a single model $\theta$ in the space of models, that may be provoked by a single observation x, may include two information contents I(x) and I(x|$\theta$) which are generated by the mentioned single observation (and not only one information content as might be expected). Therefore, in equation (7), -I(x)(bits) + I(x|$\theta$)(bits), may represent the information transferred to a single model $\theta$ due to the observation x. The Transfer Information Content (TIC) may be defined as:

$$TIC(x \to \theta)(bits) = I(x)(bits) - I(x|\theta)(bits) \qquad (Eq.\ 8)$$

So that, equation (7) can be expressed as:

$$I(\theta|x)(bits) = I(\theta)(bits) - TIC(x \to \theta)(bits) \qquad (Eq.\ 9)$$

In equation (8) the first term I(x) may be a Shannon's information content associated to the evidence (e.g., it is a Shannonian surprise) and it may contribute with positive bits to decrease the surprise of the Posterior Information Content I($\theta$|x). The second term in equation (8), I(x|$\theta$), may also be a Shannon information content, but associated to the Bayes' likelihood, so that it may be at the same time Bayesian. This second term may contribute with negative bits to increase the surprise of the Posterior Information Content I($\theta$|x). For example, this may ponder the surprise of the evidence I(x), so that only those bits relevant to the single model $\theta$ may be transferred to it. Therefore, the TIC may allow the computation of the information gain between prior and posterior beliefs (such as Bayesian surprise). Because the Shannon information content associated to the Bayes' likelihood may be the link between the probabilistic and the deterministic frameworks, this component of the TIC may be a candidate to model the Bayesian brain collapse (as further described below).

As already discussed above, the TIC can describe the information flow, thus it is not surprising that its sign may be of interest. In particular, if the negative bits of the information content of the Bayes' likelihood, I(x|$\theta$), do not cancel the positive bits of the information content of the evidence I(x), then the TIC is positive (i.e., there may be a positive net transfer of information); on the contrary, if the negative bits corresponding to I(x|$\theta$) are bigger than the bits of the evidence I(x), then the TIC is negative, and there may be a negative net information transfer. As such, the TIC may overcome the limitation of other information gain models.

According to one embodiment, a conditional TIC can be defined as:

$$Conditional\ TIC = \log_2\left[\frac{P(x|y,\theta)}{P(x|y)}\right] = TIC(x \to \theta|y) = -\log_2 P(x|y) + \log_2 P(x|y,\theta) \quad (Eq.\ 10)$$

The conditional TIC may represent the information transferred to a single model $\theta$ due to the observation x given that the event y has already occurred. As the unconditional TIC of equation (8), the conditional TIC of equation (10) can take positive or negative values.

Using Equation (9) in the context of action selection, the information transfer that triggers or not an action a when the stimulus s occurs may be expressed in terms of the so called sensorimotor TIC (s $\to$ a):

$$I(a|s) = I(a) - TIC(s \to a) \quad (Eq.\ 11)$$

where, s may denote a stimulus, and a may denote an action. Equation (11) shows that, in action selection, the TIC from the stimulus s to the action a may be decreasing (TIC>0) or increasing (TIC<0) the uncertainty about the later. In the extreme case in which a TIC>0 may cancel the a priori uncertainty I(a), then there may be certitude that the action will take place, i.e., I(a|s)=0. In other words, in case the uncertainty about an action is decreased to zero (via the TIC), a decision may be taken to perform the action. As discussed above, the TIC may be the subtraction of two information contents. Adding and subtracting information contents can be completed in a short timescale (e.g., a timescale at which the brain attention operates), as opposed to multiplying and dividing probabilities which may be the case if the brain (such as a Bayesian brain) may not be collapsed onto estimates, (e.g., onto Shannon information contents). Therefore, it may be proposed that the TIC may be the (deterministic) neural response, that may reflect action selection (or decision making). This may bridge the gap between belief and knowledge. Because the net information transfer due to the TIC can be positive or negative, this gap can be reduced or increased, and that in both cases, they may provide accuracy to the previously estimated uncertainty.

The TIC of equation (11) may be broken down as follows:

$$I(a|s) = I(a) - TIC(s \rightarrow a) = I(a) - I(s) + I(s|a) \quad \text{(Eq. 12)}$$

This TIC breakdown may allow to identify and separate a bottom-up component and a top-down component in action selection (or decision making). This breakdown into a bottom-up component and a top-down component may correspond to an attentional model for action selection called Shannonian brain model.

The first component of the TIC, I(s), may represent information content associated to the evidence, (e.g., the stimulus-bounded Shannonian surprise provoked by the stimulus s). Therefore, I(s) may be a bottom-up component in an attentional model, which may generate positive bits of information, and therefore may contribute to decrease the information content of an action, (e.g., it may decrease, or even cancel, its uncertainty). The second component of the TIC, I(s|a), (e.g., the information content associated to the Bayes' likelihood), may represent the negative information generated to compute the Bayesian surprise that may increase the information content of the action (such as increasing its uncertainty). Therefore, I(s|a), may be a top-down component in the attentional model, and thus, proper treatment of top-down attention signals may be given. In other words, a high top-down contribution may cause that an action is not taken.

According to a further embodiment, the information transfer that may trigger or not an action a when the stimulus s occurs in a context c may also be expressed in terms of the TIC. The information content corresponding to the probability of triggering the action a conditioned on the occurrence of a stimulus s in the context c may be given by:

$$I(a|s,c) = -\log_2[P(a|s,c)] \quad \text{(Eq. 13)}$$

Applying the conditioned version of the Bayes' Theorem to the a posteriori probability in equation (13) may give:

$$I(a|s,c) = -\log_2[P(a|s,c)] = -\log_2\left[\frac{P(a|s)P(c|a,s)}{p(c|s)}\right] \quad \text{(Eq. 14)}$$

Applying logarithm rules, may give:

$$I(a|s,c) = -\log_2[P(a|s)] - \log_2\left[\frac{P(c|a,s)}{p(c|s)}\right] \quad \text{(Eq. 15)}$$

The first term in equation (15) may be given by the sensorimotor TIC of equation (11). From equation (10), it can be seen that the second term is a conditional TIC, called the contextual conditional TIC: TIC(c → a|s) and it may represent the bits of information transferred to the action a when a stimulus s occurs in a context c. Therefore,

$$I(a|s,c) = I(a) - TIC(s \rightarrow a) - TIC(c \rightarrow a|s) \quad \text{(Eq. 16)}$$

Now, the sensorimotor TIC and the contextual conditional TIC in equation (16) can be broken down into their two components:

(Eq. 17)

$$I(a|s,c) = I(a) - TIC(s \to a) - [I(c|s) - I(c|a,s)] = I(a) - I(s) + I(s|a) - I(c|s) + I(c|a,s)$$

As for the sensorimotor TIC, the components of the contextual TIC in equation (17), I(c|s) and I(c|a,s), may represent bottom-up and top-down components in action selection, respectively. It may be possible that the sensorimotor TIC has a different sign than the contextual TIC, so that an informative stimulus with positive sensorimotor TIC, when considered in a given context, can generate a negative contextual TIC that compensates the positive sensorimotor TIC, making the stimulus uninformative for that context.

In an additional embodiment the stimulus s may occur in a context c, and in an episode (such as a temporal context) e, and the information transfer that may trigger or not an action a may be computed in terms of the TIC. In this case the information content may be given by:

$$I(a|s,c,e) = -\log_2[P(a|s,c,e)] \qquad \text{(Eq. 18)}$$

Applying the conditioned version of the Bayes' Theorem to the a posteriori probability in equation (18) may yield:

$$I(a|s,c,e) = -\log_2[P(a|s,c,e)] = -\log_2\left[\frac{P(a|s,c)P(e|a,s,c)}{p(e|c,s)}\right] \quad \text{(Eq. 19)}$$

And then, applying logarithm rules, may give:

$$I(a|s,c,e) = -\log_2[P(a|s,c)] - \log_2\left[\frac{P(e|a,s,c)}{p(e|c,s)}\right] \qquad \text{(Eq. 20)}$$

The first term in equation (20) may be given by equations (13) and (16). From equation (10), the second term may be the episodic conditional TIC: TIC(e → a|s,c) and it may represent the bits of information transferred to the action when a stimulus occurs in a given context c and episode e. Therefore, the information content may read:

$$I(a|s,c,e) = I(a) - TIC(s \to a) - TIC(c \to a|s) - TIC(e \to a|s,c) \quad \text{(Eq. 21)}$$

In addition, the sensorimotor TIC, the contextual conditional TIC, and the episodic conditional TIC in equation (21), can be broken down into their two components:

$$I(a|s,c,e) = I(a) - TIC(s \to a) - TIC(c \to a|s) - [I(e|s,c) - I(e|a,s,c)] \qquad \text{(Eq. 22)}$$
$$= I(a) - I(s) + I(s|a) - I(c|s) + I(c|a,s) - I(e|s,c) + I(e|a,s,c)$$

The components of the episodic conditional TIC in equation (22), I(e|s,c) and I(e|a,s,c), may represent bottom-up and top-down components in action selection, respectively. Following the reasoning of the contextual case, it is possible that an uninformative stimulus in the given context may become informative if its episodic conditional TIC in equation (22) is positive and compensates the negativity of the contextual conditional TIC.

From equation (22) it can be seen that uncertainty about an action when a stimulus takes place, in a given context and episode, may be increased or decreased, depending on the bottom-up and top-down contributions of information contents (e.g., of log-probabilities, which may be added and subtracted), as further described in the following. In Equations (21) and (22) I(a) may represent the bits of uncertainty about an action before any stimulus appears, and I(a|s,c,e) is the uncertainty that is left (which, e.g., can be higher or lower than I(a)) depending on the sign of each of the TICs after the stimulus has appeared and a subject has taken into account the context c and, if it applies, the episode e. The first component of each of the three TICs (being I(s), I(c|s), I(e|s,c)) may correspond to the bottom-up contribution of the stimulus s and it may always contribute to decrease the uncertainty of the action (e.g., pressing a button). Because these bottom-up components appear with negative sign in equation 22, i.e., they contribute to decrease the bits of uncertainty about the action (increase its probability of occurrence). They may always appear with positive sign in the TIC formula, that is why they are considered positive information components (e.g., the Shannonian surprises). The opposite can be said about the second components of each of the three TICs (being I(s|a), I(c|a,s), I(e|a,s,c), being the top-down components). They appear with positive sign in equation 22, i.e., they may contribute to increase the uncertainty about the action. Because they appear with negative sign in their TIC, they may be negative information components, and therefore, if they are bigger in absolute value than their respective Shannonian surprises (being I(s), I(c|s), I(e|s,c)), then the correspondent TIC may be negative and its net contribution is to increase the uncertainty about the action (decrease its probability of occurrence). Since these second components of the TICs may contribute to make the stimulus (depending on the context or the episode) behaviourally relevant (e.g., when they are low or zero) or non-behaviourally relevant (e.g., when they are large), they may be considered as the top-down components. For example, a subject having a cognitive condition such as a cognitive disorder like ADHD may have difficulties in distinguishing between behaviourally relevant and non-behaviourally relevant stimuli so that it may be possible that the top-down components of the TIC may be treated differently by this subject (as compared to a healthy subject).

In the prior art respective roles of the dorsal attention network and the ventral attention network are known; on the other hand, how the two networks communicate with each other is still a subject of great debate.

Once a task is explained to a subject, the task may be stored in the memory (i.e., the brain) of the subject. Then the dorsal attention network may send one or more top-down signals through the Middle Frontal Gyrus (MFG), which may correspond to non-targets, distracters or any other stimulus different from targets, to the ventral attention network, which are then stored in a region of the brain called the temporoparietal junction (TPJ) of the brain. The TPJ may include a posterior sector of a superior temporal sulcus (STS), a posterior sector of the superior temporal gyrus (STG), and an inferior parietal lobule (IPL) (as discussed in Corbetta et al., 2008, The Reorienting System of the Human Brain: From Environment to Theory of Mind, Neuron 58, pages 306-324). The inferior parietal lobule includes an angular gyrus (AG) and a supramarginal gyrus (SMG).

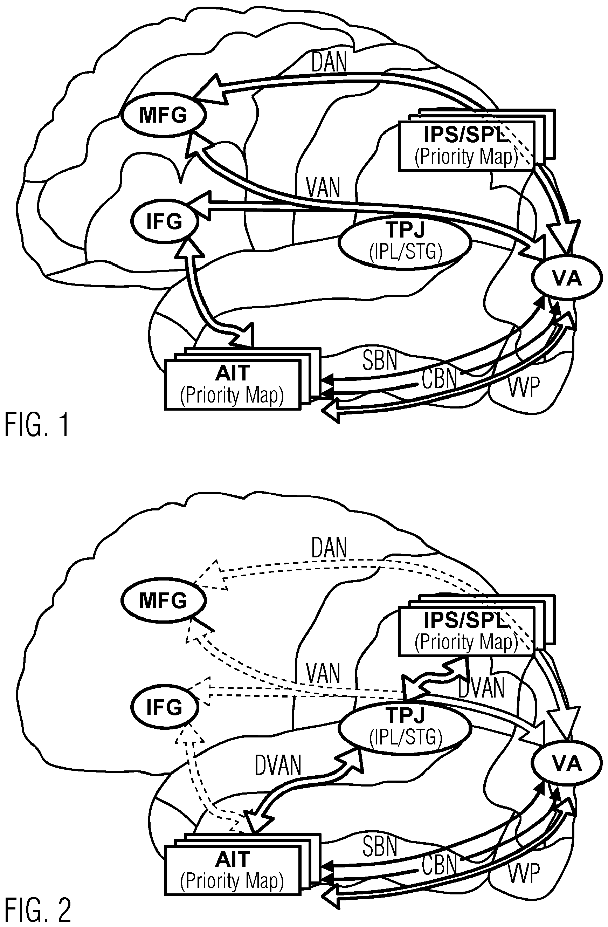

The information flow, of positive and negative bits, that may take place between the dorsal attention network (DAN) and the ventral attention network (VAN), (e.g., inside the Shannonian brain model) is explained in the following with reference to Figure 1. As shown further above, the top-down contribution in the Shannonian brain model may be given by the information content associated to the Bayes' likelihood which may generate negative bits of information that may counteract the bits generated by the evidence (or surprise). Therefore, the TPJ (including, for example, the IPL and the STG) may act as a memory of negative informative bits. This is in accordance with the role of the ventral attention network (VAN), which may only be activated when the stimulus is behaviorally relevant (e.g., one or more targets or contingent distracters) in a given context or episode (e.g., if the stimulus is informative such as when TIC > 0). Then the information content associated with the Bayes' likelihood may be low or zero, so that the bottom up bits (e.g., generated in visual areas of the brain), which may be transmitted to the dorsal attention network (DAN) by the stimulus, may not be counteracted by top down negative bits coming from the ventral attention network, and the stimulus may be prioritized and enhanced in one or more visual cortical areas. On the other hand, if the stimulus is uninformative (TIC ≤ 0) then the information content associated with the Bayes' likelihood may be rather high in absolute value, so that the ventral attention network (VAN) may send, to the dorsal attention network (DAN), a significant amount of negative bits which may counteract the positive bottom up bits of the stimulus. Then, the non-behaviorally relevant stimuli (non-targets, distracters, etc...) may lose priority and, consequently, they may be overridden.

This may be applicable to the sensorimotor, contextual, and episodic TIC's framework (e.g., due to the linearity of equations 21 and 22 in which the contribution of these TICs is considered). Thus, the TPJ may act as a memory of sensorimotor, contextual, and episodic negative informative bits given by I(s|a), I(c|a,s), and I(e|a,s,c) (such as the top-down contributions of the TIC), respectively. In the above discussed Shannonian brain model, these three information contents associated with the Bayes' likelihood may be subtracted, in a priority map brain area, with their correspondent bottom-up contributions of the TIC, i.e., I(s), I(c|s), and I(e|s,c), respectively. Therefore, the sensorimotor, contextual, and episodic TICs may be generated, subtracted from an a priori uncertainty of the action I(a), and an a posteriori uncertainty I(a|s,c,e) may be derived. As described above, different situations depending on the sign of these TICs can arise. For example, the contextual TIC can be negative, making the stimulus uninformative in a context, but if the episodic TIC becomes positive it can counteract the contextual TIC making the stimulus informative in that new temporal context. This is illustrated in Figure 1.

As shown in Figure 1, the TPJ may act as a memory of sensorimotor, contextual, and episodic negative informative bits given by I(s|a), I(c|a,s), and I(e|a,s,c), respectively. These three components may be subtracted, in one or more of two priority map brain areas (e.g., in an Anterior Inferior Temporal Cortex (AIT) and/or in an Intraparietal Sulcus (IPS)/Superior Parietal Lobule (SPL)), from the sensorimotor, contextual, and episodic bottom-up contributions, I(s), I(c|s), and I(e|s,c), respectively. Thus, the sensorimotor, contextual, and episodic TICs may be generated, subtracted from the a priori uncertainty of the action I(a), and the a posteriori uncertainty I(a|s,c,e) may be derived. The dorsal attention network (DAN) and the ventral attention network (VAN) may communicate through the MFG with the involvement of the salience network (SN) (not shown in Figure 1).

The ventral attention network (VAN) may send signals (one or more bottom-up signals, e.g. for re-orientation) to the dorsal attention network (DAN) not only in an activated state, but it may also send signals (negative bits of one or more top-down signals) to the dorsal attention network (DAN) in a deactivated state. Therefore, not only in the activated state, but also in the deactivated state, there may be a flow of information from the ventral attention network (VAN) to the dorsal attention network (DAN). This may be counterintuitive because deactivation and flow of information may be considered as antagonistic concepts, but on the other hand it could help to understand the also counterintuitive concept of negative information because this may be the type of information expected coming from a deactivated source.

In addition, a color-biased network (CBN) is known in the prior art (inside the Visual Ventral Pathway (VVP)) which may include a bilateral lingual gyrus, a fusiform gyrus, a right inferior occipital gyrus, and one or more adjacent occipito-temporo-parietal regions. Posterior regions of this network may be passive and may compute low-level color information (such as color perception), while anterior regions may be active and may encode more complex functions (such as color knowledge) like combining shape and color for object recognition purposes, storing in memory typical colors for given objects and associate them or storing in memory the color and the shape of a given behaviourally relevant stimulus. Due to the relationship of this network to memory aspects, it may be connected to hippocampus-brain structures. These associations are known as object-color memories. Shape and color may be treated separately throughout much of the Visual Ventral Pathway (VVP), in fact a shape-biased Lateral Occipital (LO) network is known. Both networks may converge in a most anterior color-biased region (color knowledge) (e.g., in the Anterior Inferior Temporal (AIT) cortex), where shape and color may be combined for object recognition purposes.

As in the case of other networks, like the dorsal attention network (DAN) and the ventral attention network (VAN), the cognitive functions (color perception, color naming, color knowledge) of the color-biased network (CBN) may be connected through one or more bottom-up (e.g., feed-forward) posterior to anterior pathways, and they may also be top-down modulated (e.g., feed-back) through one or more anterior to posterior pathways. Therefore, irrelevant stimuli may be inhibited in early visual cortex by a top-down modulation based on object-color knowledge encoding. The top-down modulation of the color biased network (CBN) and the shape biased network (SBN), as in the case described above for the dorsal attention network (DAN) and ventral attention network (VAN), may come from filtering signals originated in the prefrontal cortex (PFC) and stored in the TPJ of the ventral attention network (VAN) which activation may be restricted to relevant object-color knowledge. Then, the ventral attention network (VAN) may send this top-down signals to the AIT to regulate the expression of priority object-color knowledge by enhancing or inhibiting the influence of early visual cortex bottom-up inputs.

Again, the sensorimotor, contextual, and episodic TICs framework may find a natural place in this top down modulating role of ventral attention network (VAN) with the color biased network (CBN) and shape biased network (SBN). The TPJ may store the object-color knowledge negative bits correspondent to the sensorimotor, contextual, and episodic information contents associated with the Bayes' likelihood in equation (22), i.e., $I(s|a)$, $I(c|a,s)$, and $I(e|a,s,c)$, respectively. Then, each of these information contents may be subtracted from their respective bottom-up components, $I(s)$, $I(c|s)$, and $I(e|s,c)$, in the AIT to regulate the expression of priority object-color knowledge through the computation of the correspondent sensorimotor, contextual and episodic TICs in equation (21), as also illustrated in Figure 1. If the color of the stimulus is considered a temporal context, or episode, that could make it more or less behaviourally relevant, this would make the sign of the episodic TIC a contributor to the increase or decrease of the uncertainty about the action. Therefore, the ventral attention network (VAN) may be top-down modulating (e.g., inhibiting or reorienting) not only the dorsal attention network (DAN), but also the color biased network (CBN) and shape biased network (SBN).

There may exist an anterior-posterior communication between the dorsal attention network (DAN) and the ventral attention networks (VAN). In particular, frontal areas (such as the prefrontal cortex (PFC)) may be candidates for this interaction. In one example, the Inferior Frontal Gyrus (IFG) and the middle frontal gyrus (MFG) may be candidates for this interaction. In addition, this interaction may be driven by a different network, the Salience Network (SN; not shown in Fig. 1), based on the sustained activation of two of its nodes, the Anterior Cingulate Cortex (ACC) and the Anterior Insula (AI), during top-down filtering or reorienting. The same interplay may apply for the shape biased network (SBN) and color biased network, (CBN) and the ventral attention network (VAN), i.e., that their communication may be driven by the SN through the IFG and the MFG. During cognitive tasks, the SN may have a double role of 'set-maintenance' and of monitoring the decisions made to watch over the task goals. Therefore, the SN may be involved in the anterior-posterior interplay between the ventral attention network (VAN) and the other networks named above.

As shown in Figure 2, in addition to the anterior-posterior communication, a (direct) dorsal-ventral communication (DVAN) may co-exist with the anterior-posterior communication described above in the context with Figure 1. In a dorsal-ventral attention network (DVAN) the TPJ of the ventral attention network (VAN) may top-down modulate (e.g., filtering or reorienting), without the involvement of PFC areas, any one of the dorsal attention network (DAN), the ventral shape network (VVP), and the color biased network (CBN), in order to regulate sensory input. This dorsal-ventral attention network (DVAN) is illustrated in Figure 2. In particular, Figure 2 illustrates the co-exiting dorsal-ventral pathways (DVAN), in addition to the anterior-posterior communication discussed further above. This dorsal-ventral attention network (DVAN) may include one or more of an occipital visual areas, a shape biased network (SBN) and a color bias network (CBN) of the VVP, two priority maps (IPS/SPL and AIT), and posterior areas of the dorsal attention network and ventral attention networks. The TPJ of the ventral attention network may also be a node of this network, which may communicate dorsally and ventrally with the IPS and the AIT priority maps, respectively. This network may be a fast track to handle top-down violations (or filtering irrelevant stimuli) which may compute the sensorimotor, contextual, and episodic TIC's without the involvement of extra PFC resources. In particular, by using a direct link between the IPS/SPL and the TPJ, and/or between the TPJ and the AIT (both indicated with DVAN in Figure 2), information between the dorsal attention network (DAN) and the ventral attention network (VAN) may be exchanged without involvement of the MFG or IFG (as indicated by dashed lines in Figure 2). As such, these direct links may ensure that information can be processed by the brain without the involvement of frontal areas.

*Experimental Results*

A sample of 28 young children, 14 ADHD participants (age 10 years 5 months; sd = 1 year 11 months; females = 35.7%) and 14 control participants (age 10 years 7 months; sd = 1 year 8 months; females = 35.7%) were recruited for a magnetoencephalography (MEG) study. ADHD participants were diagnosed by a neurologist who evaluated them and issued a neurological report with the diagnosis of ADHD. The study was approved by Madrid's Hospital Clinico San Carlos Review Board and every participant and their legal guardians signed an informed consent before MEG recording.

In one example, MEG data including brain signals was measured using a 306-channel (102 magnetometers and 204 planar gradiometers) whole-head MEG Elekta Neuromag system. In one example, brain signals were measured using an online anti-alias band-pass filter between 0.1 and 330 Hz and a sampling rate of 1,000 Hz. In an example, participants' head shape was acquired using a three-dimensional Fastrak digitizer (Polhemus, Colchester, Vermont), in addition to three fiducial points (nasion and left and right preauricular points) as landmarks. In an example, four Head Position Indicator (HPI) coils were recorded on the participants' scalp (forehead and the mastoids). In an example, two sets of bipolar electrodes were used to record eye-blinks and heart beats, respectively.

Data preprocessing was carried out in several steps. In one example, to remove external noise from raw data, the temporal extension of the Space Signal Separation - tSSS method was applied, using a window length of 10 seconds and a correlation threshold of 0.90 as input parameters. In an example, automatic detection of ocular, cardiac and muscle artifacts was performed, which were subject to validation by a MEG expert. Finally, an independent component analysis was performed in one example to remove eye-blink and cardiac activity. In an example, the data was segmented according to the task events into 1-second trials (300 ms of baseline and 700 ms of task-related data), and trials marked as containing artifacts were discarded from subsequent analysis.

In one example, the Montreal Neurological Institute (MNI) template was used to create a homogeneous grid with 1 cm spacing between source positions inside a cranial cavity of the participant, resulting in 2459 source positions located inside the cranial cavity of the participant. In an example, these source positions were labeled according to the Automated Anatomical Labeling atlas (AAL), and only the 1188 source positions labeled as one of 76 cortical areas were maintained. In one example, this surface, together with a source model in subject space, were linearly transformed to match the position of the participant inside the MEG scanner, using the head shape as aid. According to one example the inner skull interface, in combination with the source model described above and the sensor definition provided by the system, were used to solve the forward model and construct a lead field based on a modified spherical solution. In an example, the source-level activity was reconstructed using a Linear Constrained Minimum Variance (LCMV) beamformer as inverse method, using the average of the single-trial covariance matrices for the whole segment, regularized using Tikhonov's method and a lambda factor of 10% of the average channel power. In order to compute a common beamformer filter for magnetometers and gradiometers, in one example the data and the lead field matrix were normalized channel-wise, so the signal amplitudes of different channel types were comparable. In a further example, the three-dimensional time series was projected over the spatial principal component to obtain a single source-level time series per source position.

According to an example, in order to calculate phase synchronization, source level activity was calculated separately for each classical band: theta (4 to 8 Hz), alpha (8 to 12 Hz), low beta (12 to 20 Hz), high beta (20 to 30 Hz), and low gamma (30 to 45 Hz). In an example, band-specific data was filtered using a 1,800th order FIR filter built using Hamming window, and applied in a two-pass procedure, removing any possible phase distortion. In one example, in order to avoid edge effects, the data was filtered using 1 second of real data at each side as padding. It may be understood that for each source position in the cranial cavity of the participant, a respective time-series can be obtained.

In one example, a correlation value (such as Functional Connectivity (FC) analysis) was calculated for pairs of time-series i and j (wherein each time-series corresponds to a respective source position). In one example, FC analysis was calculated by means of the Phase Locking Value (PLV; e.g., under the hypothesis of phase synchronization). The PLV may be based on the distribution of the instantaneous phase difference of two time-depending signals (such as a pair of time-series). If $\varphi_i(t, n)$ and $\varphi_j(t, n)$ are the instantaneous phase for instant $t$ and trial $n$ for signals i and j (such as time-series i and j measured by the MEG device that correspond to a brain signal at respective source positions i and j inside the cranial cavity of the participant), respectively, the value of PLV may be given by:

$$PLV_{i,j} = \frac{1}{N}\sum_{n=1}^{N}\left|\frac{1}{T}\sum_{t=1}^{T}e^{i(\varphi_i(t,n)-\varphi_j(t,n))}\right| \qquad \text{(Eq. 23)}$$

The PLV may take values between 0 and 1, with 0 indicating absolute phase independence (such as low or no correlation) and 1 indicating absolute phase dependence (such as high correlation). In one example, the instantaneous phase of the signals may be estimated using Hilbert's analytical signal after band-pass filtering. In order to avoid edge effects on the extraction of the instantaneous phase, Hilbert's analytical signal may be calculated using one second of real data at each side of the trial as padding according to one example. In one example, $PLV_{i,j}$ was calculated for each frequency band (theta, from 4 to 8 Hz; alpha, from 8 to 12 Hz; low beta, from 12 to 20 Hz; high beta, from 20 to 30 Hz; and low gamma, from 30 to 45 Hz) in a time window from 50 to 450ms after a stimulus.

In one example, $PLV_{i,j}$ was calculated independently for each task (e.g., Task1 and Task2, as further explained below) and for each condition (Go, No-Go and red E vowels, as also further explained below). In one example $PLV_{i,j}$ was calculated for each pair of source positions i and j, generating a 1188 by 1188 FC matrix (i.e., i = 1 ...1188, and j = 1 ... 1188). In another example, the regional-level $PLV_{i,j}$ was calculated as the averaged PLV of all cortical sources contained inside each one of the 76 cortical areas of interest (ROI), e.g. based on an AAL atlas, obtaining a 76 by 76 whole-brain matrix (i.e., i = 1 .. 76, and j = 1.. 76). It is apparent that other sizes for the matrix $PLV_{i,j}$ are possible. In another example, $PLV_{i,j}$ was calculated for specific cortical networks, such as the Salience Network (SN), and the whole, right and left Executive Central Network (ECN). In this case the PLV pairs of ROIs which composed each network were extracted, obtaining subsequent matrices of 8 by 8 ROIs for SN and 14 by 14 for ECN (right/left 7 by 7 ROIs).

For the whole-brain analysis example (discussed above, where i = 1...1188 and j = 1 ... 1188), permutation-based T-test was employed in order to compare PLV values of each pair of ROIs. A T-test independent-samples analysis may be employed for between-groups comparisons within each functional network and for each condition. For intra-group comparisons, related-samples analysis may be performed between pairs of conditions of interest and between similar conditions in both tasks within each group and functional network. In examples, the resulting p-values may be corrected for multiple comparisons (number of pair comparisons and number of frequency bands) with a False Discovery Rate (FDR) of 0.1 resulting in an FDR-corrected alpha threshold for each analysis. In an example, p-values below that thresholds may be reported as significant.

In one example, a Go/No-Go task (called Task1 in the following) in which one or more stimuli such as one or more letters are shown to a participant on a task presentation device, who is instructed to press a button whenever a vowel appears on the device and not press the button when a consonant appears on the device. In this example, the letters may have different colors (such as red, green, blue and white). In addition, an exception is added to the task, so that the participant is instructed to not press the button when the color of the letter is red. After finishing this task, the participant may start a new task (called Task2 in the following) which may add a temporal context or episode in which when the red vowel e is shown, the button should be pressed (such as an exception from the exception). This second task may measure cognitive flexibility and the ability to change the pattern learned during the first task.

**Table 1: Task summary**

| **Task 1** | Red color | Other color |
|---|---|---|
| Consonant | No-Go | |
| Vowel | No-Go | Go |
| **Task 2** | Red color | Other color |
| Consonant | No-Go | |
| Vowel (not 'E') | No-Go | Go |
| Letter 'E' | Go | Go |

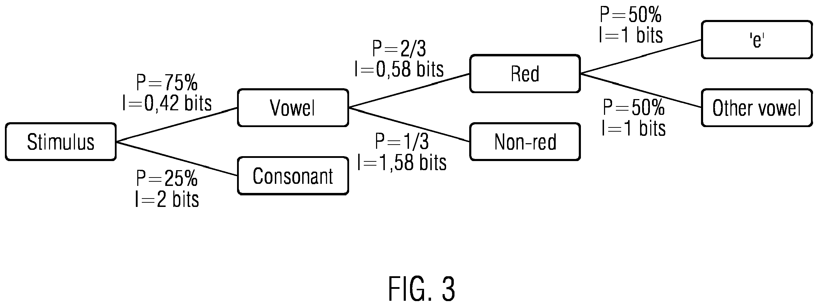

Still in this example, the stimuli may be generated in the following four uniformly distributed groups: GoA = non-red vowel; NoGoA = consonant; GoB = red letter 'e'; andNoGoB = red vowel other than 'e'. The probabilities derived from the uniformly distributed group of stimuli may lead to the following a priori probabilities for the go and no-go conditions on each task. Task 1: Go condition = GoA → P(Go) =1/4; No-Go condition = GoB + NoGoA + NoGoB → P(NoGo) = 3/4. Task 2: Go condition = GoA + GoB → P(Go) = 1/2; No-Go condition = NoGoA + NoGoB → P(NoGo) = 1/2. Figure 3 illustrates the conditioned probabilities of each relevant characteristic according to the model described above. When a certain probability tends to zero, it may be assumed a value of $10^{-5}$ to calculate the associated information, in order to avoid any infinitum.

In the condition of Task1 where a consonant is presented to the participant, there may be no relevant context, therefore, applying equation (12) may lead to:

$$I(a|s = consonant) = I(a) - I(s = consonant) + I(s = consonant|a)$$

Eq. 24

$$= -\log_2 \frac{1}{4} - \left(-\log_2 \frac{1}{4}\right) - \log_2 10^{-5} = 2 - 2 + 16.61 = 16.61 \; bits$$

Therefore, in this sensorimotor case, I(s) « I(s|a) ⇒ TIC(s → a) < 0, i.e., there may be a negative transfer of information from the stimulus of appearance of a consonant to the action a of pressing the button.

Further, in the condition of Task1 where a non-red vowel is presented to the participant, applying equation (17) to the context of a non-red color may give:

$$I(a|s, c) = I(a) - I(s = vowel) + I(s = vowel|a) - I(c = non\_red|s = vowel)$$

$$+ I(c = non\_red|a, s = vowel) = -\log_2 \frac{1}{4} - \left(-\log_2 \frac{3}{4}\right) - \log_2 1 - \left(-\log_2 \frac{1}{3}\right) - \log_2 1$$

Eq. 25

$$= 2 - 0.42 + 0 - 1.58 + 0 = 0 \; bits$$

This means that the uncertainty about the action a may be reduced to zero, and consequently the button may be pressed. In this calculation it was assumed that the task may be performed without any errors. In the real case, the terms I(s|a) and I(c|a,s) will not be exactly zero, which may give explanation of errors committed.

Further, in the condition of Task1 where a non-red vowel is presented to the participant, applying equation (17) to the context of a red color may give:

$$I(a|s, c) = I(a) - I(s = vowel) + I(s = vowel|a) - I(c = red|s = vowel)$$

Eq. 26

$$+ I(c = red|a, s = vowel) = -\log_2 \frac{1}{4} - \left(-\log_2 \frac{3}{4}\right) - \log_2 1 - \left(-\log_2 \frac{2}{3}\right) - \log_2 10^{-5}$$

$$= 2 - 0.42 + 0 - 0.58 + 16.61 = 17.61 \; bits$$

In this case, the component I(c|a,s) may generate negative bits of information that may counteract the ones generated by the evidence representing the top-down control exerted by the subject. To derive the value of the probability P(c|a,s) it can be reasoned like this: suppose that a vowel has appeared and that the button has been pressed, then which is the probability of that vowel to be red. The answer is that this probability may be very low and therefore its associated information content may be very high, (e.g., a large amount negative bits of information) are generated in order to counteract the positive bits generated by the evidence I(c|s). Therefore, in this contextual case, there may be a negative transfer of information from the evidence of appearance of a red vowel to the action a of pressing the button.

In addition, in the episodic condition in Task 2 when the red vowel 'e' is presented to the participant, the button should be pressed and, in this case, although the same context may take place as in the last section (the vowel being red), no top-down control may be generated. Applying equation (22) the values of each previous component may be the same as in the previous case, except I(a) which may be different in Task 2 because of the increase in the frequency of pulsations due to the new episode condition), but two new terms may appear to consider the episodic component information:

$$I(a|s, c, e) = I(a) - I(s = vowel) + I(s = vowel|a) - I(c = red|s = vowel)$$

$$+ I(c = red|a, s = vowel) - I(e|s = vowel, c = red) + I(e|a, s = vowel, c = red)$$

Eq. 27

$$= -\log_2 \frac{1}{2} - \left(-\log_2 \frac{3}{4}\right) - \log_2 1 - \left(-\log_2 \frac{2}{3}\right) - \log_2 \frac{1}{2} - \left(-\log_2 \frac{1}{2}\right) - \log_2 1$$

$$= 1 - 0.42 + 0 - 0.58 + 1 - 1 + 0 = 0 \; bits$$

Again, since the uncertainty about the action may be reduced to zero the outcome is the decision to press the button. Therefore, in this episodic case, I(e|s,c)>>I(e|a,s,c) $\Rightarrow$ TIC(e → a|s,c) > 0, i.e., there may be a positive transfer of information from the evidence of appearance of a red vowel e to the action of pressing the button. Although in this case the contextual TIC may be negative, it may be compensated by the episodic TIC, which is positive and larger than the contextual TIC. There are several contributions to Equation 27:

*Sensorimotor TIC*: the subject sees a vowel, i.e., the Shannonian surprise I(s) which probability of occurrence is 3/4, i.e., 0.42bits of positive information that reduces, momentarily, the uncertainty to 0.58bits; in this sensorimotor stage, the negative information generated by the other component of the TIC, I(s|a), is null and has no impact. The subject may be previously informed of the probability of occurrence of a vowel in this case or he/she may have learnt it during the execution of the task

*Contextual TIC*: the subject may take the context into account, in this case the colour of the stimulus through the bottom-up component of the contextual conditional TIC: I(c|s), this may be again a Shannonian Surprise of value 0.58bits (probability of red vowel 2/3). Then, the subject may take into account the top-down component, (e.g., I(c|a,s) which may introduce 1bit of negative information that may increase the uncertainty about pressing the button, (e.g., may decreases its probability).

*Episodic TIC* so far the 1 bit of initial uncertainty has been reduced with two Shannonian surprises, I(s) and I(c|s), by 1bit precisely (0,42+0,58), but the contextual TIC has introduced, through its top-down component I(c|a,s), 1bit of negative information that has increased the uncertainty to 1 bit again. Therefore, when the subject has processed that the stimulus is a vowel and that it is red, he/she has 1bit of uncertainty. The subject may wait to process if the stimulus is an "e", because this may be the episode condition that should provoke the pressing of the button in task2. The episodic TIC is 1bit: episode TIC(e = red e → a | s,c) = I(e = red E | s = vowel, c = red) - I(e = red E | a, s = vowel, c = red) = 1 - 0 = 1bit. Therefore, the episodic TIC may be, as expected, 1bit, and when subtracted in equation 27, may reduce the uncertainty to 0bits provoking the pulsation the button

The MEG data measured from the participants may include brain signals in the alpha, beta and theta bands. In the following experimental results are discussed in the context of Figures 3 to 12. On the left of each of these Figures, posterior, superior, left, and right views of the brain are shown together with links indicating significantly different PLV-values. On the right of each of these Figures, a circle plot is shows a schematic view of the significant links (in correspondence with the plot on the left). The labels are abbreviations for brain regions in accordance with the automated anatomical labeling atlas shown at the end of the description.

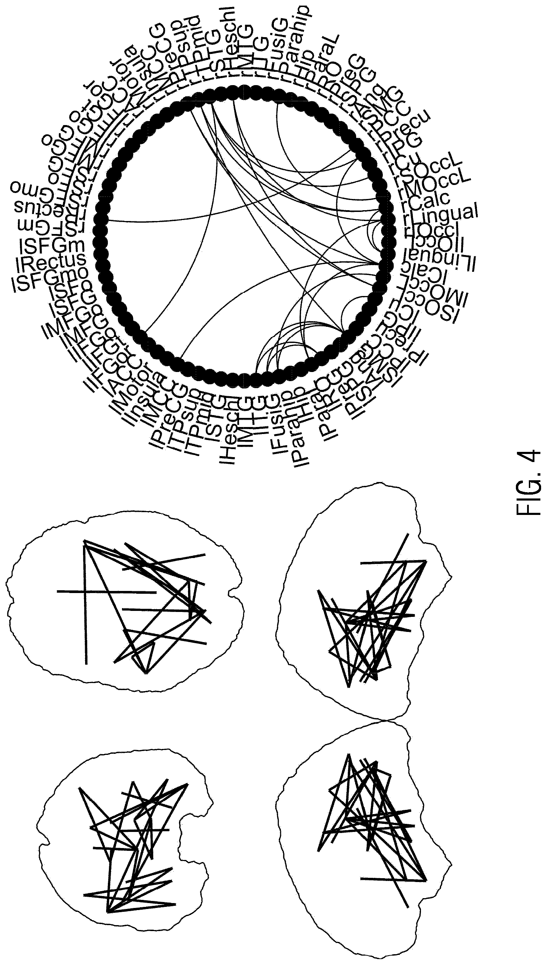

In a first example, the PLV in the healthy condition (HC) group (such as participants who are healthy) doing Task1 in the No-Go condition (consonant or red vowel) may be compared with respect to the Go condition (no red vowel) in the Alpha Band (8-12Hz). Figure 4 shows links with significantly different PLV-values for said first example. It can be observed that in the No-Go condition there is a deactivation of occipito-temporo-parietal and ventral frontal cortex. In particular, Figure 4 illustrates links with significantly different PLV-values in the comparison between the No-Go and Go conditions of the HC group performing Task 1 as lines. On the left, posterior, superior, left, and right views of the brain are shown. On the right, a circle plot shows a schematic view of the significant links. The lines indicate a decreased PLV-value in the No-Go condition with respect to the Go condition. It can be seen that there is a link with significantly different PLV-values between the left supramarginal gyrus (ISMG; an area of the temporoparietal junction), and the left inferior temporal gyrus (IITG, an area belonging to the VVP). This is consistent with the above discussed Shannonian brain model in which the ventral attention network (VAN) may send such top-down signals to the VVP networks to regulate the expression of priority object knowledge by enhancing or inhibiting the influence of early visual cortex bottom-up inputs.

In this case, in the Go condition (no red vowel), equation (25) shows that the two information contents associated with the Bayes' likelihood, I(s = vowel|a) and I(c = non_red|a, s = vowel), are zero. On the contrary, in the No-Go condition if the stimulus is a consonant, equation (24) shows that the information content associated to the Bayes' Likelihood, I(s = consonant|a), is large, and the resultant sensorimotor TIC negative; if the stimulus is a red vowel, equation (26) shows that the contextual TIC is negative due to the not null value of I(c = red|a, s = vowel). In the No-Go condition, the ventral attention network may be deactivated by these negative information content associated to the Bayes' likelihood. In addition, when these negative top-down bits are subtracted to the bottom up bits of corresponding to the Shannonian surprise of the stimulus, a negative TIC may be generated in the anterior inferior temporal which deactivates the VVP, and visual areas in the occipital cortex. This is what can be observed in Figure 4, in addition to the link between the left supramarginal gyrus of the TPJ and the left inferior temporal cortex of the VVP, there are links between the temporal and occipital areas, showing the mentioned deactivation of all these areas in the No-Go condition that may be described by the negative sensorimotor TIC in case the stimulus is a consonant, or the negative contextual TIC in case the stimulus is a red vowel. In addition, the link between the IFG and the middle temporal gyrus may indicate that interplay between the ventral attention network and the shape network is driven by the SN.

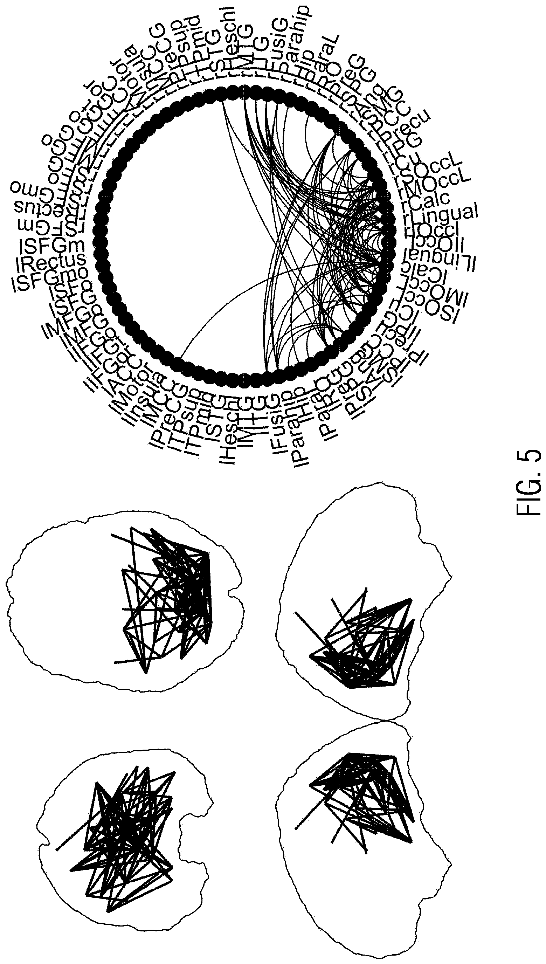

In a second example, the PLV in the Alpha Band (8-12Hz) in the HC Group doing Task1 in the No-Go condition when the stimulus is the red vowel e is compared with respect to Task2 in the Go episodic condition (red vowel e). Figure 5 shows links with significantly different PLV-values for this second example. It can be observed that in the Task1 No-Go condition (red vowel e) there is a deactivation of occipitotemporo-parietal cortex. In particular, Figure 5 illustrates links with significantly different PLV-values in the No-Go condition of Task1 (red vowel e) with respect to Task2 episodic Go condition (red vowel e) for the HC group as lines. On the left posterior, superior, left, and right views of the brain are shown. On the right a circle plot shows a schematic view of the significant links. The lines indicate a decreased PLV-value in No-Go condition of Task1 (red vowel e) with respect to Task2 episodic Go condition (red vowel e). For completeness, the links with significantly different PLV-values for this second example, as shown in Figure 5 are repeated in the following table:

**Table 2: Links shown in Figure 5**

| | |
|---|---|
| lMCC | lCalc |
| lFuisG | rAng |
| lFuisG | rPCC |
| lFuisG | lCalc |
| lFuisG | lSMG |
| lParahip | rAng |
| lParahip | rPCC |
| lParahip | lCalc |
| lParahip | lSMG |
| lHip | lSMG |
| lParaL | rMOccL |
| lRO | lIPG |
| lSPG | rParahip |
| lSPG | rCalc |
| lSPG | lLingual |
| lSPG | lCalc |
| lSPG | lSOccL |
| lSPG | lCu |
| lAng | rMOccL |
| lAng | rCalc |
| lAng | rLingual |
| lAng | lCalc |
| lAng | lSOccL |
| lSMG | rSTG |
| lSMG | lParahip |
| lPCC | lCalc |
| lPrecu | rAng |
| lPrecu | rCu |
| lPrecu | rSOccl |
| lPrecu | rCalc |

(continued)

| | |
|---|---|
| IPrecu | rLingual |
| IPrecu | ICalc |
| IIPG | rMOccL |
| IIPG | ISOccl |
| ICu | rAng |
| ICu | rSOccL |
| ICu | rMOccL |
| ICu | rCalc |
| ICu | rLingual |
| ICu | rIOccL |
| ICu | IMOccL |
| ICu | ISOccL |
| ISOccL | rMTG |
| ISOccL | rAng |
| ISOccL | rPrecu |
| ISOccL | IMOccL |
| ISOccL | ICalc |
| ISOccL | rIOccL |
| ISOccL | rMOccL |
| ISOccL | rSOccL |
| IMOccL | rHip |
| IMOccL | rMOccL |
| IMOccL | ILingual |
| IMOccL | ICalc |
| ICalc | ILingual |
| ICalc | rIOccl |
| ICalc | rLingual |
| ICalc | rCalc |
| ICalc | rMOccL |
| ICalc | rSOccL |
| ICalc | rCu |
| ICalc | rPrecu |
| ICalc | rPCC |
| ICalc | rMTG |
| ICalc | rFusiG |
| ILingual | rIOccL |
| ILingual | rSOccL |
| ILingual | rAng |
| ILingual | FusiG |

(continued)

| lLingual | rMTG |
|----------|----------|
| rIOccL | rMOccL |
| rIOccL | rSOccL |
| rIOccL | rCu |
| rIOccL | rFusiG |
| rLingual | rSOccL |
| rLingual | rCu |
| rLingual | rPrecu |
| rLingual | rParahip |
| rLingual | FusiG |
| rLingual | rITG |
| rLingual | rMTG |
| rCalc | rSOccL |
| rCalc | rCu |
| rCalc | rPrecu |
| rCalc | rFusiG |
| rCalc | rITG |
| rCalc | rMTG |
| rMOccL | rFusiG |
| rMOccL | rITG |
| rSOccL | rPCC |
| rSOccL | rRO |
| rSOccL | rMTG |
| rSOccL | rSTG |
| rCu | rAng |

The PLV results are also consistent with the Shannonian brain model discussed above. In the case of the No-Go condition in Task1, the information content associated to Bayes' likelihood, I(c = red|a, s = vowel), may generate negative bits that may be stored in the TPJ of the ventral attention network, which may then be sent to priority maps areas to counteract bottom-up bits from input signals. In the case of a red vowel, the above discussed Shannonian brain model may predict that negative bits of information corresponding to the term I(c = red|a, s = vowel) may be stored in the TPJ of the ventral attention network, and when the red vowel is presented, they may be sent to inhibit the color-bias network. This is can be observed in Figure 5. There are links with significantly different PLV-values between areas of the TPJ (such as left supramarginal gyrus, bilateral angular gyrus) and color-biased network areas like the bilateral lingual gyrus and fusiform gyrus, and the right inferior occipital gyrus.

In addition, there are links between the superior parietal gyrus (an area belonging to the dorsal attention network in which a priority map has been identified), and occipital visual areas, which also belong to the dorsal attention network. These links may confirm that the top down modulation of occipital visual areas by higher-level regions is occurring in the dorsal attention network as well. In Task2, as opposed to the deactivation of the color-biased network in Task1, as shown in equation (32), the uncertainty about the action a may be completely cancelled by the episodic TIC, so that the color-biased network may be enhanced. Therefore, the differences in PLV values that may be observed in Figure 5 may not only be due a deactivation of the color-biased network in Task1, but to an enhancement of this network in Task2. In addition, Figure 5 shows, that the reconfiguration of mental resources of the task switching implied by the different treatment of the red vowel e in Task1 and Task2 may be achieved without the involvement of the extra PFC recruitment. This result may confirm the postulated dorsal-ventral attention network illustrated in Figure 2 in which the TPJ of the ventral attention network top-down may modulate (e.g., filtering or reorienting), without the involvement of PFC areas, not only the dorsal attention network, but also the ventral shape and color networks, to regulate the sensory input.

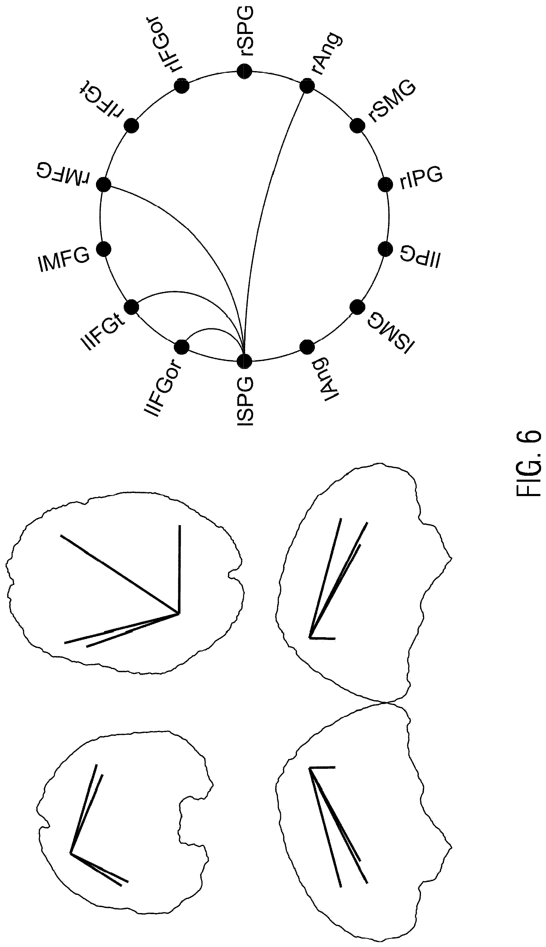

In a third example, shown in Figure 6, the PLV in the Alpha Band (8-12Hz) in the ADHD Group doing Task1 in the NoGo condition when the stimulus is the red vowel e is compared with respect to Task2 in the Go episodic condition (red vowel e). In Figure 6 a Central Executive Network (CEN) is shown for the ADHD group (e.g., the participants having a diagnosis for ADHD). Links with significantly different PLV-values in the No-Go condition of Task1 (red vowel e) with respect to Task2 episodic Go condition (red vowel e) are shown as lines. On the left, posterior, superior, left, and right views of the brain are shown. On the right, a circle plot shows a schematic view of the significant links. The lines indicate a decreased PLV-value in No-Go condition of Task1 (red vowel e) with respect to Task2 episodic Go condition (red vowel e). In Figure 6, links with significantly different FC-values can be seen. In contrast to the HC group, in the ADHD group the reconfiguration of mental resources of the task switching implied by the different treatment of the red vowel e in Task1 and Task2 may be achieved with the involvement of extra PFC recruitment. For example, the areas of the PFC involved are the IFG (e.g., the left inferior frontal triangular and opercular gyrus) and the right middle frontal gyrus, showing a communication between the dorsal attention network and ventral attention networks through the SN. There are three links between these three areas of the PFC and a node of the dorsal attention network, the superior parietal gyrus (e.g., the left superior parietal gyrus; lSPG). In addition, a direct dorsal-ventral communication may be observed in a link between the mentioned left superior parietal gyrus and the right angular gyrus of the ventral attention network.

It can be argued that the dorsal-ventral attentional network (DVAN) discussed above with regard to Figure 2, in which the ventral attention network (VAN) may have the role of communicating dorsally and ventrally with the dorsal attention network (DAN) and the VVP, respectively, may represent a direct path for filtering an irrelevant stimulus, or reorienting the a behaviorally relevant stimulus, much more efficient than the anterior posterior pathway which may involve the PFC and may be driven by the SN through the PFC (such as through the IFG and the MFG; as discussed with regard to Figure 1). Therefore, a subject performing Task1 and Task2 may show the inefficiency of a subject having a cognitive condition (such as ADHD), because such a subject may need to increase the activity of the PFC pathway when dealing with task switching (e.g., with a change in the temporal context or episode, such as an exception to a condition). The HC group may perform in a task switching by increasing the activity of the dorsal-ventral network without increasing the activity of the PFC pathway. It can be argued that Figure 1 may represent ADHD brain connectivity, and that Figure 2 may represent HC brain connectivity, in a reconfiguration of mental resources due to a task switching

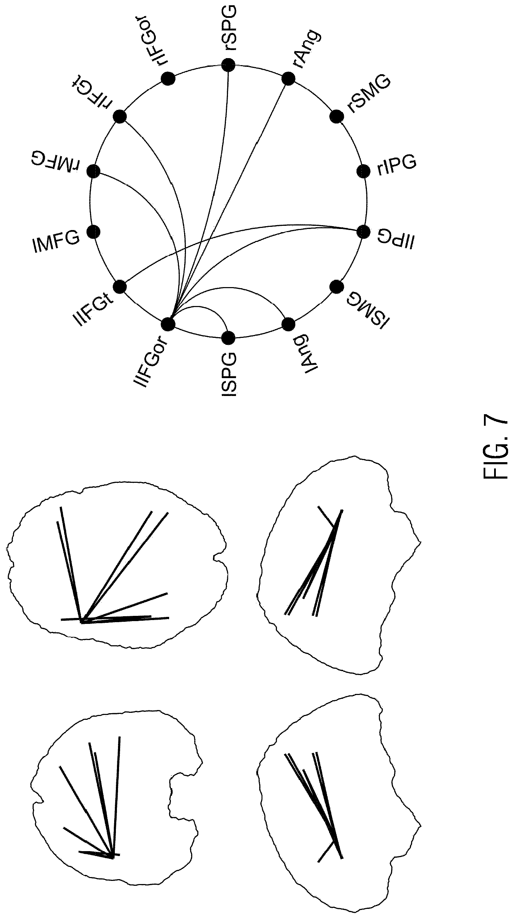

In a fourth example, PLV values in the Go condition for both Task1 and Task2 are compared. In Task1, Go condition (non-red vowel), connectivity results show increased PLV-values in the HC group with respect to the ADHD group in the alpha band (8-12Hz). Thus, the behaviorally relevant stimulus, non-red vowels, may be top-down modulated, and at the same time, the irrelevant stimuli (consonants and red vowels) may be inhibited. Figure 7 shows links of the Central Executive Network with significantly different PLV-values in the Go condition of Task1 (non-red vowel) of the HC group with respect to the ADHD group in the Alpha Band (8-12Hz). On the left, posterior, superior, left, and right views of the brain are shown. On the right, a circle plot shows a schematic view of the significant links. Lines indicate an increased PLV-value in Go condition of Task1 (non-red vowel) of the HC group with respect to the ADHD group. In particular, Figure 7 shows links belonging to the Central Executive Network (CEN), which includes the dorsal attention network and ventral attention network. It can be seen that the PLV-values of the HC subjects with respect to the ADHD group in links between areas of the dorsal attention network (e.g., superior parietal gyrus), the ventral attention network (e.g., bilateral angular gyrus), areas of the IFG (e.g., operocular and triangular inferior frontal gyrus), and middle frontal gyrus are increased. These links show an interplay between the dorsal attention network and the ventral attention networks, through the SN, being the middle frontal gyrus the link between the two. This may imply that the ventral attention network, in the HC group, is sending stronger bottom-up reorienting signals to the dorsal attention network than in the ADHD group.

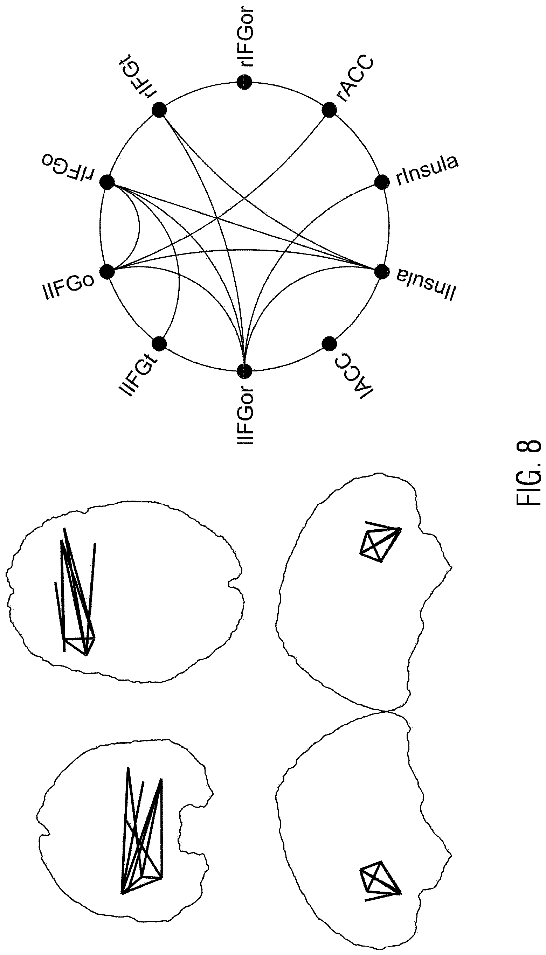

In addition, Figure 8 shows links with significantly different (i.e., increased) PLV-values between the HC and the ADHD groups in the Go-condition (non-red vowel), Alpha Band (8-12Hz). The network shown is the salience network (SN), also known as the cingulo-opercular network. Increased PLV-values of HC with respect to the ADHD group can be observed in links between most of the nodes of the cingulo-opercular network (e.g., bilateral insula, bilateral triangular inferior frontal gyrus, bilateral orbital inferior frontal gyrus, left opercular inferior frontal gyrus, and right anterior cingular cortex). The better cognitive performance in Task1 of the HC group with respect the ADHD group may also be explained by this increased PLV-values in the SN, which may have the role of providing stable 'set-maintenance' over the entire task epoch, and of monitoring the achievement of the desired goals. In particular, Figure 8 shows links of the salience network with significantly different PLV-values in the Go condition of Task1 (non-red vowel) of the HC group with respect to the ADHD group in the Alpha Band (8-12Hz). On the left, posterior, superior, left, and right views of the brain are shown. On the right, a circle plot shows a schematic view of the significant links. Lines indicate an increased PLV-value in Go condition of Task1 (non-red vowel) of the HC group with respect to the ADHD group.

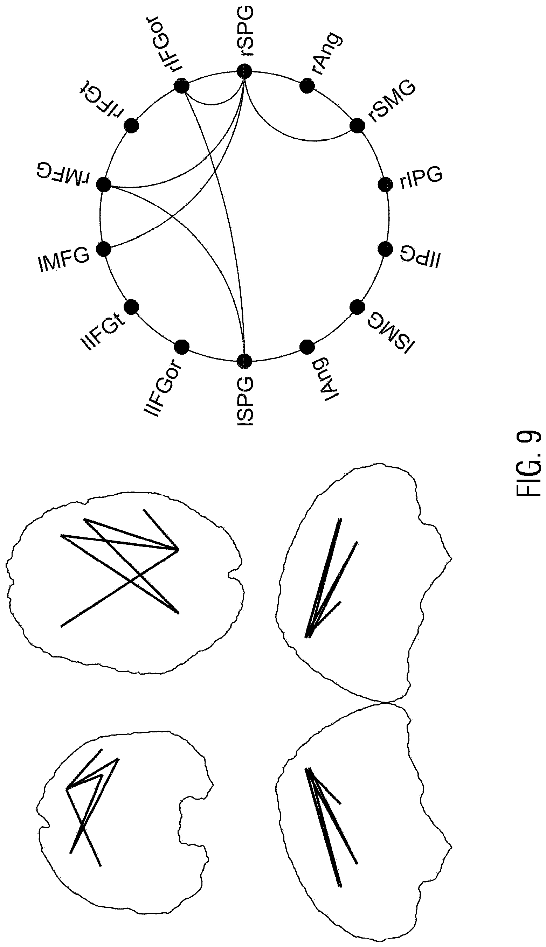

In a fifth example, links in the Beta Band (20-30Hz) with significantly decreased PLV-values in Go condition of Task2 (non-red vowel and red vowel e) of the HC group are compared with respect to the ADHD group. In Task2, in the Go condition, the vowel e may be an episodic top-down violation that may demand behavioral flexibility which may be gained by the decrease in the beta band. Therefore, the high beta band synchronization of the ADHD group may explain the deficit in performance of this group in Task2, shown as a lower percentage of correct hits (86.23% in ADHD vs 92.63% in HC, p-value=0.009) both in non-red vowels (89.93% in ADHD vs 95.00% in HC, p-value=0.017) and in the red vowel e (82.53% in ADHD vs 90.27% in HC, p-value=0.022). Figure 9 shows links of the Central Executive Network (CEN) with significantly different PLV-values in Go condition of Task2 of the HC group with respect to the ADHD group in the Beta Band (20-30Hz). On the left, Posterior, superior, left, and right views of the brain are shown. On the right, a circle plot shows a schematic view of the significant links. Lines indicate a decreased PLV-value in Go condition of Task2 of the HC group with respect to the ADHD group. From the PLV links in Figure 9, it can be derived that the HC group deals with more cognitive flexibility (less beta band synchronization) with the dorsal-ventral connection between the right supramarginal gyrus of the ventral attention network, and the right superior parietal gyrus of the dorsal attention network. Nevertheless, there may be other links connecting areas of the dorsal attention network (e.g., bilateral superior parietal gyrus) with the IFG and the middle frontal gyrus, indicating the involvement of the SN.

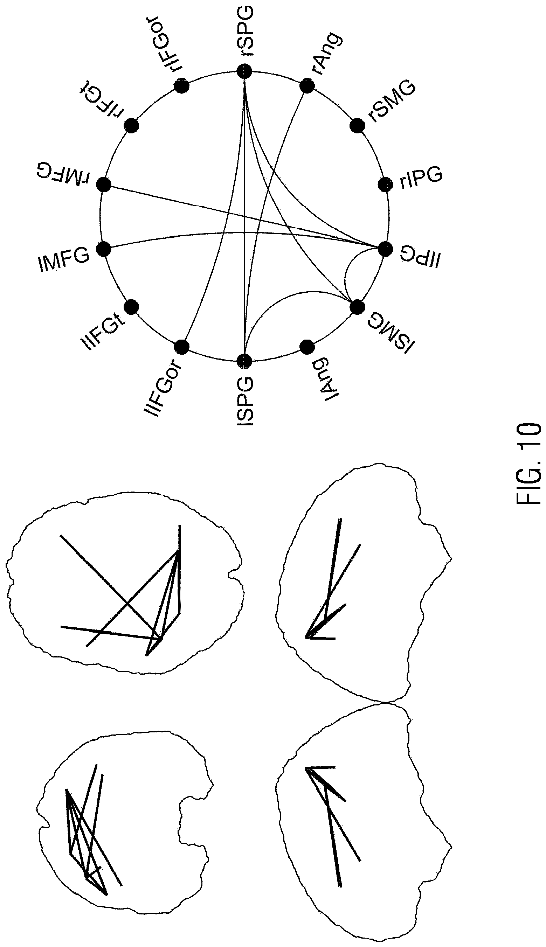

In a sixth example links with significantly different (i.e., increased) PLV-values in Go condition of Task2 of the HC group are compared with respect to the ADHD group in the Theta Band (4-8Hz). Increased PLV-values can be observed between areas of the dorsal attention network (such as a bilateral superior parietal lobule) and areas of the ventral attention network (such as a left supramarginal, right angular gyrus and inferior parietal gyrus). There are shown two links between the inferior parietal gyrus of the ventral attention network and the bilateral middle frontal gyrus. Figure 10 shows links of the Central Executive Network with significantly different PLV-values in Go condition of Task2 of the HC group with respect to the ADHD group in the Theta Band (4-8Hz). On the left, Posterior, superior, left, and right views of the brain are shown. On the right, a circle plot shows a schematic view of the significant links. Lines indicate an increased PLV-value in Go condition of Task2 of the HC group with respect to the ADHD group.

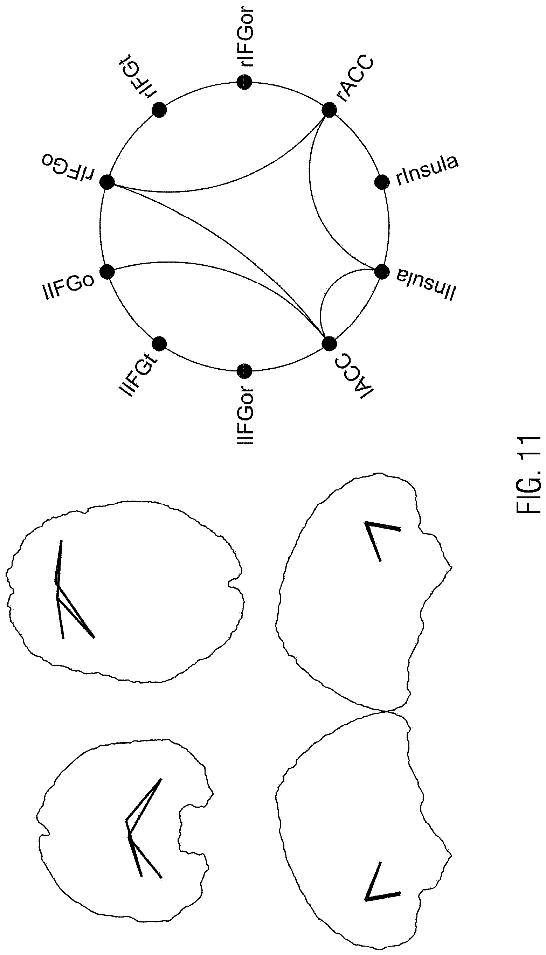

In a seventh example, PLV-values in the No-Go condition for both Task1 and Task2 are considered. Figure 11 shows links with significantly different (i.e., increased) PLV-values, in the alpha band, between the HC and the ADHD groups in the No-Go-condition (consonant or red vowel). As in the previous case the network shown is the SN. Increased PLV-values of HC with respect to the ADHD group may be observed in links between nodes of the SN (e.g., left insula, bilateral orbital inferior frontal gyrus, bilateral anterior cingular cortex). In this No-Go condition, negative informative bits stored in the TPJ may be sent, through the SN, to the dorsal attention network. Therefore, the increased alpha band PLV-values of the HC group may imply a better inhibition of irrelevant stimuli (e.g., a better management of negative information). This may be observed as a higher percentage of hits in the no-go condition for the ADHD group (6.04% in ADHD vs 4.04% in HC, p-value=0.031). Figure 11 shows links of the salience network with significantly different PLV-values in No-Go condition of Task1 (consonant or red vowel) of the HC group with respect to the ADHD group In the Alpha Band (8-12Hz). On the left, a posterior, superior, left, and right views of the brain are shown. On the right, a circle plot shows a schematic view of the significant links. Lines indicate an increased PLV-value in No-Go condition of Task1 (consonant or red vowel) of the HC group with respect to the ADHD group.

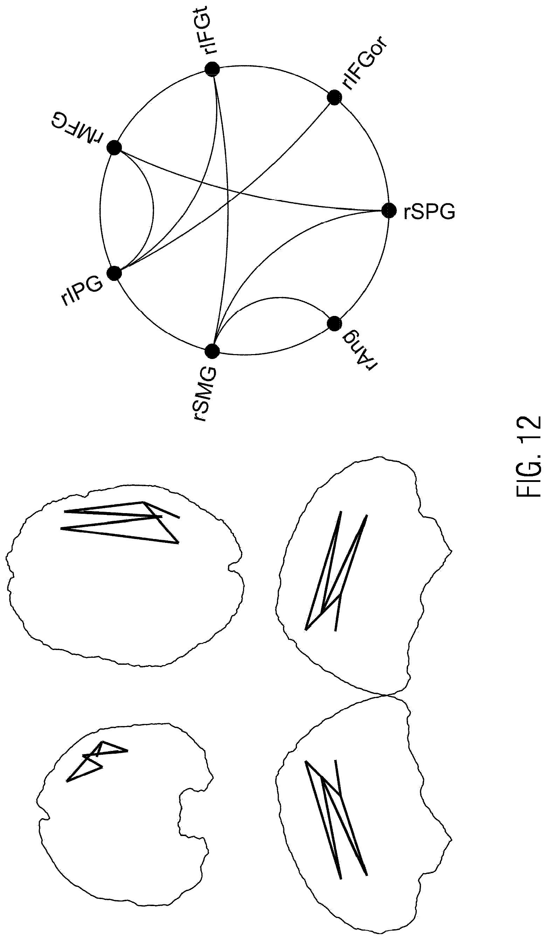

In addition, Figure 12 shows links, in the Beta Band (20-30Hz), with significantly decreased PLV-values in No-Go condition of Task1 (consonant and red vowel) of the HC group with respect to the ADHD group. In Task1, in the No-Go condition, red vowels may represent a top-down violation in which a decrease in the beta-band, to provide cognitive flexibility, may be desirable. This may explain the better performance in Task1 of the HC group with respect to the ADHD group. Again, the links seem to indicate that the HC group deals with more cognitive flexibility (decreased beta band synchronization) the interplay between areas of ventral attention network (right angular gyurs, right supramarginal gyrus), and the dorsal attention network (right superior parietal gyrus) via direct dorsal-ventral pathway, and also, via the SN with the involvement of areas of the IFG and the middle frontal gyrus. In particular, Figure 12 shows links of the Central Executive Network with significantly different PLV-values in No-Go condition of Task1 (consonant or red vowel) of the HC group with respect to the ADHD group In the Beta Band (20-30Hz). On the left, posterior, superior, left, and right views of the brain are shown. On the right a circle plot shows a schematic view of the significant links. Lines indicate a decreased PLV-value in No-Go condition of Task1 (consonant or red vowel) of the HC group with respect to the ADHD group.

As discussed above, Bayes' Theorem may be expressed in terms of Shannon's information contents. By applying the -log-function on both sides of Bayes' Theorem, a Shannon's metric may be defined, called the Transfer Information Content (TIC), which may measure information transferred by a stimulus to reduce (in case of an informative stimulus or TIC>0) or increase (in case of an uninformative stimulus or TIC<0) an uncertainty about an action selection. The informative or uninformative character of the stimulus may depend on its behaviorally relevance, for example, in a sensorimotor type cognitive task like the one discussed above. The TIC may be a function of Shannon information contents, and at the same time may allow the Bayesian model update by measuring the uncertainty increase or decrease about the action uncertainty (e.g., it may reconcile Shannon and Bayesian surprises).

Significantly decreased HC group PLV-values, in the Alpha Band (8-12Hz), in the No-Go condition of Task1 (consonant or red vowel) with respect to PLV-values in the Go condition (non-red vowel) may indicate that the ventral attention network is deactivated by the negative information content associated to the Bayes' likelihood. In addition, when these negative top-down bits are subtracted from the bottom-up bits corresponding to the Shannonian surprise of the stimulus, a negative TIC may be generated in the anterior inferior temporal which deactivates the VVP, and visual areas in the occipital cortex. Interestingly, one of the areas with significant different FC-values between the No-Go and Go conditions is the left fusiform gyrus, an area which responds significantly more to letters than digits. This is consistent with the nature of Task1 in which the stimuli are letters.

In addition, the HC group PLV-values were compared, also in the Alpha Band (8-12Hz), in the HC Group doing Task1 in the No-Go condition when the stimulus is the red vowel e with respect to Task2 in the Go episodic condition (red vowel e). It may be observed that in the Task1 No-Go condition (red vowel e) there is a deactivation of the color-biased network with respect to the same stimulus in Task2, in which the uncertainty about the action a is completely cancelled by the episodic TIC, so that the mentioned color-biased network may be enhanced. It may be observed that, not only the color-biased network, but the whole of the dorsal-ventral attention network discussed above may be activated by the HC group to manage the top down violation that the red vowel e implies in Task2.

The above results may show the inefficiency of ADHD subjects as they need to increase the activity in the PFC when dealing with task switching (such as an exception to a condition), in this case, with a change in the temporal context or episode. On the contrary, the HC group may perform in a task switching by increasing the activity of the novel temporoparietal dorsal-ventral attention network without increasing the activity of the PFC pathway. A decrease of PLV-values of the ADHD group with respect to the HC group is observed, in the Go and in the No-Go conditions in the alpha band, in links that may indicate an interplay between the dorsal and the ventral attention networks through the SN. Therefore, the ventral attention network, in the HC group, may send stronger reorienting (Go condition) or filtering (No-Go condition) signals to the dorsal attention network, than in the ADHD group. In addition, again in links showing the interplay between the dorsal and the ventral attention networks through the SN, significant increased PLV-values can be observed in the ADHD group with respect to the HC group in the Beta Band (20-30Hz) in the No-Go condition in Task1 (red vowel) and in the Go condition in Task2 (red vowel e). These two conditions may imply a top-down violation which requires certain cognitive flexibility (e.g., a low beta band synchronization).

*System, computer-readable medium and method for generating a score indicative of a cognitive condition*

In view of the above considerations and experiments it has been realized that a system, computer-readable medium and method can be provided that may be suitable for generating a score that is indicative for a subject (such as a person) having a cognitive condition, such as a cognitive disorder like ADHD. In particular, the above-discussed results show that information in the brain may be transferred via different pathways (or networks) in case the subject is having a cognitive condition (such as a cognitive disorder like ADHD) as compared to a healthy subject. A pathway may comprise two positions (or nodes) in the brain between which information is transferred. As such, it is suggested to look for one or more pathways that distinguish (e.g., a pathway that is active for a healthy subject and inactive or less active for a subject having the cognitive condition or vice versa) between healthy subjects and a subject that is having a cognitive condition (such as ADHD) in order to generate a score that is indicative for a subject having such a cognitive condition. According to one embodiment, information transferred via a particular pathway may correspond to a correlation value (such as the PLV discussed above) between two time series measured at respective two source positions located inside a cranial cavity of the subject (the time series being obtained from an EEG or MEG device). These two source positions may be the two positions of a pathway that distinguishes between healthy subject and a subject having the condition as mentioned above. As shown further above, a subject having a cognitive condition such as a cognitive disorder like ADHD may lack the ability to increase activity in the dorsal-ventral network (DVAN) without increasing activity in the PFC pathway (which a healthy subject may have due to the above discussed dorsal-ventral attention network (DVAN) as shown in Figures 1 and 2 and in Figures 5 and 6, for example).

This lack of the ability to increase the activity of the dorsal-ventral network (DVAN) may not only be present for subjects having ADHD, but may also be present for subjects having other cognitive conditions (e.g., conditions that may be associated with the subject having difficulties with staying focused or with switching between mental tasks). As such, the score generated according to the present disclosure may also be indicative for such cognitive conditions and may assist a physician in diagnosing the subject having such conditions. In addition, the score generated according to the present disclosure may be used when assessing whether a subject is capable to work in certain professions for which staying focused or switching between mental tasks is important (such as pilots, truck drivers, air traffic controllers, etc.).

According to a first embodiment, a correlation value between a pair of time series of source positions inside the cranial cavity of the subject (i.e. in the brain of the subject) that are included in the PFC pathway may be used for generating a score being indicative for the subject having a cognitive condition such as a cognitive disorder like ADHD. As shown above, subjects having such a cognitive condition may have an increased activity in the PFC pathway (e.g., as compared to subjects not having the cognitive condition). According to one embodiment, the PFC pathway may include a frontal lobe (e.g., a first source position of the pathway) of the brain and a superior parietal lobule (e.g., a second source position of the pathway). As such, a correlation value may be calculated (that corresponds to the information transfer) based on a pair of time series including a first time series that was measured at the frontal lobe of the subject and a second time series that was measured at the superior parietal lobule (SPL) of the subject. In one embodiment, the frontal lobe (i.e., the first source position) may include at least one of a middle frontal gyrus (MFG) and an inferior frontal gyrus (IFG).

In a second embodiment, a correlation value between a time series of a source position (inside the cranial cavity of the subject) that is part of the ventral attention network and another time series of a source position (inside the cranial cavity of the subject) that is part of the dorsal attention network may be used for generating a score being indicative for the subject having a cognitive condition such as a cognitive disorder like ADHD. As shown and discussed above, a healthy subject may have the ability to transfer information (directly) between these two source positions (i.e., a healthy subject may use the above discussed dorsal-ventral network (DVAN)), whereas a subject having a cognitive condition such as a cognitive disorder like ADHD may lack this ability. As such, one or more correlation values between pairs of time series between these two source positions may be suitable to generate a score indicative of the cognitive condition (such as a cognitive disorder like ADHD). In one embodiment, the position being part of the ventral attention network may be the temporoparietal junction (TPJ) and the position being part of the dorsal attention network may be the superior parietal lobule (SPL). As discussed above, the TPJ may include a posterior sector of a superior temporal sulcus (STS), a posterior sector of the superior temporal gyrus (STG), and an inferior parietal lobule (IPL). The inferior parietal lobule comprises an angular gyrus (AG) and a supramarginal gyrus (SMG).

In a third embodiment a correlation value between a time series of a source position (inside the cranial cavity of the subject) that is part of the ventral attention network and a position that is part of the ventral visual pathway (VVP) may be used for generating a score being indicative for the subject having a cognitive condition such as a cognitive disorder like ADHD. As shown and discussed above, a healthy subject may have the ability to transfer information between these two positions, whereas a subject having a cognitive condition such as a cognitive disorder like ADHD may lack this ability. As such, a correlation values between a pair of time series of these two positions may be suitable to generate a score indicative of the cognitive condition (such as a cognitive disorder like ADHD). In one embodiment, the position being part of the ventral attention network may be the TPJ and the position being part of the ventral visual pathway may be the inferior temporal gyrus.

It is apparent that not only a single correlation value may be used for the generation of the score. Instead multiple correlation values can be used (such as multiple correlation values from the first, second or third embodiment discussed above). In addition, the above discussed first to third embodiments may be combined such that one or more correlation values obtained according to one of these embodiments may be used together with one or more correlation values obtained from another one or both of these embodiments for generating the score indicative for the subject having a cognitive condition such as a cognitive disorder like ADHD.

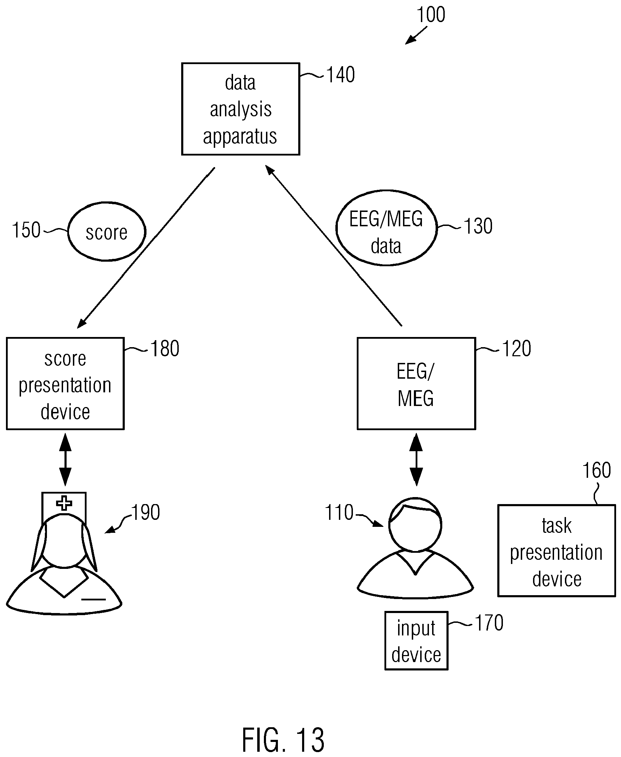

Figure 13 shows an example system 100 that may be used for generating a score 150 that is indicative for a subject 110 having a cognitive condition, such as a cognitive disorder like ADHD. The system 100 may comprise an electroencephalography (EEG) or magnetoencephalography (MEG) device 120 for measuring EEG or MEG data 130, and a data analysis apparatus 140 for generating the score 150. In one embodiment, the system 100 may additionally include a task presentation device 160 for presenting one or more tasks to the subject 110 and an input device 170 for receiving an input from the subject 110 as a reaction of the subject to the presented task. In yet an additional embodiment, the system 100 may further include a score presentation device 180 for presenting the score 150. In one embodiment, the score 150 is presented to a physician 190 who may diagnose whether the subject 110 is having a cognitive condition, such as a cognitive disorder like ADHD.

The EEG/MEG device 120 may be a standard EEG or MEG device as it is known in the prior art. The EEG/MEG device 120 may measure a plurality of signals of respective source positions located inside a cranial cavity of the subject 110. Each source position may correspond to a known brain area, such as a lobe, a lobule, a sulcus or a gyrus. In some examples, the plurality of signals may be measured while the subject is performing one or more tasks that may be presented to the subject 110 on the task presentation device 160. A signal measured by the EEG/MEG device 120 may be divided into one or more bands based on a frequency range of the signal. For example, the signal may include (among others) an alpha band (e.g., in a frequency range of 8 to 12 Hz), a beta band (e.g., in a frequency range of 12 to 30 Hz), and/or a theta band (e.g., in a frequency range of 4 to 8 Hz). In one or more embodiments, a signal may correspond to the measurement of an electrode of an EEG device or to the measurement of a sensor coil of an MEG device, respectively. In some embodiments, the signal may correspond to an aggregated signal (such as an average signal) over the measurements of multiple electrodes (in case of EEG) or multiple sensor coils (in case of MEG). Such an aggregated signal may correspond to an area of the brain that comprises the respective positions of the signals that are being aggregated in the aggregated signal.

The data analysis apparatus 140 is configured to receive EEG/MEG data 130 and to generate a score 150 that is indicative for a subject 110 having a cognitive condition, such as a cognitive disorder like ADHD. The data analysis apparatus 140 may be located on the same site (e.g., on premise) as the EEG/MEG device 120 (e.g., the data analysis apparatus may be a computing system located at the hospital where the EEG/MEG device 120 is located) or the data analysis apparatus 120 may be located elsewhere (e.g., off premise such as in the cloud) and may communicate with the EEG/MEG device 140, the input device 170 and/or with the score presentation device 180 via a network (such as the Internet). Further details about the data analysis apparatus 140 will be discussed further below with regard to Figure 2.

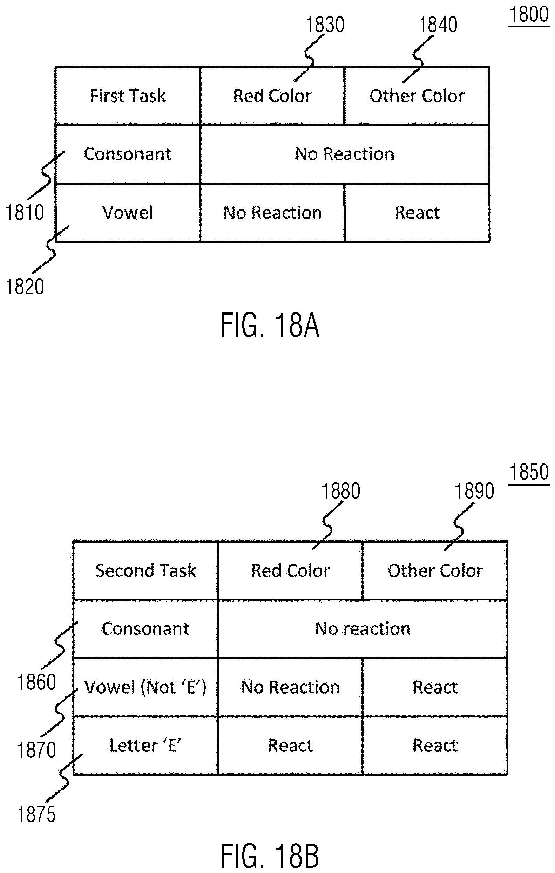

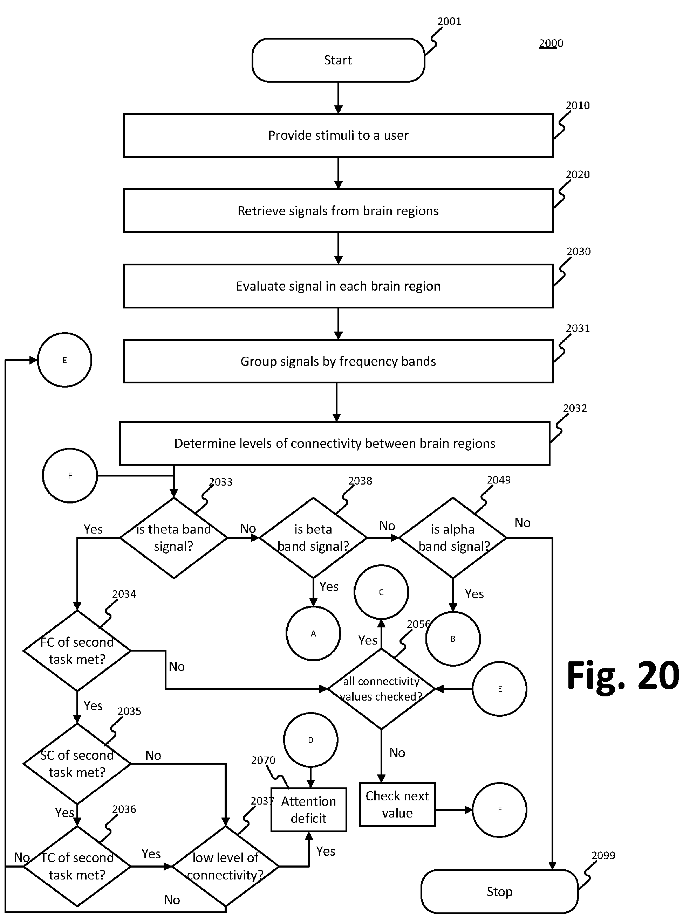

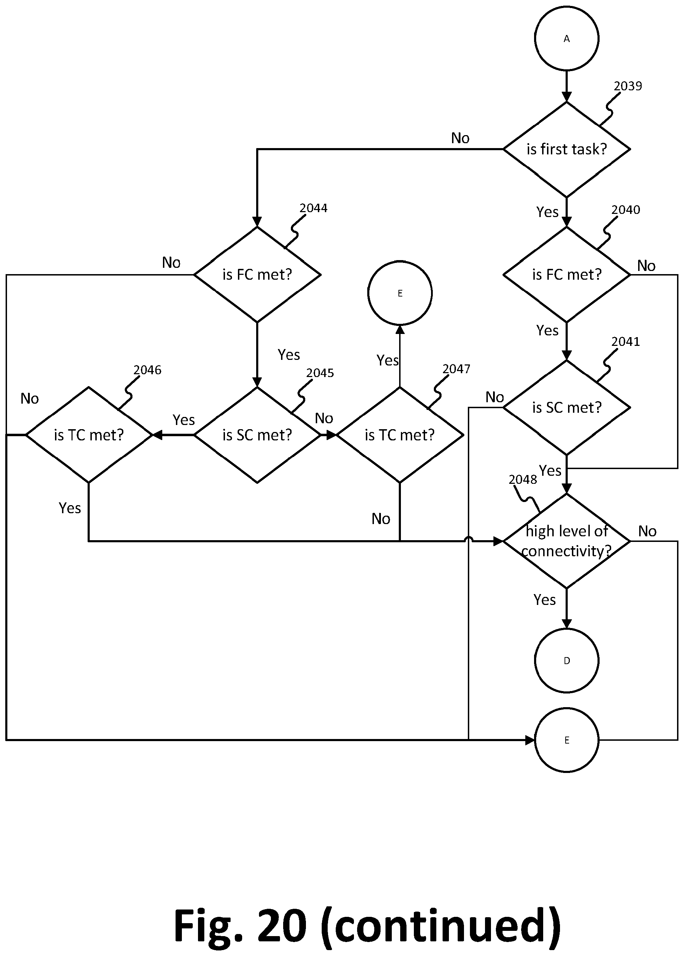

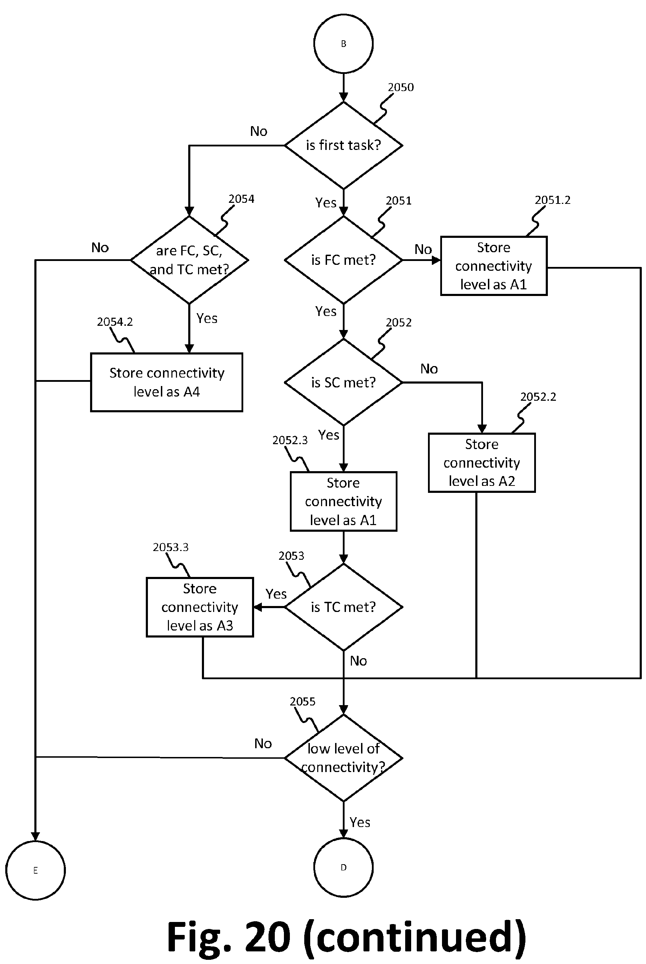

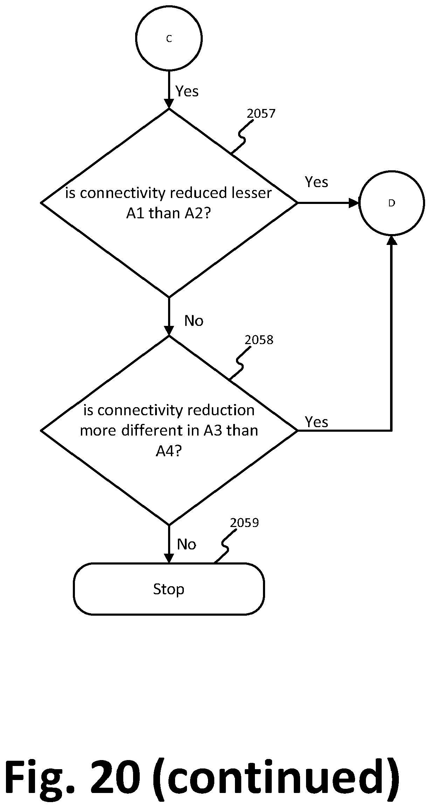

The task presentation device 160 may present one or more tasks to the subject 110 while the EEG/MEG device 120 is measuring brain signals of the subject 110. In one embodiment, the task presentation device 160 may present a first task for a first period of time during which the MEG/EEG device 120 is measuring brain signals of the subject 110. The first task may include three conditions: a first condition, a second condition, and an exception from the second condition (exceptional condition). Each condition may comprise one or more stimuli s and corresponding actions a to be performed by the subject 110 when a stimuli of the one or more stimuli is presented to the subject 110. In an example, the subject 110 may be instructed to: not interact with the input device 170 (such as by not pressing a button) when the first condition is presented during the first task; interact with the input device 170 (such as by pressing a button) when the second condition is presented during the first task; and not interact with the input device 170 (such as by pressing a button) when the exception to the second condition is presented. In one embodiment the first task could be the Task1 example discussed further above (in the context of Figure 3) and as further detailed below in the context of Figures 18 and 20.

In addition, the task presentation device 160 may present a second task for a second period of time (the second period of time being after the first period of time) during which the MEG/EEG device 120 is (still or again) measuring the brain signals of the subject 110. In one embodiment, the second task may include three conditions as the first task discussed above and an additional fourth condition that is an exception from the exception from the second condition so that the subject 110 may be instructed to interact with the input device when the fourth condition occurs during the second task. In an example, the second task may include a first condition, for which the subject 110 is instructed not to interact with the input device 170 and a second condition, for which the subject 110 is instructed to interact with the input device 170. In addition, the second task may include a third condition that defines an exception from the second condition, for which the subject 110 is instructed not to interact with the input device 170. In addition, the second task may include a fourth condition that defines an exception from the exception from the second condition, for which the subject 110 is instructed to interact with the input device 170. In one embodiment the second task could be the Task2 example discussed further above. The task presentation device 160 may be any device that is suitable for presenting the one or more tasks, such as a screen (e.g., a screen of a tablet or of a computing device).

The different conditions of the first task and second task may occur multiple times during the first and second time period, respectively, such as periodically (e.g., every five seconds). In addition, the different conditions may occur with a pre-defined frequency for each of the different conditions (e.g., each condition may occur with the same frequency, being 1/3 in the first task discussed above or 1/4 in the second task discussed above). The multiple times at which the different conditions are presented by the task presentation device 160 may be matched to the brain signals measured by the MEG/EEG device 120 so that the reaction of the brain to the respective different conditions can be identified. The different conditions (i.e., the three conditions of the first task and the four conditions of the second task discussed above), each may correspond to a respective stimulus for the brain of the subject 110.

The input device 170 may be any device that is capable of receiving an input from the subject 110 via an interaction of the subject 110 with the input device 170. In one embodiment, the input device 170 may comprise a button that can be pressed by the subject 110. In another embodiment, the input device may detect a gesture or a facial expression of the subject 110 that may correspond to an interaction of the subject 110 with the input device 170. The input of the subject 110 received by the input device 170 can be used to determine whether the interaction of the subject 110 with the input device 170 was a correct (or incorrect) action in response to the stimulus of a presented condition of a task presented by the task presentation device 160. As discussed above, the subject 110 may be instructed to interact with the input device 170 in some conditions (such as the second condition and the exception from the exception from the second condition) and not to interact with the input device in other conditions (such as the first condition and the exception from the second condition). The input of the subject 110 may be used to determine whether the subject 110 interacted with the input device 170 in accordance with these instructions (correct response) or not (incorrect response).

The score presentation device 180 may be any device that is capable of presenting one or more scores 150 to a physician 190 who may diagnose whether the subject 110 is having a cognitive condition, such as a cognitive disorder like ADHD. In particular, the one or more scores 150 presented by the score presentation device 180 may be helpful for the physician 190 in diagnosing whether the subject 110 is having a cognitive condition, such as a cognitive disorder like ADHD.

The EEG/MEG data 130 may be sent to the data analysis apparatus 140. In one embodiment, the EEG/MEG data 130 may include the brain signals measured by the EEG/MEG device 120. In one embodiment, the EEG/MEG data 130 may include first EEG/MEG data that may be measured while the subject 110 was performing one task (such as the first task discussed above) and second EEG/MEG data that may be measured while the subject 110 was performing another task (such as the second task discussed above). In one embodiment, the brain signals correspond to raw brain signals (such as the brain signals measured by the EEG/MEG device 120 without pre-processing). In another embodiment, the brain signals may be pre-processed (e.g., by removing external noise or ocular, cardiac and/or muscle artifacts) before the EEG/MEG data 130 is sent to the data analysis apparatus 140. In one embodiment, the EEG/MEG data 130 may comprise all brain signals measured by the EEG/MEG device 120. In another embodiment, the EEG/MEG data 130 may comprise only a subset of the brain signals measured by the EEG/MEG device 120. The subset of brain signals may be brain signals from the positions inside the cranial cavity of the subject 110 discussed above for the first, second and third embodiment, such as from a frontal lobe (e.g., a IFG and/or MFG) and a superior parietal lobe (cf. first embodiment discussed above), from a position that is part of the ventral attention network (e.g., a TPJ) and a position that is part of the dorsal attention network (e.g., the superior parietal lobe; cf. second embodiment discussed above), and/or from a position that is part of the ventral attention network (e.g., a TPJ) and a position that is part of the ventral visual pathway (e.g., an inferior temporal gyrus; cf. third embodiment discussed above).

In one embodiment, the EEG/MEG data 130 may further include information (such as time information) that allows matching the times at which the conditions of a task were presented to the subject 110 by the task presentation device 160 to the brain signals measured by the EEG/MEG device 120. In addition, the EEG/MEG data 130 may include data from the input device 170 being indicative about the interaction of the subject 110 with the input device 170.

Figure 2 shows an example for a data analysis apparatus 140, 200. As discussed above, the data analysis apparatus 140, 200 may be on premise (such as located within a local area network of the EEG/MEG device 120) or off premise (such as located in the cloud). For example, the data analysis apparatus may be a computing system (such as a server). The data analysis apparatus 140, 200 may include a reception component 210, a time-series determination component 220, a correlation value calculation component 230, a score generation component 240, and a score output component 250.

The reception component 210 may be configured to receive the EEG/MEG data 130, such as the first and/or second EEG/MEG data discussed above. In one embodiment, the reception component 210 may receive the EEG/MEG data 130 from the EEG/MEG device 120.

The time-series determination component 220 may be configured to determine a first plurality of time series based on the received EEG/MEG data 130 (e.g., based on the first EEG/MEG data that was measured while the subject 110 was performing a task). Each of the first plurality of time series may correspond to a respective source position located inside a cranial cavity of the subject 110. In addition, the time-series determination component 220 may be configured to determine a second plurality of time series based on the received EEG/MEG data 130 (e.g., based on second EEG/MEG data that was measured while the subject 110 was performing another task). Each of the second plurality of time series may correspond to a respective source position located inside a cranial cavity of the subject. For example, the first plurality of time series and the second plurality of time series may correspond to the same respective source positions.

In one embodiment, determining the first (and/or second) plurality of time series may comprise filtering the first (and/or second) EEG/MEG data 130 series such that the first (and/or) second plurality of time series may only include a subset of all available time series (such as a subset of all available time series that can be obtained from the EEG/MEG device 120). The subset may include only time series between the source positions discussed above with regard to the first, second and third embodiment (such as a frontal lobe, a SPL, a TPJ, and/or an ITG). In addition, the filtering may also include filtering out certain frequencies of the EEG/MEG data (such as restricting the time series to the alpha, beta and theta band, to only the alpha band, to only the beta band and/or to only the theta band). Filtering the EEG/MEG data 130 may reduce the amount of data that needs to be processed by the data analysis apparatus 140. For example, filtering the EEG/MEG data 130 may help to get the data needed to be processed for the analysis by the data analysis apparatus 130.

In addition, according to one embodiment, the determining the first (and/or second) plurality of time series may comprise pre-processing the first (and/or second) EEG/MEG data 130, such as by removing external noise, removing ocular, cardiac and muscle artifacts from the EEG/MEG data (e.g., from the brain signals measured by the EEG/MEG device 120), segmenting the EEG/MEG data 130 such that the time series may have the same length (e.g., 1 second). In one embodiment, the time series determination component 220 may be configured to match times at which the conditions of a task were presented to the subject 110 by the task presentation device 160 to the EEG/MEG data 130 (such as to the brain signals measured by the EEG/MEG device 120) so that each of the determined plurality of time series includes a respective starting time of a condition of a task. This may ensure that a reaction of the subject's brain to the condition is included in the time series. In one particular embodiment, the pre-processing may be performed in accordance with the pre-processing example further described above with regard to the experimental results.

It is apparent that the time series determination component 220 may not only determine one time series but a plurality of time series. In particular, and as discussed above, the time series determination component 220 is capable of determining a respective time series for each source position, for each condition of a particular task, and for each subject being examined. The amount of time series to be determined and/or processed may be reduced by considering only a subset of all available time series (as discussed above).

The correlation value calculation component 230 may calculate a correlation value based on pairs of time series that are included in the plurality (such as the first or second plurality) of time series determined by the time series determination unit 220. In particular, in one embodiment, the correlation value calculation component 230 may calculate a first correlation value for a first pair of time series, the first pair of time series being included in the determined first plurality of time series. In addition, the correlation value calculation component 230 may calculate a second correlation value for a second pair of time series, the second pair of time series being included in the determined first plurality of time series. In addition, the correlation value calculation component 230 may calculate a third correlation value for a third pair of time series, the third pair of time series being included in the determined first plurality of time series. In addition, the correlation value calculation component 230 may calculate a fourth correlation value for a fourth pair of time series, the fourth pair of time series being included in the determined second plurality of time series. In one embodiment, the correlation value may be associated with a phase of the pair of time series (e.g., the correlation value may be given by the PLV as discussed above or by a circular correlation coefficient). In another embodiment, the correlation value may be associated with an amplitude of the pair of time series (e.g., the correlation value may be given by a Pearson correlation).

The score generation component 240 may generate one or more scores 150 based on the first correlation value, the one or more scores being indicative for the subject 110 having a cognitive condition, such as a cognitive disorder like attention deficit hyperactivity disorder, ADHD. In one embodiment, each of the one or more scores 150 could be given by a respective correlation value as calculated by the correlation value calculation component 230. In one embodiment, a score could be the correlation value for a time series corresponding to the frontal lobe and a time series corresponding to the SPL (being a first pair of time series). Another score could be the correlation value for a time series corresponding to the SPL and a time series corresponding to the TPJ (being a second pair of time series). Yet another score could be the correlation value for a time series corresponding to the TPJ and a time series corresponding to the ITG (being a third pair of time series). In one embodiment these scores could be combined into a single score, while in another embodiment all three scores could be output.

In accordance with the considerations (such as the TIC, its top-down contributions, and/or the dorsal-ventral attention network (DVAN)) and the experimental results discussed above, a high correlation value between the time series corresponding to the frontal lobe and a time series corresponding to the SPL may indicate that the subject is having a cognitive condition, such as a cognitive disorder like ADHD (because due to the lack of dorsal-ventral communication, the PFC pathway may be increased). Likewise, a high correlation value between a time series corresponding to the TPJ and a time series corresponding to the SPL may indicate that the subject is not having the cognitive condition (because the dorsal-ventral communication may be active). Further, a high correlation value between a time series corresponding to the TPJ and a time series corresponding to the ITG may indicate that the subject is not having the cognitive condition (because the dorsal-ventral communication may be active). A physician being presented with the one or more scores (such as one or more of the three scores discussed above) may use the scores for diagnosing whether the subject is having a cognitive condition, such as a cognitive disorder like ADHD.

In addition or alternatively, the one or more scores 150 may be generated based on comparing the correlation value calculated by the correlation calculation component 230 to one or more other values (e.g., obtained from second EEG/MEG data). In particular, a comparison value may be calculated between the correlation value and the other value and the score may be generated based on the comparison value.

In one embodiment, the correlation value can be compared to another correlation value that is obtained for another pair of time series from the same source positions, the same subject and the same task, but where the subject 110 may be being presented another condition of the task (e.g., via task presentation device 160). For example, the condition for the correlation value may be the second condition of the first task discussed above and the another condition for the another correlation value may be the exception from the second condition of the first task discussed above. In another example, the condition for the correlation value may be the exception from the second condition of the second task discussed above and the another condition for the another correlation value may be the exception from the exception to the second task as discussed above. The score generation component 240 may be configured to calculate a comparison value between these two correlation values. In one example, the score 150 may be the comparison value. In this case, the score may be indicative for the subject's capability to deal with exceptions. The another correlation value may be calculated by the correlation value calculation component 230 (e.g., based time series from second EEG/MEG data) in the same way the correlation value is calculated.

In another embodiment, the correlation value can be compared to another correlation value that is obtained for another pair of time series from the same source positions, the same subject, the same condition, but where the subject 110 is performing another task (e.g., presented via the task presentation device 160). For example, the task for the correlation value may be the first task as discussed above (where the exception to the exception from the second condition is not present) and the another task may be the second task as discussed above (where the exception to the exception from the second condition is present). As discussed above, the second task may be performed by the subject after the first task so that the subject needs to switch, e.g., from not interacting with the input device 170 in the first task, to interacting with the input device 170 in the second task (although the task presentation device 160 may present the same stimulus). The score generation component 240 may be configured to calculate a comparison value between these two correlation values. In one example, the score 150 may be the comparison value. In this case, the score may be indicative for the subject's capability to deal with switching from one task to another task (i.e., re-orientation). The another correlation value may be calculated by the correlation value calculation component 230 (e.g., based time series from second EEG/MEG data) in the same way the correlation value is calculated.

In yet another embodiment, the correlation value can be compared to another correlation value that was obtained for another pair of time series from the same source positions, the same condition and the same task, but for at least one other subject that is known to have the cognitive condition such as a cognitive disorder like ADHD. This another correlation value could be stored in a data store of the data analysis apparatus 140, 200 (not shown in Figure 2). Likewise, the correlation value can be compared to another correlation value that was obtained for another pair of time series from the same source positions, the same condition and the same task, but for at least one other subject that does not have the cognitive condition such as a cognitive disorder like ADHD. Again, this another correlation value could be stored in the data store of the data analysis apparatus 140, 200.

In addition, the score may be generated based on information from the input device 170 that is indicative on whether the subject 110 interacted with the input device correctly in response to a condition of a task or not. In one example, a high number of incorrect responses may be indicative for the subject having the cognitive condition.

Each of the above discussed scores can be output separately as the one or more scores 150, for example to assist a physician 190 in diagnosing the subject 110. Alternatively, a combined score could be generated (e.g., based on a weighted average of the above discussed scores) and the combined score could be output to the physician 190.

The score output component 250 is configured to output the one or more scores 150. In one example, the score output component 250 may send the one or more scores 150 to a score presentation device 180 that may present the one or more scores to a physician 190.

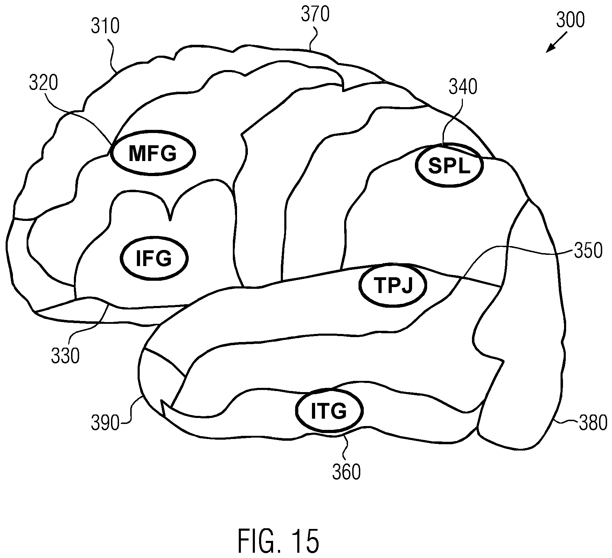

Figure 15 illustrates a brain inside a cranial cavity 300 of a subject 110. The brain may comprise a frontal lobe 310, a parietal lobe 370, an occipital lobe 380, and a temporal lobe 390. As discussed above, subjects having a cognitive condition such as a cognitive disorder like ADHD may lack the ability to activate a dorsal-ventral network (DVAN) that may link a ventral attention network area with a dorsal attention network area and/or a ventral attention network area with a ventral visual pathway area. Instead, subjects having such a cognitive condition may have an increased activity in in a PFC pathway. Therefore, according to an embodiment, the one or more scores indicative for the subject having such a cognitive condition, may be generated based on at least one of: a correlation value between a time series from the frontal lobe 310 (e.g., from the MFG 320 and/or from the IFG 330) and a time series from the SPL 340 of the subject, a correlation value between a time series from the SPL 340 and a time series from the TPJ 350 of the subject, and a correlation value between a time series from the TPJ 350 and a time series from the ITG 360 of the subject. In addition, to any of these three correlation values (which may also be combined as discussed further above), a correlation value for a pair of time series may be used where the source position of the first time series (of the pair of time series) is a position where a link (i.e., any one shown in any one of Figures 4 to 12 starts), and where the source position of the second time series (of the pair of time series) is a position where the same link ends. One example, would be a correlation value between a time series from the left supramarginal gyrus (ISMG) and the left inferior temporal cortex (IITG) shown as a link in Figure 4. Another example, would be a correlation value between a time series from the left superior parietal gyrus (ISPG) and the left inferior frontal gyrus orbital (IIFGo) shown as a link in Figure 6. It is apparent that any other link shown in Figures 4 to 12 could be used to calculate a correlation value to be used for generating the score indicative of the subject having a cognitive condition, as discussed above.

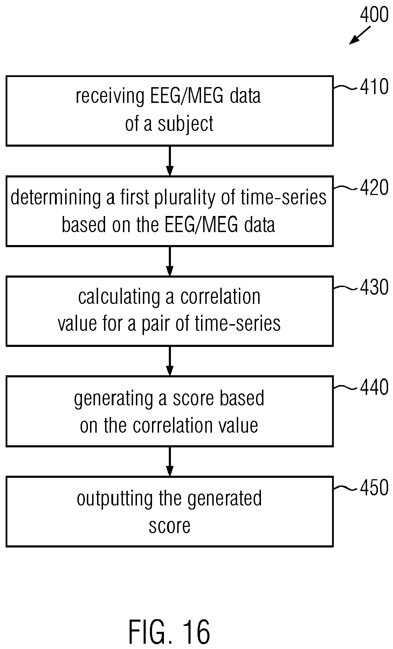

Figure 16 shows a computer-implemented method 400 according to an embodiment of the disclosure. The method steps shown in Figure 16 and discussed in the following may also be implemented as computer-readable medium that causes a computing system (such as data analysis apparatus 140) to carry out these steps. In a first step 410 EEG/MEG data 130 (such as first or second EEG/MEG data) of a subject is received. In a next step 420, a plurality of time series is determined based on the EEG/MEG data. For example, a first plurality of time series is determined based on first EEG/MEG data and a second plurality of time series is determined based on second EEG/MEG data. Each time series of the plurality of time series may correspond to a respective source position inside a cranial cavity 300 of the subject 110. Then, at step 430, a correlation value may be calculated for a pair of time series included in the plurality of time series. At step 440 a score 150 is generated based on the correlation value. The score 150 may be indicative for the subject having a cognitive condition, such as a cognitive disorder like attention deficit hyperactivity disorder, ADHD. Next, at step 450, the generated score is being output.

*Further examples that may be helpful for understanding the invention*

Systems and methods consistent with the present disclosure are directed to efficient and accurate detection of ADHD in individuals. Systems and methods described below include techniques of presenting simple tasks to accurately detect the presence of ADHD and the probability of certainty of ADHD. The invention further identifies a metric for modeling the tasks and calculating the probability of certainty of ADHD. In some embodiments, the disclosed techniques include multiple tasks for presenting content with different conditions that cause a reaction to the content. As described below, specialized tasks with reaction conditions can result in various technological improvements in the accuracy of ADHD detection using the underlying system, hardware, and software, and other applications being executed on the underlying hardware and software.

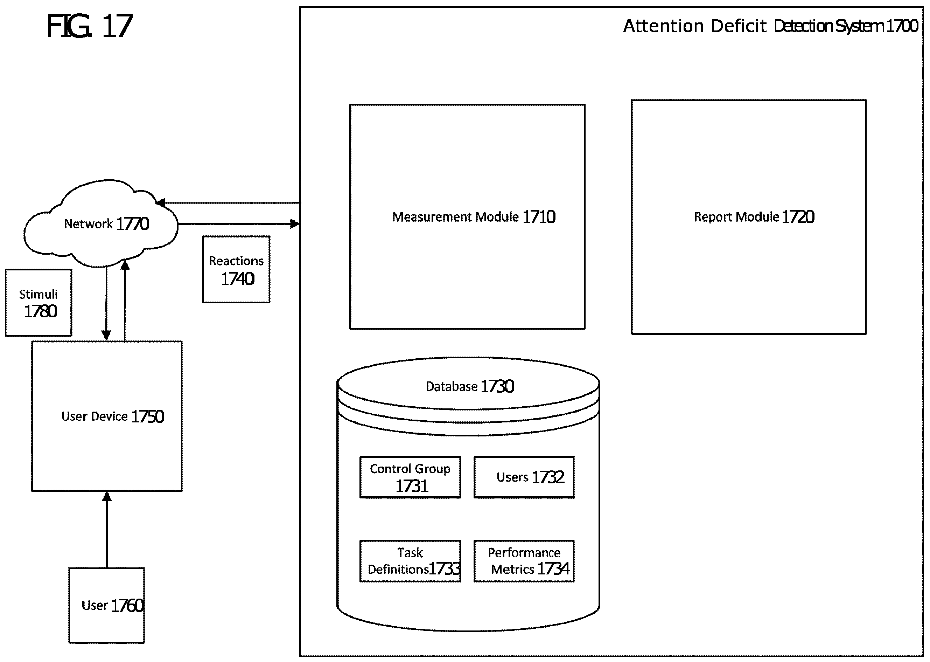

Fig. 17 is a block diagram showing various exemplary components of an attention deficit detection system (ADDS) 1700 for the accurate detection of ADHD, according to some embodiments of the present disclosure. In one embodiment, ADS 1700 may be the system 100 shown in Figure 13 and described above. Detection of ADHD may include evaluating users of ADDS 1700 to detect the presence of ADHD and the percentage of certainty of ADHD. A measure of indication of ADHD may be based on an amount of matches between the user's reactions and the expected results of the tasks. In some embodiments, ADDS 1700 may measure the amount of task reaction deviation from the task reactions defined by a user of ADDS 1700 as part of the initialization of ADDS 1700. A user of ADDS 100 may define attention-level measures of healthy individuals when configuring ADDS 1700. For example, user 1760 may configure ADDS 1700 by supplying a text configuration file to be parsed by ADDS 1700.

As illustrated in Fig. 17, ADDS 1700 may include measurement module 1710 to evaluate the attention levels of individuals and database 1730 to store the evaluated attention levels in performance metrics 1734. Measurement module 1710 may help determine attention levels using data from database 1730. Database 1730 may aid in evaluating attention levels based on control group 1731 associated with users 1732 through task definitions 1733. Report module 1720 may help evaluate the attention levels to determine the attention deficiencies of users 1732 associated with control group 1731. ADDS 1700 may utilize reactions 1740 provided using user device 150 to determine the attention levels of control group 1731 and users 1732. User device 1750 may be a processor or a complete computing device, such as laptops, desktop computers, mobile devices, smart home appliances, IoT devices, etc.

Measurement module 1710 may measure the attention levels of a user (e.g., user 1760) by measuring the the oscillatory signals in different regions of the brain of the user. Measurement module 1710 may measure user 1760's attention levels by determining the level of connectivity between different regions of the brain of user 1760. Measurement module 1710 may measure oscillatory signals upon receiving reactions 1740 from user 1760 from user device 1750. ADDS 1700 may be configured to allow measurement module 1710 to collect signals from different brain regions based on reactions. The configurations may include whether to collect brain signals and when to collect brain signals, and from which regions to collect brain signals. A user of ADDS 1700 may configure measurement module 1710 using a configuration provided using user device 1750 over network 1770.

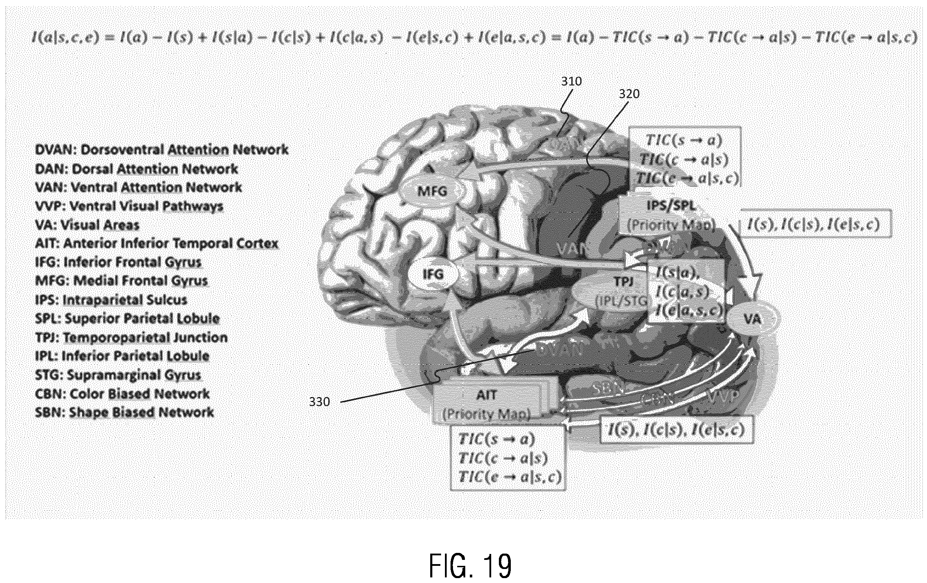

Report module 1720 may evaluate the measured signals to determine the presence of ADHD and calculate the percentage of certainty of ADHD. Report module 1720 may evaluate signals by measuring signal connectivity. In some embodiments, report module 1720 may evaluate signals by comparing determined levels of connectivity. A detailed description of various evaluation techniques is provided in Fig. 19 and Fig. 20 descriptions below.

In various embodiments, database 130 may take several different forms. For example, database 1730 may be an SQL database or an NoSQL database, such as those developed by MICROSOFT™, REDIS, ORACLE™, CASSANDRA, or MYSQL, or various other types of databases, including data returned by calling a web service, data returned by calling a computational function, sensor data, IoT devices, or various other data sources. Database 1730 may store data that is used or generated during the operation of applications, such as data generated by measurement module 1710. For example, if measurement module 1710 is configured to evaluate reactions 1740, then it may access task definitions 1733 to share stimuli 1780 and measure signals using levels of connectivity upon receiving reactions 1740 and store them as performance metrics 1734. Similarly, if report module 1720 is configured to determine ADHD, then report module 1720 may access performance metrics 1734 to retrieve the evaluated signal levels of connectivity associated with a user (e.g., user 1760) for stimuli against the levels of connectivity of signals for the same stimuli provided to a control group 1731. In some embodiments, database 1730 may be fed data from an external source (e.g., server, database, sensors, IoT devices, etc.). In some embodiments, database 1730 may provide data storage for a distributed data processing system (e.g., Hadoop Distributed File System, Google File System, ClusterFS, and/or OneFS).

Network 1770 may take various forms. For example, network 1770 may include or utilize the Internet, a wired Wide Area Network (WAN), a wired Local Area Network (LAN), a wireless WAN (e.g., WiMAX), a wireless LAN (e.g., IEEE 802.11, etc.), a mesh network, a mobile/cellular network, an enterprise or private data network, a storage area network, a virtual private network using a public network, or other types of network communications. In some embodiments, network 1770 may include an on-premises (e.g., LAN) network, while in other embodiments, network 1770 may include a virtualized (e.g., AWS™, Azure™, IBM Cloud™, etc.) network. Further, network 1770 may in some embodiments be a hybrid on-premises and virtualized network, including components of both types of network architecture.

ADDS 1700 may provide stimuli 1780 to user 1760 of user device 1750 by presenting content matching conditions of task definitions 1733. User 1760 provides reactions 1740 to stimuli 1780 using devices attached to user device 1750. For example, user 1760 of user device 1750 may react to a task presented to user 1760 by clicking buttons or touching a screen to respond to stimuli 1780. Reactions 1740 to stimuli associated with a task may include sharing the answer to the task. For example, a task may include a question that provides a list of reaction options that can be selected by user 1760 in response to the question. In some embodiments, reactions 1740 may include a non-reaction of a user for a task. Non-reactions may be recorded based on no response to stimuli after a certain time period. The time period to determine non-reactions to stimuli may be configurable by the user (e.g., user 1760) of ADDS 1700.

Task definitions 1733 include rules of reaction that may be presented beforehand for user 1760 of user device 150 to react to stimuli 1780 based on rules of a task in task definitions 1733. Rules may include when to react and how to react. In some embodiments, rules may include multiple conditional statements dividing and sub-dividing reaction types and reaction times based on content satisfying a task definition. In some embodiments, a task may include rules which extend the rules of another task.

Figs. 18A-B are tabular representations of exemplary tasks for detection of ADHD and its probabilities, according to some embodiments of the present disclosure. The tabular representations include various response reaction conditions presented as row and column headers 1810-1840 and 1860-1890. As illustrated in Fig. 18A, row headers 1810 and 1820 divide the possible content presented to a user (e.g., user 1760 of Fig. 17) on a user device (e.g., user device 1750 of Fig. 17). The divided content indicates when a user is supposed to react to the content presented as stimuli 1780 on user device 1750. For example, task 1800 divides content into consonants and vowels with a rule to not react when consonants are presented as stimuli. A task may include additional conditions to apply restrictions to reactions to stimuli (e.g., stimuli 1780 of Fig. 17) presented to a user. In task 1800, additional conditions are included as column headers (e.g., column headers 1830 and 1840). The additional conditions may apply restrictions when the first condition for a reaction is met. For example, as illustrated in Fig. 18A, task 1800 includes a second condition of letter color, which limits the reaction for vowels when it is a red-colored vowel.

Tasks may include multiple sub-conditions that add different restrictions or allowances. For example, Fig. 18B presents task 1850 in a tabular form where vowels may include the condition to determine whether it is letter 'E' or other vowels as one condition and whether the color of the letters is 'red' or 'other color' as a second condition.

Reactions are a user's (e.g., user 1760) actions on a user device (e.g., user device 1750). A reaction may include a positive reaction (e.g., clicking a button) and a negative reaction (e.g., clicking a different button). In some embodiments, a negative reaction may be no reaction. For example, in task 1800, when a consonant is displayed (rule 1810), user 1760 is expected to have a negative reaction by not reacting to the displayed letter. A task condition may divide the possible universe of content into active and silent states. Active and silent states of a condition indicate when user 1760 is expected to react or not react to an instance from the universe of content presented as stimuli 1780. For example, as illustrated in Fig. 18A, task 1800 includes the universe of letters as content and is divided into consonants associated with a negative reaction and vowels associated with a positive reaction. In some embodiments, a task may include restrictions on a condition to further restrict the expectation of an active state. For example, in task 1800, condition 1820 may have a limitation to not react when the displayed vowel is displayed in a red color. In some embodiments, the second condition may apply restrictions on content belonging to both active and silent states.

Fig. 18B illustrates a tabular representation of task 1850 with additional conditions for reversal of restrictions on active state content. As illustrated in Fig. 18B, rules 1860 and 1870 include the active and silent states of the first condition, similar to rules 1810 and 1820 of the first condition in task 1800. Similar to task 1800, task 1850 includes a second condition with rules 1880 and 1890 to place a restriction when the first condition matches the active state. A third condition can split the active state content besides the second condition. The third condition splits the active state content to reverse the restriction placed by second condition rules 1880 and 1890. For example, as illustrated in Fig. 18B, task 1850 includes a third condition to split the vowels content in the active state to letter 'E' and other vowels. When the third condition is positive, the task allows for reversal of the restriction on reaction placed on active state content by the second condition. For example, in task 1850, second condition rule 1880 places a restriction to not react when the vowel is 'Red Color,' but rule 1875 of the third condition reverses this restriction for letter 'E' by allowing reaction.

A user (e.g., user 1760) of ADDS 1700 may be presented with content belonging to the universe of content that meets the first, second, or third conditions as defined in tasks 1800 and 1850 for reaction along with rules of expected reactions. ADDS 1700 may then collect the reactions (e.g., reactions 1740 of Fig. 17) and signals generated in the brain of user 1760 when submitting reactions 1740. Brain signals collected on receiving user 1760's reactions 1740 on display of stimuli 1780 are evaluated to determine the level of connectivity between regions of user 1760's brain from where brain signals are collected. A detailed description of different brain regions and determination of levels of connectivity is described in Fig. 19 description below.

Fig. 3 illustrates conditional probabilities of each output based on a cascade model, according to some embodiments of the present disclosure. The conditional probabilities and information transfer are calculated using the Transfer Information Content (TIC) metrics presented below. For example, using the TIC metric below, in task 1800, when the consonant data is observed by a user (e.g., user 1760 of Fig. 1), the TIC metric calculates the information transfer amount using the TIC metric defined below as follows:

$$I(a|s = \text{consonant}) = I(a) - I(s = \text{consonant}) + I(s = \text{consonant}|a)$$

$$= -\log_2 \frac{1}{4} - \left(-\log_2 \frac{1}{4}\right) - \log_2 10^{-5} = 2 - 2 + 16.61 = 16.61 \text{ bits}$$

In the above scenario, I(s) << I(s|a) $\Rightarrow$ TIC(s $\rightarrow$ a) < 0, i.e., there is a negative transfer of information from stimuli 1780, including a display of a consonant as part of task 1800 that causes a reaction. The negative information bits are an indication of inhibition or user 1760 limiting himself/herself from providing a positive reaction (e.g., clicking a button). As a result, because I(a|s) is much larger than I(a), the outcome is not to press the button.

If vowel data is presented as part of task 1800 to user 1760, then the user 1760 needs to check whether the color of the letter is red (e.g., red vowel) or not. In the case of non-red vowel, the information transfer is calculated using the conditional TIC metric as follows:

$$I(a|s, c) = I(a) - I(s = \text{vowel}) + I(s = \text{vowel}|a) - I(c = \text{non}_{\text{red}}|s = \text{vowel})$$

$$+ I(c = \text{non}_{\text{red}}|a, s = \text{vowel})$$

$$= -\log_2 \frac{1}{4} - \left(-\log_2 \frac{3}{4}\right) - \log_2 1 - \left(-\log_2 \frac{1}{3}\right) - \log_2 1 = 2 - 0.42 + 0 - 1.58 + 0$$

$$= 0 \text{ bits}$$

Both sensorimotor and context TIC are positive and subtracted from the initial uncertainty, which is reduced to zero. Consequently, the button is pressed.

If red vowel data is presented as part of task 1800 to user 1760, then user 1760 needs to check whether the letter is red. Applying conditional TIC to the context of a red color, we have:

$$I(a|s, c) = I(a) - I(s = \text{vowel}) + I(s = \text{vowel}|a) - I(c = \text{red}|s = \text{vowel}) + I(c = \text{red}|a, s = \text{vowel})$$

$$= -\log_2 \frac{1}{4} - \left(-\log_2 \frac{3}{4}\right) - \log_2 1 - \left(-\log_2 \frac{2}{3}\right) - \log_2 10^{-5}$$

$$= 2 - 0.42 + 0 - 0.58 + 16.61 = 17.61 \text{ bits}$$

In this case, the component I(c|a, s) generates negative bits of information that counteract the ones generated by the evidence representing the top-down control exerted by the subject. To derive the value of the probability P(c|a, s), whose information content we are interested in, we can reason like this: suppose that a vowel has appeared and that the user (e.g., user 1760 of Fig. 17) has pressed the button, then what is the probability of that vowel being red? The answer is that this probability is very low and, therefore, its associated information content is very high, i.e., a huge amount of negative bits of information have been generated in order to counteract the positive bits generated by the evidence I(c|s).

Therefore, in this contextual case, there is a negative transfer of information from the evidence of an appearance of a red vowel to the action a of pressing the button.

If red vowel data is presented as part of task 1850 to user 1760, then user 1760 needs to check whether the letter is "E" or not. The information transfer is calculated using an episodic TIC metric as follows:

$$I(a|s, c, e) = I(a) - I(s = \text{vowel}) + I(s = \text{vowel}|a) - I(c = \text{red}|s = \text{vowel})$$

$$+ I(c = \text{red}|a, s = \text{vowel}) - I(e|s = \text{vowel}, c = \text{red}) + I(e|a, s = \text{vowel}, c = \text{red})$$

$$= -\log_2 \frac{1}{2} - \left(-\log_2 \frac{3}{4}\right) - \log_2 1 - \left(-\log_2 \frac{2}{3}\right) - \log_2 \frac{1}{2} - \left(-\log_2 \frac{1}{2}\right) - \log_2 1$$

$$= 1 - 0.42 + 0 - 0.58 + 1 - 1 + 0 = 0 \text{ bits}$$

The positive sensorimotor TIC reduces the uncertainty about the action, but the negative context TIC increases the uncertainty, and the net contribution is zero. Finally, the positive episode TIC further reduces the uncertainty and it is reduced to zero. Consequently, the button is pressed.

The amount of information bits described above can be used as a default value for comparing different users to determine attention levels. ADDS 1700 may begin with these default values as expected signal values and revise them as signals from more individuals considered to be part of the control group are added.

Fig. 19 illustrates connection networks between regions of the brain, according to some embodiments of the present disclosure. ADDS 1700 may identify the presence of signals in various brain regions representing different connection networks when a user (e.g., user 1760 of Fig. 17) provides reactions 1740. ADDS 1700 may use identified signals in connection networks to determine the level of connectivity between different regions of the brain. ADDS 1700 may evaluate the levels of connectivity between various regions of the brain to determine attention levels and and whether the user is indicative of having ADHD based on those determined attention levels.

ADDS 1700 may determine and utilize signals of varying frequencies to determine attention levels and deficiencies. ADDS 1700 may group signals by frequency spectrum when evaluating signals and connectivity using signals to determine attention levels and attention deficiencies. ADDS 1700 groups signals in the 8-12 Hz frequency range as an alpha group, signals in the 20-30 Hz frequency range as a beta group, and signals in the 4-8 Hz frequency range as a theta group. In some embodiments, ADDS 1700 may collect signals of different frequencies at different times and from different regions of the brain.

Each of the signal groups indicates a certain aspect of task stimuli provision and reaction behavior. For example, the alpha band oscillation amplitude may indicate inhibition of activity. In some embodiments, signal group characteristics (e.g., amplitude) may have multiple meanings. For example, the same alpha band oscillation amplitude may also indicate active processing resulting in it being considered task-relevant information. Any change in signals or characteristics such as the amplitude of signals may be considered as a difference in the cognitive activity in the brain of user 1760 generating signals.

In some embodiments, a decrease in connectivity between regions of the brain indicates an increase in attention. The increase of connectivity is based on tasks used to activate different regions of user 1760's brain.

As illustrated in Fig. 19, the disclosed embodiments of ADDS 1700 evaluate communication between various known regions of the brain (e.g., regions within the dorsal attention network (DAN) 1910 or ventral attention network (VAN) 1920) used for evaluating attention. The disclosed embodiments of ADDS 1700 may utilize tasks defined in Figs. 18A-B to generate information flow of positive and negative bits that takes place between the DAN 1910 and VAN 1920, and identify the pathway involved in the flow of this information. ADDS 1700 may also identify alternate pathways and connectivity networks that may include signals when reacting to the same stimuli by different individuals. For example, ADDS 1700 may detect signals in dorsoventral attention network (DVAN) 1930.

ADDS 1700 determines a user's (user 1760 of Fig. 17) is indicative of having ADHD by determining and comparing the level of connectivity between regions of the brain generated in a user's brain to that of a known healthy group of individuals. The disclosed embodiments of ADDS 1700 provide stimuli using tasks as ways to trigger activity within brain networks and generate signals by transfer of information (positive and negative bits), and then use the generated signal connectivity to test for ADHD in a user (e.g., user 1760 of Fig. 17). For example, ADDS 1700 may use tasks 1800 and 1850 to activate and test for the presence of signals in DVAN 1930 by presenting instances of the universe of content defined by tasks 1800 and 1850 presented for user reaction. The usage of DVAN 1930 is tested by comparing measured signals of different types as evaluated in user 1760 to a previously identified healthy set of users (e.g., control group 1731) with no ADHD. A detailed description of the comparative analysis of signal groups between different users is presented in the description of Fig. 20 below.

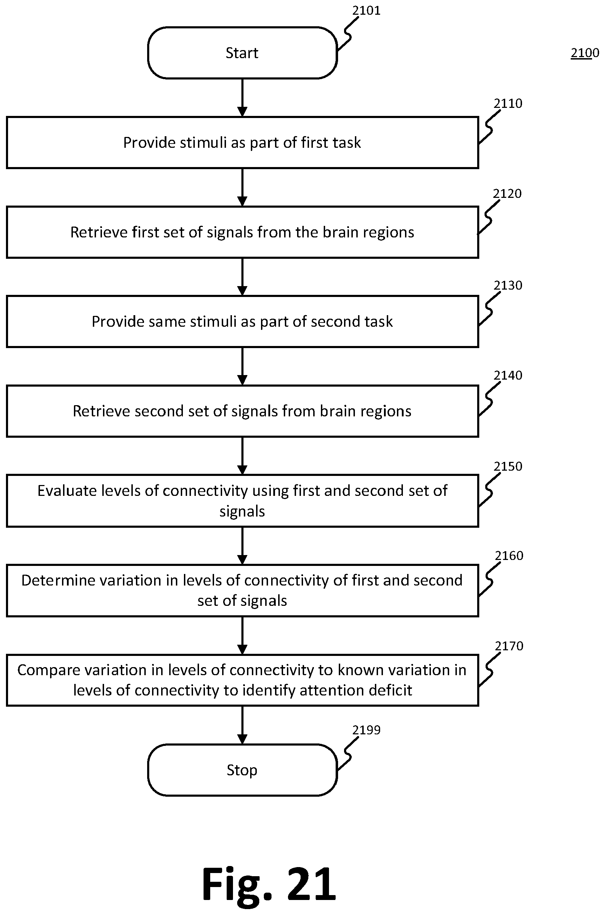

In some embodiments, attention deficiency may also be determined by comparing variation in connectivity with switched tasks with different rules. In such a scenario, an instance of a universe of content is chosen that has opposite reactions defined in tasks. For example, a red 'E' letter presented as part of task 1800 would result in a no-go condition with an active state restricted by a negative second condition. The same red 'E' letter presented as part of task 1850 results in a restricted second condition reversed by the third condition. A detailed description of attention network analysis in task switching scenarios is described in detail in Fig. 21 description below.

Disclosed embodiments of ADDS 1700 may help model the probability of ADHD levels based on the presented tasks for actions. The probability of ADHD levels helps accurately predict the presence of ADHD and the probability of ADHD. The model that identifies the probability of the level of ADHD can be implemented using example tasks 1800 and 1850 presented in the description of Fig. 3 above.

The Bayesian brain theory establishes that the entire cortex represents probability distributions which only collapse onto estimates when decisions are needed. The effect of data x as stimuli 1780 on user 1760 under Bayes Theorem to change the prior distribution $P(\theta)$ into the posterior distribution $P(\theta|x)$ is presented as follows:

$$P(\theta|x) = P(x|\theta)P(\theta)/P(x)$$

Where P(x) is known as the evidence and P(x|θ) is the likelihood of the Bayes' Theorem formula.

The same formula presented using -log functions used for representing information content transferred in the brain when data x is presented as stimuli 1780 is as follows:

$$-log_2[P(\theta|x)] = -log_2[P(x|\theta)P(\theta)/P(x)]$$

$$-log_2[P(\theta|x)] = -log_2 P(x|\theta) - log_2 P(\theta) + log_2 P(x)$$

The information content (measured in bits) of the event x_i, also known as self-information, is given by:

$$I(x_i) = -log_2[p(x_i)]\,bits$$

So the above logarithmic equation represented as information bits is as follows:

$$I(\theta|x)\;(bits) = I(\theta)(bits) - I(x)(bits) + I(x|\theta)(bits)$$

An aspect above equation is that the mentioned change of information content of a single model θ in the space of models that provokes a single observation x that occurrence on presenting data x, is not compounded, but is instead two information contents I(x) and I(x|θ) which are generated by a single observation. Therefore, in the equation above, -I(x)(bits) + I(x|θ)(bits), represents the information transferred to a single model θ due to the observation x. ADDS 1700 calculates this information transfer using a novel metric called the Transfer Information Content (TIC), which is defined as:

$$TIC(x \to \theta)(bits) = I(x) - I(x|\theta)$$

*I(θ)* may also be interpreted as the a priori uncertainty about model 0, and I(θ|x) as the a posteriori uncertainty about the model θ after a single observation x.

When the TIC metric is applied to the information content equation above, it is transformed as follows:

$$I(\theta|x)\;(bits) = I(\theta)(bits) - TIC(x \to \theta)(bits)$$

ADDS 1700 uses the TIC metric to describe the information flow. If the negative bits of the information content of the Bayes' Likelihood, I(θ|x), do not cancel the positive bits of the information content of the evidence, I(x), then the TIC metric is positive, i.e., there is a positive net transfer of information; on the contrary, if the negative bits corresponding to I(θ|x) are bigger than the bits of the evidence I(x), then the TIC is negative, and there is a negative net information transfer. A detailed description of the usage of this gain or loss of bits in form signal transfer between two regions to detect ADHD is provided in detail in the description of Fig. 20 below.

Using the above-defined TIC metric, the information content when represented using stimuli (e.g., stimuli 1780 of Fig. 17) and action (e.g., reactions 1740 of Fig. 17) transforms to

$$I(a|s) = I(a) - TIC(s \to a)(bits)$$

I(a) may also be interpreted as the a priori uncertainty about the action a, and I(a|s) as the a posteriori uncertainty about the action a taken by a user (e.g., user 1760 of Fig. 17) after stimulus s occurs.

The information content transfer that triggers or not an action a when the stimulus s occurs in a context c which is defined in tasks 1800 and 1850 using different conditions, is as follows:

$$I(a|s,c) = -log_2[P(a|s,c)]$$

$$I(a|s,c) = -\log_2[P(a|c,s)] = -\log_2\left[\frac{P(a|s)P(c|a,s)}{P(c|s)}\right]$$

$$I(a|s,c) = -\log_2[P(a|s)] - \log_2\left[\frac{P(c|a,s)}{P(c|s)}\right]$$

ADDS 1700 may use a conditional TIC metric to describe the contextual information content transfer. The conditional metric is defined as follows:

$$I(a|s,c) = I(a) - TIC(s \rightarrow a) - TIC(c \rightarrow a|s)$$

$$I(a|s,c) = I(a) - TIC(s \rightarrow a)(bits) - [I(c|s) - I(c|a,s)]$$
$$= I(a) - I(s) + I(s|a) - I(c|s) + I(c|a,s) \text{ (bits)}$$

I(a) may also be interpreted as the a priori uncertainty about the action a, and I(a|s, c) as the a posteriori uncertainty about the action a after stimulus s occurs in a context c.

ADDS 1700 may use conditional TIC to describe transferred information bits when an additional condition, such as a second condition in task 1800 defined using rules 1830 and 1840, is presented to user 1760.

In some embodiments, ADDS 1700 may consider the occurrence of stimulus s in a context c and in an episode (temporal context) e to compute the TIC metric for the information transfer that may trigger an action a. The information transfer is as follows:

$$I(a|s,c,e) = -\log_2[P(a|s,c,e)]$$

Similar to the contextual case, ADDS 1700 may then apply the conditioned version of Bayes' Theorem to the a posteriori probability as follows:

$$I(a|s,c,e) = -\log_2[P(a|c,s,e)] = -\log_2\left[\frac{P(a|s,c)P(e|a,s,c)}{P(e|s,c)}\right]$$

And then, applying logarithm rules, we have:

$$I(a|s,c,e) = -\log_2[P(a|s,c)] - \log_2\left[\frac{P(e|a,s,c)}{P(e|s,c)}\right]$$

ADDS 1700 may use a conditional TIC metric to describe the episodic information content transfer. The episodic metric is defined as follows:

$$I(a|s,c,e) = I(a) - TIC(s \rightarrow a) - TIC(c \rightarrow a|s) - TIC(e \rightarrow a|s,c)$$

Now, the sensorimotor TIC, the contextual conditional TIC, and the episodic conditional TIC can be broken down into their two components:

$$I(a|s,c,e) = I(a) - TIC(s \rightarrow a) - TIC(c \rightarrow a|s) - [I(e|s,c) - I(e|a,s,c)]$$

$$= I(a) - I(s) + I(s|a) - I(c|s) + I(c|a,s) - I(e|s,c) + I(e|a,s,c)$$

The components of the episodic conditional TIC, I(e|s, c) and I(e|a, s, c), represent bottom-up and top-down contributions in action selection, respectively. Following the reasoning of the contextual case, it may be possible that an uninformative stimulus in the given context may become informative if its episodic conditional TIC is positive and compensates the negativity of the contextual conditional TIC. ADDS 1700 can also interpret I(a) as the a priori uncertainty about the action a, and I(a|s, c, e) as the a posteriori uncertainty about the action a after stimulus s occurs in a context c and in an episode e.

ADDS 1700 may use a conditional TIC to describe transferred information bits when an additional condition, such as a second condition in task 1850 defined using rules 1880 and 1890, is presented to user 1760. A detailed description of conditional probabilities and bits of information transferred on presenting various stimuli to a user (e.g., user 1760 of Fig. 1) is presented in the description of Fig. 3 above.

Fig. 20 is a flowchart depicting operations of an exemplary method for attention deficiency detection, according to some embodiments of present disclosure. The steps of method 2000 may be performed by ADDS 1700 for purposes of illustration. It will be appreciated that the illustrated method 2000 may be altered to modify the order of steps and to include additional steps.

In step 2010, ADDS 1700 may provide stimuli to a user (e.g., user 1760 of Fig. 17) that causes a reaction. ADDS 1700 may provide stimuli by presenting a task (e.g., tasks 1800 and 1850 of Fig. 18) on a user device (e.g., user device 1750 of Fig. 17). ADDS 1700 may present a task by displaying the rules of a task followed by content to review and cause a reaction. User 1760 of user device 1750 may react to provided stimuli which is an instance of possible content based on rules of a task. ADDS 1700 may continuously provide stimuli by presenting content matching a task. In some embodiments, ADDS 1700 may loop through different tasks to provide stimuli to the user 1760 of ADDS 1700.

In step 2020, ADS 1700 may retrieve signals from sensors for presented stimuli. Sensors can be probes from an electroencephalography (EEG) or magnetoencephalography (MEG) device, or other functional neuroimaging technique for mapping brain activity like functional Magnetic Resonance Imaging (fMRI) and functional Near-Infrared Spectroscopy (fNIRS). Sensors are placed on the head matching different known regions (e.g., brain regions 1910-1930 of Fig. 19). ADDS 1700 may retrieve signals based on certain trigger events. In some embodiments, trigger events may include a period of time. The period of time may include a wait time since a stimulus was provided in step 2010. ADDS 1700 may allow the configuration of trigger events by allowing the selection of trigger events to retrieve signals and parameters of the trigger events. For example, ADDS 1700 may allow the amount of wait time after providing a stimulus before retrieving signals from different brain regions. The EEG/MEG device may be a standard EEG or MEG device. The EEG/MEG device may measure a plurality of signals of respective source positions located inside a cranial cavity of user 1760. Each source position may correspond to a known brain area, such as a lobe, a lobule, a sulcus, or a gyrus. In some examples, the plurality of signals may be measured while the subject is performing one or more tasks that may be presented to user 1760 by ADDS 1700. In one or more embodiments, a signal may correspond to the measurement of an electrode of an EEG device or to the measurement of a sensor coil of an MEG device. In some embodiments, the signal may correspond to an aggregated signal (such as an average signal) over the measurements of multiple electrodes (when an EEG is used) or multiple sensor coils (when an MEG is used). Such an aggregated signal may correspond to an area of the brain that comprises the respective positions of the signals that are being aggregated in the aggregated signal.

MEG signals can be measured using a 306-channel (102 magnetometers and 204 planar gradiometers) whole-head MEG Elekta Neuro image system. In some embodiments, brain signals can be measured using an online anti-alias bandpass filter between 0.1 and 330 Hz and a sampling rate of 1,000 Hz. In some embodiments, a user's (e.g., user 1760 of Fig. 17) head shape may be acquired using a three-dimensional Fastrak digitizer (Polhemus, Colchester, Vermont), in addition to three fiducial points (nasion and left and right preauricular points) as landmarks. In another embodiment, four Head Position Indicator (HPI) coils may be recorded on the user 1760's scalp (forehead and the mastoids). In some embodiments, two sets of bipolar electrodes may be used to record eye blinks and heart beats, respectively.

In step 2030, ADDS 1700 may evaluate the signals of each brain region from the retrieved brain signals of step 2020. ADDS 1700 may evaluate signal quality to remove artifacts from signals and reconstruct the brain activity using signals retrieved from each sensor on the user's head approximately representing each brain region. ADDS 1700 may improve data by removing external noise, such as ocular, cardiac, or muscle artifacts, from the EEG/MEG data measured, and segmenting the EEG/MEG data such that the time series may have the same length (e.g., 1 second).

In step 2031, ADDS 1700 may filter the extracted signals in step 2030 into multiple frequency bands by using, for example, pass filters. The frequency bands may be functionality-relevant signal bands. As provided in the description of Fig. 19 above, a signal may be filtered into different frequencies to indicate different attention behaviors of users of ADDS 1700. For example, ADDS 1700 extracts signals into frequency bands: theta (4 to 8 Hz), alpha (8 to 12 Hz), low beta (12 to 20 Hz), high beta (20 to 30 Hz), and low gamma (30 to 45 Hz). In some embodiments, the frequency ranges describing theta, alpha, low beta, high beta, and low gamma bands may be different. In some embodiments, there may be additional groups of signals in between the example frequency bands. ADDS 1700 may allow configuration of frequency band ranges to be part of a signal group. ADDS 1700 extracts signals by frequency bands by filtering and removing signals not falling into one of the selected frequency bands.

In step 2032, ADDS 1700 may determine the level of connectivity between two brain regions based on dependency measures between signals of two brain regions for each frequency band. For example, ADDS 1700 may measure the phase synchronization between signals grouped under a frequency band to calculate the connectivity value. Phase synchronization measures include Phase-Lag Index or Phase-Locking Value (PLV) and its derivatives, imaginary part of PLV (iPLV) and corrected imaginary part of PLV (ciPLV). ADDS 1700 may use other different connectivity measures, including Information Theory measures like Mutual Information or Transfer Entropy, Granger causality measures, and classical measures like Correlation, Cross-Correlation, Coherence, or Phase-Slope Index. ADDS 1700 may measure the level of connectivity between two brain regions based on metrics derived from TIC. In some embodiments, dependency measures may be based on connectivity between brain regions between different frequency bands.

In step 2033, ADDS 1700 may check if the type of signal matches theta band signal frequencies by filtering theta band signals. If the answer is "No," then jump to step 2038.

In step 2034, ADDS 1700 may check if the first condition of the second task is met. If the answer to the question is "Yes," then jump to step 2035. If the answer to the question is "No then jump to step 2056.

In step 2035, ADDS, 1700 may check if second condition of second task is met. If the answer to the question is 'No," then jump to step 2037.

In step 2036, ADDS 1700 may check if the third condition of the second task is met. If the answer to the question is "Yes," then proceed to step 2037. If the answer to the question is "No," then jump to step 2056.

In step 2037, ADDS 1700 may check whether the level of connectivity of the user as calculated in step 2032 is less than the level of connectivity of a healthy control group. If the answer to the question is "Yes," then jump to step 2070. ADDS 1700 may retrieve the level of connectivity of the healthy control group stored in population database 1730. ADDS 1700 may receive control group type user as input and stores the results in control group 1731. ADDS 1700 may compare the level of connectivity by checking whether the calculated connectivity level is less than the range of connectivity levels possible for the healthy control group. ADDS 1700 may determine the range of values of the connectivity level of a healthy control group based on the level of connectivity of an individual identified to have ADHDas evaluated by method 2000.

In step 2038, ADDS 100 may check if the type of signal matches beta band signal frequencies by filtering beta band signals. If the answer is "No," then jump to step 2049.

In step 2039, ADDS 1700 may check if the stimulus is from the first task. If the answer to the question is "No," then jump to step 2044.

In step 2040, ADDS 1700 may check if the first condition of the first task is met. If the answer to the question is "No," then jump to step 2048.

In step 2041, ADDS 1700 may check if the second condition of the first task is met. If the answer to the question is "Yes," then jump to step 2048. If the answer to the question is "No," then jump to step 2056.

In step 2044, ADDS 100 may check if the first condition of the second task is met. If the answer to the question is "Yes," then proceed to step 2045. If the answer to the question is "No," then jump to step 2056.

In step 2045, ADDS 1700 may check if the second condition of the second task is met. If the answer to the question is "Yes," then proceed to step 2046. If the answer to the question is "No," then jump to step 2047.

In step 2046, ADDS 100 may check if the third condition of the second task is met. If the answer to the question is "Yes," then jump to step 2048. If the answer to the question is "No," then jump to step 2056.

In step 2047, ADDS 1700 may check if the third condition of the second task is met. If the answer to the question is "No," then proceed to step 2048. If the answer to the question is "Yes," then jump to step 2056.

In step 2048, ADDS 1700 may check whether the level of connectivity of the user as calculated in step 2032 is greater than the level of connectivity of the healthy control group. If the answer to the question is "Yes," then jump to step 2070. ADDS 1700 may retrieve the level of connectivity of the healthy control group stored in population database 1730. ADDS 1700 may receive control group type user as input and stores the results in control group 1731. ADDS 1700 may compare the level of connectivity by checking whether the calculated connectivity level is less than the range of connectivity levels possible for the healthy control group. ADDS 1700 may determine the range of values of the connectivity level of a healthy control group based on the level of connectivity of an individual identified to have ADHD as evaluated by method 2000.

In step 2049, ADDS 1700 may check if the type of signal matches alpha band signal frequencies by filtering alpha band signals. If the answer to the question is "Yes," then proceed to step 2050. If the answer to the question is "No," then jump to step 2056.

In step 2050, ADDS 1700 may check if the stimulus is from the first task. If the answer to the question is "No," then jump to step 2054.

In step 2051, ADDS 1700 may check if the first condition of first task is met. If the answer to the question is "No," then proceed to step 2051.2; otherwise, jump to step 2052.

In step 2051.2, if the level of connectivity of user 1760 as calculated in step 2032 is stored in a container A1, then jump to step 2055. Container A1 may be a data structure, such as a list or an array. In some embodiments, container A1 may be persisted on a disk in a flat file or a database. In step 2052, ADDS 1700 may check if the second condition of the first task is met. If the answer to the question is "No," then proceed to step 2052.2; otherwise, jump to step 2052.3.

In step 2052.2, if the level of connectivity of the user as calculated in step 2032 is stored in a container A2, then jump to step 2055. Container A2 may be a data structure, such as a list or an array. In some embodiments, container A2 may be persisted on a disk in a flat file or a database. Container A2 will contain alpha band connectivity values between brain areas when the first condition of the first task is met and the second condition is not met.

In step 2052.3, the level of connectivity of user 1760 as calculated in step 2032 is stored in a container A1.

In step 2053, ADDS 1700 may check if the third condition of the first task is met, i.e., it is a red-colored vowel "E." If the answer to the question is "Yes," then proceed to step 2053.2; otherwise, jump to step 2055.

In step 2053.2, if the level of connectivity of the user as calculated in step 2032 is stored in a container A3, then jump to step 2055. Container A3 may be a data structure, such as a list or an array. In some embodiments, container A3 may be persisted on a disk in a flat file or a database. Container A3 will contain alpha band connectivity values between brain areas when the first, second, and third conditions of the first task are met, i.e., it is a red-colored vowel "E."

In step 2054, ADDS 1700 may check if the first, second, and third conditions of the second task are met. If the answer to the question is "Yes," then proceed to step 2054.2; otherwise, jump to step 2056.

In step 2054.2, if the level of connectivity of the user as calculated in step 2032 is stored in container A4, then proceed to step 2055. Container A4 may be a data structure, such as a list or an array. In some embodiments, container A4 may be persisted on a disk in a flat file or a database. Container A4 will contain alpha band connectivity values between brain areas when the first, second, and third conditions of the second task are met, i.e., it is a red-colored vowel "E."

In step 2055, ADDS 1700 may check whether the level of connectivity of the user as calculated in step 2032 is less than the level of connectivity of the healthy control group. If the answer to the question is "Yes," then jump to step 2070. ADDS 1700 may retrieve the level of connectivity of the healthy control group stored in population database 1730. ADDS 1700 may receive control group type user as input and stores the results in control group 1731. ADDS 1700 may compare the level of connectivity by checking whether the calculated connectivity level is less than the range of connectivity levels possible for the healthy control group. The range of values to include for the connectivity level of a healthy control group may be determined based on the level of connectivity of an individual identified to have ADHD.

In step 2056, ADDS 1700 may check if all connectivity values calculated in step 2032 have been checked for ADHD identification by going through steps 2033 to 2055. If the answer to the question is "Yes," then proceed to step 2057. If the answer to the question is "No," the next connectivity value is checked by jumping to step 2033.

In step 2057, ADDS 1700 may retrieve connectivity level values in containers A1 and A2 from steps 2051.2, 2052.2, and 2052.3. ADDS 1700 may compare the change in connectivity levels. If the reduction in the connectivity level is less than the reduction in the connectivity level of the healthy control group, then jump to step 2070.

In step 2058, ADDS 1700 may retrieve connectivity level values in containers A3 and A4 from steps 2053.2 and 2054.2, respectively. ADDS 1700 may compare the change in connectivity levels. If the variation in the connectivity level between brain regions of a user is different than the reduction in the connectivity level between the same regions of a healthy control group, then jump to step 2070.

In some embodiments, ADDS 1700 may compare the variation in the level of connectivity between two regions based on levels of connectivity based on signals determined in steps 2033, 2038, and 2049 of method 2000. ADDS 1700 may calculate the probability of ADHD based on the difference in the variation of level connectivity between the user of ADDS 1700 and the healthy control group. ADDS 1700 may collect multiple levels of connectivity of the same user or a set of users to save them as healthy control group 1731 data or for those identified with ADHD as users 1732. ADDS 1700 may use the stored values of levels of connectivity to determine the variation in the level of connectivity. In some embodiments, the variations in the levels of connectivity may only include observed levels of connectivity for the same data for a different condition. For example, a user of ADDS 1700 may be presented with the same data and then presented with the rules of the task to determine levels of connectivity for the same data across multiple tasks. In step 2060, ADDS 1700 may mark user (e.g., user 1760) whose brain signals are evaluated for level of connectivity to have an indication of ADHD. ADDS 1700, upon completion of step 2060, completes (step 2099) executing method 2000.

Fig. 21 is a flowchart depicting operations of an exemplary method for detection of ADHD, according to some embodiments of the present disclosure. The steps of method 2100 may be performed by ADDS 1700 for purposes of illustration. It will be appreciated that the illustrated method 2100 may be altered to modify the order of steps and to include additional steps.

In step 2110, ADDS 1700 may provide stimuli to a user (e.g., user 1760 of Fig. 17) that causes a reaction that match a first task (e.g., task 1800 of Fig. 18A). ADDS 1700 may select a set of tasks for testing a user (e.g., user 1760 of Fig. 17) for ADHD that can satisfy rules (e.g., rules 1820 and 1830 of task 1800, rules 1875 and 1880 of task 1850). ADDS 1700 may provide stimuli by displaying content that satisfies the rules of task 1800 on a user device (e.g., user device 1750 of Fig. 17).

In step 2120, ADDS 1700 may retrieve a first set of signals from brain regions. ADDS 1700 may retrieve signals from different brain regions by collecting electric charges from probes attached to the head of user 1760 being tested for ADHD. ADDS 1700 may retrieve signals when a user reacts to the stimuli provided in step 2110 by performing an action. Action performance may include clicking a button or selecting something presented on the screen using a pointing device.

In step 2130, ADDS 1700 may provide the same stimuli as part of a second task (e.g., task 1850 of Fig. 2B). ADDS 1700 may select tasks where the rules are satisfied by the first and second tasks. ADDS 1700 may only select tasks from task definitions 1733 where the same content satisfies different rules requiring opposite user reaction. For example, when a red-colored letter "E" is presented as a stimulus under task 1800, then rules 1820 and 1830 are satisfied, and the user is expected not to react, but under task 1850, rules 1875 and 1880 are satisfied, and the user is expected to react.

In step 2140, ADDS 1700 may retrieve a second set of signals from brain regions. Similar to step 2120, ADDS 1700 may retrieve signals by collecting brain images or electric charges from user 1760's brain upon viewing stimuli in step 2130. The signal may be generated as part of the reaction performed by user 1760.

In step 2150, ADDS 1700 may evaluate levels of connectivity using the first and second set of signals. ADDS 1700 may evaluate levels of connectivity by measuring the signals in certain regions of the brain (e.g., dorsal attention network (DAN) 1910 of Fig. 19) and measuring the signal in other regions of the brain (e.g., ventral attention network (VAN) 1920 of Fig. 19) to determine the amount of transferred signals indicating the level of connectivity.

In step 2160, ADDS 1700 may determine variations in the levels of connectivity of the first and second set of signals. ADDS 1700 may determine variations in the levels of connectivity by calculating the levels of connectivity by providing multiple stimuli as part of the first and second tasks in steps 2110 and 2130, and retrieving signals of user 1760's reaction in steps 2120 and 2140.

In step 2170, ADDS 1700 may compare variations in the levels of connectivity to known variations in the levels of connectivity to identify ADHD. ADDS 1700 may compare variations in the levels of connectivity calculated in step 2160 to variations in the levels of connectivity of a control group (e.g., control group 1731). ADDS 1700 may retrieve variations in the levels of connectivity of control group 1731 from previously generated performance metrics 1734. In some embodiments, ADDS 1700 may need to evaluate the variations in the levels of connectivity from previously calculated levels of connectivity stored in performance metrics 1734. ADDS 1700 may indicate that user 1760 has ADHD if the variations of the levels of connectivity of user 1760 are different than the variations of the levels of connectivity of control group 1731. ADDS 1700 may consider the variations of the levels of connectivity to be different if the range of values of the levels of connectivity is different. In some embodiments, the variations of levels of connectivity are considered different if the ranges of values do not overlap. ADDS 1700 may store attention deficit results in database 130 under users 1732. ADDS 1700, upon completion of step 2170, completes (step 2199) executing method 2100.

Example 1: A non-transitory computer readable medium including instructions that, when executed by at least one processor, cause the at least one processor to perform operations for automated detection of attention deficit hyperactivity disorder (ADHD), the operations comprising: providing one or more stimuli to a user to activate a plurality of brain regions representing an attention network, wherein the one or more stimuli include displaying information with a first condition that causes a reaction and a second condition as an exception to the first condition; retrieving a plurality of signals from the plurality of brain regions for each of the one or more stimuli, wherein each signal of the plurality of signals is accessed over a period of time, wherein the period of time starts when the information is displayed and ends when the reactions of the user are captured; and evaluating the plurality of signals to detect ADHD based on a level of connectivity within an attention network when each signal of the plurality of signals corresponds to the one or more stimuli.

Example 2: The non-transitory computer readable medium of example 1, wherein displaying information with a first condition that causes a reaction and a second condition as an exception to the first condition further comprises: presenting an instance of data matching a first task, wherein the first task includes the first condition for expectation of an active state and a silent state of a first reaction, the second condition restricting the expectation of the active state of the first reaction.

Example 3: The non-transitory computer readable medium of example 2, wherein the first reaction further comprises: displaying the information on a screen meeting the first condition for expectation of the active state and the silent state of the first reaction; and waiting for a threshold period for any reaction shared by the user.

Example 4: The non-transitory computer readable medium of example 2, wherein the first reaction is at least one of clicking a pointing device, pressing a button, or taking no action for a time threshold.

Example 5: The non-transitory computer readable medium of example 2, wherein evaluating the plurality of signals to detect ADHD based on the level of connectivity within the attention network further comprises: filtering alpha band oscillation signals of two or more regions of the plurality of brain regions of the user upon receiving the first reaction and determining connectivity between two regions of the brain using the alpha band oscillation signals; and comparing the level of connectivity between the two regions of the brain of the user to the level of connectivity of a healthy control group of users when the first condition of the first task satisfies the active state, and the second condition is negative, wherein if the level of connectivity of the user is less than the level of connectivity of the healthy control group of users, then the user is indicative of having ADHD.

Example 6: The non-transitory computer readable medium of example 2, wherein evaluating the plurality of signals to detect ADHD based on the level of connectivity within the attention network further comprises: filtering alpha band oscillation signals of two or more regions of the plurality of brain regions of the user upon receiving the first reaction and determining connectivity between two regions of the brain using the alpha band oscillation signals; and comparing the level of connectivity between the two regions of the brain of the user to the level of connectivity of a healthy control group of users when the first condition of the first task satisfies the silent state, the second condition is positive, wherein if the level of connectivity of the user is less than the level of connectivity of the healthy control group of users, then the user is indicative of having ADHD.

Example 7: The non-transitory computer readable medium of example 2, the operations further comprising: filtering beta band oscillation signals of two or more brain regions upon receiving the first reaction and determining connectivity between two regions of the brain using the beta band oscillation signals; and comparing the level of connectivity between the two regions of the brain of the user to the level of connectivity of a healthy control group of users when the first condition of the first task satisfies the silent state, the second condition is positive, wherein if the level of connectivity of the user is greater than the level of connectivity of the beta band oscillation signals of the healthy control group of users, then the user is indicative of having ADHD.

Example 8: The non-transitory computer readable medium of example 2, the operations further comprise: filtering alpha band oscillation signals of two or more brain regions upon receiving the first reaction and determining connectivity between two regions of the brain using the alpha band oscillation signals; determining the level of connectivity between the two regions of the brain of the user when the first condition of the first task satisfies the silent state and the first condition of the first task satisfies the active state; and comparing a reduction in the level of connectivity of the user between when the first task satisfies the silent state and when the first task satisfies the active state to a reduction in the level of connectivity of a healthy control group of users between when the first task satisfies the silent state and when the first task satisfies the active state, wherein if the reduction in the level of connectivity of the user is less than the reduction in the level of connectivity of the healthy control group of users, then the user is indicative of having ADHD.

Example 9: The non-transitory computer readable medium of example 1, wherein evaluating the plurality of signals to detect ADHD based on the level of connectivity within the attention network further comprises: combining signal data of each of the plurality of signals, wherein combining signal data includes determining an increase in the level of connectivity compared to an existing level of connectivity between regions from the plurality of regions or a reduction in the level of connectivity from the existing level of connectivity of the region of the plurality of regions.

Example 10: The non-transitory computer readable medium of example 1, wherein displaying information with a first condition includes displaying a category of text or graphic.

Example 11: The non-transitory computer readable medium of example 10, wherein the second condition includes displaying the category of text or graphic in a particular color.

Example 12: The non-transitory computer readable medium of example 1, wherein the one or more stimuli further comprises: displaying second information with the first condition that causes a reaction, the second condition as the exception to the first condition, and a third condition as an exception to the second condition.

Example 13: The non-transitory computer readable medium of example 12, wherein displaying second information with the first condition that causes a reaction, the second condition as the exception to the first condition, and a third condition as an exception to the second condition further comprises: presenting an instance of data matching a second task, wherein the second task includes the first condition for expectation of an active state and a silent state of a second reaction, the second condition restricting expectation of the active state of the second reaction, and the third condition that is an exception to the second condition for expectation of silent state of the second reaction.

Example 14: The non-transitory computer readable medium of example 13, wherein the second reaction further comprises: displaying information on a screen meeting the first condition for expectation of the active state and the silent state of the second reaction; and waiting for a threshold period for any input shared by the user.

Example 15: The non-transitory computer readable medium of example 13, wherein the third condition includes: selecting a sub-category of a category of text or graphic meeting the first condition; and displaying the sub-category of the text or graphic.

Example 16: The non-transitory computer readable medium of example 13, wherein the second reaction is at least one of clicking a pointing device, pressing a button, or taking no action for a threshold time.

Example 17: The non-transitory computer readable medium of example 13, the operations further comprising: filtering beta band oscillation signals of two or more regions of the plurality of brain regions of the user upon receiving the second reaction and determining connectivity between two regions of the brain using the beta band oscillation signals; and comparing the level of connectivity between the two regions of the brain of the user to the level of connectivity of a healthy control group of users when the first condition of the second task satisfies the active state, the second condition is negative and the third condition is negative, or when the first condition of the second task satisfies the active state, the second condition is positive and the third condition is positive, wherein if the level of connectivity of the user is greater than the level of connectivity of the healthy control group of users, then the user is indicative of having ADHD.

Example 18: The non-transitory computer readable medium of example 13, the operations further comprise: filtering theta band oscillation signals of two or more regions of the plurality of brain regions of the user upon receiving the second reaction from the user and determining connectivity between two regions of the brain using the theta band oscillation signals; and comparing the level of connectivity between the two regions of the brain of the user to the level of connectivity of a healthy control group of users when the first condition of the second task satisfies the active state, the second condition is negative and the third condition is negative, or when the first condition of the second task satisfies the active state, the second condition is positive and the third condition is positive, wherein if the signal data of the level of connectivity of the user is less than the level of connectivity of the healthy control group of users, then the user is indicative of having ADHD.

Example 19: The non-transitory computer readable medium of example 13, the operations further comprise: filtering alpha band oscillation signals of two or more regions of the plurality of brain regions of the user upon receiving the second reaction from the user and determining connectivity between two regions of the brain using the alpha band oscillation signals; determining a variation in the level of connectivity between the two regions of the brain of the user when presenting the instance of data for the second task where the first condition of the second task satisfies the active state, the second condition of the second task is positive and the third condition of the second task is positive to the level of connectivity between the two regions of the brain of the user when presenting the instance of data for the first task where the first condition of the first task satisfies the active state, and the second condition of the first task is positive; and comparing the variation in the level of connectivity of the user to the variation in the level of connectivity of a healthy control group of users, wherein if the variation in the level of connectivity of the user is different than the variation in the level of connectivity of the healthy control group of users, then the user is indicative of ADHD.

Example 20: The non-transitory computer readable medium of example 13, filtering alpha, beta, and theta band oscillation signals of two or more regions of the plurality of brain regions of the user upon receiving the first reaction and the second reaction and determining connectivity between regions of the brain using the alpha, beta, and theta band oscillation signals; and evaluating the probability of the detection of ADHD based on the comparison of the level of connectivity, reduction in the level of connectivity, and the variation in the level of connectivity between the regions of the brain of the user to the level of connectivity of a healthy control group of users when varying the first condition, the second condition of the first task, and the first condition, the second condition, and the third condition of the second task.

Example 21: A computer-implemented method for automated detection of attention deficit hyperactivity disorder (ADHD), the method comprising: providing one or more stimuli to a user to activate a plurality of brain regions representing an attention network, wherein the one or more stimuli include displaying information with a first condition that causes a reaction and a second condition as an exception to the first condition; retrieving a plurality of signals from the plurality of brain regions for each of the one or more stimuli, wherein each signal of the plurality of signals is accessed over a period of time, wherein the period of time starts when the information is displayed and ends when the reactions of the user are captured; and evaluating the plurality of signals to detect ADHD based on a level of connectivity in the attention network when each signal of the plurality of signals corresponds to the one or more stimuli.

Example 22: An attention deficit detection system, comprising: one or more memory devices storing processor-executable instructions; and one or more processors configured to execute instructions to cause the attention deficit detection system to perform operations comprising: providing one or more stimuli to activate a plurality of brain regions representing an attention network, wherein the one or more stimuli include displaying information with a first condition that causes a reaction and a second condition as an exception to the first condition; retrieving a plurality of signals from the plurality of brain regions for each of the one or more stimuli, wherein each signal of the plurality of signals is accessed over a period of time, wherein the period of time starts when the information is displayed and ends when the reactions of the user are captured; and evaluating the plurality of signals to detect ADHD based on a level of connectivity in the attention network when a signal of the plurality of signals corresponds to the one or more stimuli.

Example 23: An attention deficit detection system, comprising: one or more memory devices storing processor-executable instructions; and one or more processors configured to execute instructions to cause the attention deficit detection system to perform operations comprising: receiving first signal data from a user; determining a first plurality of time series based on the first signal data, wherein each of the first plurality of time series corresponds to a respective source position located inside a cranial cavity of the user; calculating a first correlation value for a first pair of time series, the first pair of time series being included in the determined first plurality of time series; generating a score based on the first correlation value, the score being indicative of a patient having a cognitive impairment, such as an attention deficiency or ADHD; and outputting the generated score.

**Table 3:** Automated Anatomical Labeling atlas (used, e.g., for Figures 4 to 12).

| NAME | ADBREVIATION |
|------|------|
| Left Precentral gyrus | lPreCG |
| Right Precentral gyrus | rPreCG |
| Left Superior Frontal gyrus | lSFG |
| Right Superior Frontal gyrus | rSFG |
| Left Superior Frontal gyrus, Orbital | lSFo |
| Right Superior Frontal gyrus, Orbital | rSFo |
| Left Middle Frontal gyrus | lMFG |
| Right Middle Frontal gyrus | rMFG |
| Left Middle Frontal gyrus, Orbital | lMFGo |
| Right Middle Frontal gyrus, Orbital | rMFGo |
| Left Inferior Frontal gyrus, Opercular | lIFGor |
| Right Inferior Frontal gyrus, Opercular | rIFGor |
| Left Inferior Frontal gyrus, Triangular | lIFGt |
| Right Inferior Frontal gyrus, Triangular | rIFGt |
| Left Inferior Frontal gyrus, Orbital | lIFGo |
| Right Inferior Frontal gyrus, Orbital | rIFGo |
| Left Rolandic operculum | lRO |
| 'Right Rolandic operculum | rRO |
| Left Supplementary Motor area | lMotor |
| Right Supplementary Motor area | rMotor |
| Left Superior Frontal gyrus, Medial | lSFGm |
| Right Superior Frontal gyrus, Medial | rSFGm |
| Left Superior Frontal gyrus, Medial Orbital | lSFGmo |
| Right Superior Frontal gyrus, Medial Orbital | rSFGmo |
| Left Gyrus Rectus | lRectus |
| Right Gyrus Rectus | rRectus |
| Left Insula | lI\nsula |
| Right Insula | rInsula |
| Left Cingulate gyrus, Anterior part | lACC |
| Right Cingulate gyrus, Anterior part | rACC |
| Left Cingulate gyrus, Middle part | lMCC |
| Right Cingulate gyrus, Middle part | rMCC |
| Left Cingulate gyrus, Posterior part | lPCC |
| Right Cingulate gyrus, Posterior part | rPCC |
| Left Hippocampus | lHip |
| Right Hippocampus | rHip |
| Left Parahippocampus | lParahip |

(continued)

| NAME | ADBREVIATION |
|---|---|
| Right Parahippocampus | rParahip |
| Left Calcarine fissure and surrounding cortex | lCalc |
| Right Calcarine fissure and surrounding cortex | rCalc |
| Left Cuneus | lCu |
| Right Cuneus | rCu |
| Left Lingual gyrus | lLingual |
| Right Lingual gyrus | rLingual |
| Left Superior Occipital lobe | lSOccL |
| Right Superior Occipital lobe | rSOccL |
| Left Middle Occipital lobe | lMOccL |
| Right Middle Occipital lobe | rMOccL |
| Left Inferior Occipital lobe | lIOccL |
| Right Inferior Occipital lobe | rIOccl |
| Left Fusiform gyrus | lFusiG |
| Right Fusiform gyrus | rFusiG |
| Left Postcentral gyrus | lPreG |
| Right Postcentral gyrus | rPreG |
| Left Superior Parietal gyrus | lSPG |
| Right Superior Parietal gyrus | rSPG |
| Left Inferior Parietal gyrus | lIPG |
| Right Inferior Parietal gyrus | rIPG |
| Left Supramarginal gyrus | lSMG |
| Right Supramarginal gyrus | rSMG |
| Left Angular gyrus | lAng |
| Right Angular gyrus | rAng |
| Left Precuneus | lPrecu |
| Right Precuneus | rPrecu |
| Left Paracentral lobule | lParaL |
| Right Paracentral lobule | rParaL |
| Left Heschls gyrus | lHeschl |
| Right Heschls gyrus | rHeschl |
| Left Superior Temporal gyrus | lSTG |
| Right Superior Temporal gyrus | rSTG |
| Left Temporal pole, Superior Temporal gyrus | lTPsup |
| Right Temporal pole, Superior Temporal gyrus | rTPsup |
| Left Middle temporal gyrus | lMTG |
| Right Middle temporal gyrus | rMTG |
| Left Temporal pole, Middle temporal gyrus | lTPmid |

(continued)

| NAME | ADBREVIATION |
|------|--------------|
| Right Temporal pole, Middle temporal gyrus | rTPmid |
| Left Inferior Temporal gyrus | lITG |
| Right Inferior Temporal gyrus | rITG |

N. Tzourio-Mazoyer; B. Landeau; D. Papathanassiou; F. Crivello; O. Etard; N. Delcroix; Bernard Mazoyer & M. Joliot (January 2002). "Automated Anatomical Labeling of activations in SPM using a Macroscopic Anatomical Parcellation of the MNI MRI single-subject brain". NeuroImage. 15 (1): 273-289. doi:10.1006/nimg.2001.0978. PMID 11771995

**Claims**

1. An apparatus (140; 200), configured to:

   receive (410) first electro- or magnetoencephalography, EEG/MEG, data (130) of a subject (110);
   determine (420) a first plurality of time series based on the first EEG/MEG data, wherein each of the first plurality of times series corresponds to a respective source position located inside a cranial cavity (300) of the subject;
   calculate (430) a first correlation value for a first pair of time series, the first pair of time series being included in the determined first plurality of time series;
   generate (440) one or more scores (150) based on the first correlation value, the one or more scores being indicative for the subject having a cognitive condition, such as a cognitive disorder like attention deficit hyperactivity disorder, ADHD; and
   output (450) the generated one or more scores.

2. The apparatus (140; 200) of claim 1,

   wherein the respective source position of a first time series of the first pair of time series is located in a frontal lobe (310), such as in a middle frontal gyrus (320), MFG, and/or in an inferior frontal gyrus (330), IFG, in the cranial cavity (300) of the subject (110), and
   wherein the respective source position of a second time series of the first pair of time series is located in a superior parietal lobe (340), SPL, in the cranial cavity of the subject.

3. The apparatus (140; 200) of claim 1 or 2, further configured to:

   calculate a second correlation value for a second pair of time series, the second pair of time series being included in the determined first plurality of time series; and
   wherein the one or more scores are generated based on the first correlation value and the second correlation value; and/or
   wherein the respective source position of a first time series of the second pair of time series is located in a ventral attention network area, such as in a temporoparietal junction (350), TPJ, in the cranial cavity (300) of the subject (110), and
   wherein the respective source position of a second time series of the second pair of time series is located in a dorsal attention network area, such as in a superior parietal lobe (340), SPL, in the cranial cavity of the subject.

4. The apparatus (140; 200) of any of claim 3, further configured to:

   calculate a third correlation value for a third pair of time series, the third pair of time series being included in the determined first plurality of time series; and
   wherein the one or more scores are generated based on the first correlation value, the second correlation value and the third correlation value; and/or
   wherein the respective source position of a first time series of the third pair of time series is located in the ventral attention network area, such as in the temporoparietal junction (350), TPJ, in the cranial cavity (300) of the subject (110), and
   wherein the respective source position of a second time series of the second pair of time series is located in a ventral visual pathway area, such as in an inferior temporal gyrus (360), ITG, in the cranial cavity of the subject.

**5.** The apparatus (140; 200) of any of claims 1 to 4, further configured to:

receive second EEG/MEG data of the subject (110);
determine a second plurality of time series based on the second EEG/MEG data, wherein each of the second plurality of times series corresponds to a respective source position located inside the cranial cavity (300) of the subject;
calculate a fourth correlation value for a fourth pair of time series, the fourth pair of time series being included in the determined second plurality of time series, and the fourth pair of time series corresponding to the same respective source positions as the first pair of time series;
calculate a comparison value between the fourth correlation value and the first correlation value; and
wherein the one or more scores (150) are generated based on the comparison value.

**6.** The apparatus of any of claims 1 to 5,
wherein the determining (420) the first plurality of time series comprises filtering the first EEG/MEG data (130) such that the first plurality of time series only includes a subset of all available time series in the first EEG/MEG data, the subset including the first pair of time series, the second pair of time series and the third pair of time series.

**7.** A system (100) comprising:

the apparatus (140) according to any one of claims 1 to 6;
an electro- or magnetoencephalography, EEG/MEG, device (120) for measuring at least the first EEG/MEG data (130) of the subject (110); and
a task presentation device (160) for presenting one or more tasks to the subject while the EEG/MEG device is measuring at least the first EEG/MEG data (130) of the subject.

**8.** A computer-implemented method (400), the method comprising:

receiving (410) first electro- or magnetoencephalography, EEG/MEG, data (130) of a subject (110);
determining (420) a first plurality of time series based on the first EEG/MEG data, wherein each of the first plurality of times series corresponds to a respective source position located inside a cranial cavity (300) of the subject;
calculating (430) a first correlation value for a first pair of time series, the first pair of time series being included in the determined first plurality of time series;
generating (440) one or more scores (150) based on the first correlation value, the one or more scores being indicative for the subject having a cognitive condition, such as a cognitive disorder like attention deficit hyperactivity disorder, ADHD; and
outputting (450) the generated one or more scores.

**9.** The method (400) of claim 8,

wherein the respective source position of a first time series of the first pair of time series is located in a frontal lobe (310), such as in a middle frontal gyrus (320), MFG, and/or in an inferior frontal gyrus (330), IFG,, in the cranial cavity (300) of the subject (110), and
wherein the respective source position of a second time series of the first pair of time series is located in a superior parietal lobe (340), SPL in the cranial cavity of the subject.

**10.** The method (400) of claim 8 or 9 , further comprising:

calculating a second correlation value for a second pair of time series, the second pair of time series being included in the determined first plurality of time series, and
wherein the one or more scores are generated based on the first correlation value and the second correlation value; and/or
wherein the respective source position of a first time series of the second pair of time series is located in a ventral attention network area, such as in a temporoparietal junction (350), TPJ, in the cranial cavity (300) of the subject (110), and
wherein the respective source position of a second time series of the second pair of time series is located in a dorsal attention network area, such as in a superior parietal lobe (340), SPL, in the cranial cavity of the subject.

**11.** The method (400) of claim 10, further comprising:

calculating a third correlation value for a third pair of time series, the third pair of time series being included in the determined first plurality of time series; and
wherein the one or more scores are generated based on the first correlation value, the second correlation value and the third correlation value; and/or
wherein the respective source position of a first time series of the third pair of time series is located in the ventral attention network area, such as in the temporoparietal junction (350), TPJ, in the cranial cavity (300) of the subject (110), and
wherein the respective source position of a second time series of the second pair of time series is located in a ventral visual pathway area, such as in an inferior temporal gyrus (360), ITG, in the cranial cavity of the subject.

**12.** The method (400) of any of claims 8 to 11, further comprising:

receiving second EEG/MEG data of the subject (110),
determining a second plurality of time series based on the second EEG/MEG data, wherein each of the second plurality of times series corresponds to a respective source position located inside the cranial cavity (300) of the subject (110),
calculating a fourth correlation value for a fourth pair of time series, the fourth pair of time series being included in the determined second plurality of time series, and the fourth pair of time series corresponding to the same respective source positions as the first pair of time series,
calculating a comparison value between the fourth correlation value and the first correlation value, and
wherein the one or more scores (150) is generated based on the comparison value.

**13.** The method (400) of any of claims 8 to 12,
wherein the determining (420) the first plurality of time series comprises filtering the first EEG/M EG data (130) such that the first plurality of time series only includes a subset of all available time series in the first EEG/MEG data, the subset including the first pair of time series, the second pair of time series, and the third pair of time series.

**14.** A computer-readable medium comprising instructions that when executed by a processor perform the method (400) of any of claims 8 to 13.

FIG. 1

FIG. 2

FIG. 3

FIG. 4

FIG. 5

FIG. 6

FIG. 7

FIG. 8

EP 4 335 370 A1

FIG. 9

FIG. 10

FIG. 11

FIG. 12

EP 4 335 370 A1

FIG. 13

140,200

| reception component | ~210 |

| time-series determination component | ~220 |

| correlation value calc. component | ~230 |

| score generation component | ~240 |

| score output component | ~250 |

FIG. 14

FIG. 15

400

```
┌──────────────────────────────┐
│      receiving EEG/MEG data   │~410
│        of a subject           │
└──────────────────────────────┘
               │
               ▼
┌──────────────────────────────┐
│ determining a first plurality of time-series │~420
│    based on the EEG/MEG data  │
└──────────────────────────────┘
               │
               ▼
┌──────────────────────────────┐
│      calculating a correlation │~430
│   value for a pair of time-series │
└──────────────────────────────┘
               │
               ▼
┌──────────────────────────────┐
│     generating a score based  │~440
│      on the correlation value │
└──────────────────────────────┘
               │
               ▼
┌──────────────────────────────┐
│      outputting the generated │~450
│             score             │
└──────────────────────────────┘
```

FIG. 16

FIG. 17

Attention Deficit Detection System 1700

Measurement Module 1710

Report Module 1720

Network 1770

Reactions 1740

Stimuli 1780

User Device 1750

User 1760

Database 1730

Control Group 1731

Users 1732

Task Definitions 1733

Performance Metrics 1734

1800

1830    1840

| First Task | Red Color | Other Color |
|---|---|---|
| Consonant | No Reaction | |
| Vowel | No Reaction | React |

1810

1820

FIG. 18A

1850

1880    1890

| Second Task | Red Color | Other Color |
|---|---|---|
| Consonant | No reaction | |
| Vowel (Not 'E') | No Reaction | React |
| Letter 'E' | React | React |

1860

1870

1875

FIG. 18B

$$I(a|s,c,e) = I(a) - I(s) + I(s|a) - I(c|s) + I(c|a,s) - I(e|s,c) + I(e|a,s,c) = I(a) - TIC(s \to a) - TIC(c \to a|s) - TIC(e \to a|s,c)$$

FIG. 19

EP 4 335 370 A1

Fig. 20

A

2039

is first task?

No

Yes

2040

is FC met?

No

2044

is FC met?

No

E

Yes

2045

is SC met?

No

2047

is TC met?

No

2046

is TC met?

No

Yes

Yes

Yes

2041

is SC met?

No

Yes

2048

high level of connectivity?

No

Yes

D

E

Fig. 20 (continued)

Fig. 20 (continued)

C

Yes

2057

is connectivity reduced lesser A1 than A2?

Yes

D

No

2058

is connectivity reduction more different in A3 than A4?

Yes

2059

No

Stop

# Fig. 20 (continued)

Fig. 21

Europäisches Patentamt

European Patent Office

Office européen des brevets

# EUROPEAN SEARCH REPORT

Application Number

EP 22 38 2837

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/060125 A1 (ZEMAN PHILIP MICHAEL [CA] ET AL) 7 March 2013 (2013-03-07) * paragraphs [0022], [0077] - [0093], [0109] - [0111], [0174] - [0198], [0218] - [0248], [0339] - [0367] * * paragraphs [0485] - [0495], [0522] - [0551]; claims; figures * | 1-14 | INV. A61B5/16 A61B5/246 A61B5/374 A61B5/378 A61B5/00 ADD. A61B5/055 |
| X | CHEN MU-HONG ET AL: "Functional connectivity of specific brain networks related to social and communication dysfunction in adolescents with attention-deficit hyperactivity disorder", PSYCHIATRY RESEARCH, ELSEVIER IRELAND LTD, IE, vol. 284, 13 January 2020 (2020-01-13), XP086027207, ISSN: 0165-1781, DOI: 10.1016/J.PSYCHRES.2020.112785 [retrieved on 2020-01-13] * page 2 - page 6; figures; tables * | 1-14 | |
| X | SON YOUNG DON ET AL: "A functional connectivity comparison between attention deficit hyperactivity disorder and bipolar disorder in medication-naïve adolescents with mood fluctuation and attention problems", PSYCHIATRY RESEARCH: NEUROIMAGING, vol. 263, 30 May 2017 (2017-05-30), - 30 May 2017 (2017-05-30), pages 1-7, XP085007633, ISSN: 0925-4927, DOI: 10.1016/J.PSCYCHRESNS.2017.02.006 * the whole document * | 1-14 | TECHNICAL FIELDS SEARCHED (IPC) A61B G16H |

-/--

The present search report has been drawn up for all claims

| Place of search | Date of completion of the search | Examiner |
|---|---|---|
| The Hague | 11 February 2023 | Crisan, Carmen-Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

# EUROPEAN SEARCH REPORT

Application Number

EP 22 38 2837

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SEMRA ICER ET AL: "Differences in brain networks of children with ADHD: Whole-brain analysis of resting-state fMRI", INTERNATIONAL JOURNAL OF IMAGING SYSTEMS AND TECHNOLOGY, WILEY AND SONS, NEW YORK, US, vol. 29, no. 4, 3 June 2019 (2019-06-03), pages 645-662, XP071751274, ISSN: 0899-9457, DOI: 10.1002/IMA.22348 * page 646 - page 661 * | 1-14 | |
| X | SIDLAUSKAITE JUSTINA ET AL: "Altered intrinsic organisation of brain networks implicated in attentional processes in adult attention-deficit/hyperactivity disorder: a resting-state study of attention, default mode and salience network connectivity", EUROPEAN ARCHIVES OF PSYCHIATRY AND CLINICAL NEUROSCIENCE, SPRINGER BERLIN HEIDELBERG, BERLIN/HEIDELBERG, vol. 266, no. 4, 11 August 2015 (2015-08-11), pages 349-357, XP035691983, ISSN: 0940-1334, DOI: 10.1007/S00406-015-0630-0 [retrieved on 2015-08-11] * the whole document * | 1-14 | |

**TECHNICAL FIELDS SEARCHED (IPC)**

The present search report has been drawn up for all claims

| Place of search | Date of completion of the search | Examiner |
|---|---|---|
| The Hague | 11 February 2023 | Crisan, Carmen-Clara |

EPO FORM 1503 03.82 (P04C01)

## ANNEX TO THE EUROPEAN SEARCH REPORT
## ON EUROPEAN PATENT APPLICATION NO.

EP 22 38 2837

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2023

| Patent document cited in search report | | Publication date | Patent family member(s) | | Publication date |
|---|---|---|---|---|---|
| US 2013060125 | A1 | 07-03-2013 | US | 2013060125 A1 | 07-03-2013 |
| | | | WO | 2011127609 A1 | 20-10-2011 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RAO, R.P.N.** Bayesian Computation in Recurrent Neural Circuits. *Neural Comput.,* 2004, vol. 16, 1-38
- **POUGET, A. et al.** Probabilistic brains: knowns and unknowns. *Nat. Neurosci.,* 2013, vol. 16, 1170-1178
- **CORBETTA et al.** The Reorienting System of the Human Brain: From Environment to Theory of Mind. *Neuron,* 2008, vol. 58, 306-324

- **N. TZOURIO-MAZOYER ; B. LANDEAU ; D. PAPATHANASSIOU ; F. CRIVELLO ; O. ETARD ; N. DELCROIX ; BERNARD MAZOYER ; M. JOLI-OT.** Automated Anatomical Labeling of activations in SPM using a Macroscopic Anatomical Parcellation of the MNI MRI single-subject brain. *NeuroImage,* January 2002, vol. 15 (1), 273-289